# FASCICULE DE BREVET EUROPEEN

(11) **EP 0 550 691 B1**
(45) Date de publication et mention de la délivrance du brevet: **13.03.1996**
(21) Numéro de dépôt: 91919407.6
(22) Date de dépôt: 27.09.1991
(51) Int. Cl.: C12N 15/35, C07K 14/155, C12N 15/49, C07K 5/00, C12N 15/62, A61K 39/395, A61K 39/02, G01N 33/569, C12P 21/08

(54) **PEPTIDES INDUCTEURS D'ANTICORPS INHIBANT DES RETROVIRUS DU TYPE HIV ET ANTICORPS DIRIGES CONTRE CES PEPTIDES**
PEPTIDE, WELCHE HIV-RETROVIREN-HEMMENDE ANTIKÖRPER INDUZIEREN, SOWIE GEGEN DIESE PEPTIDE GERICHTETE ANTIKÖRPER
ANTIBODY INDUCTOR PEPTIDES INHIBITING RETROVIRUSES OF THE HIV TYPE AND ANTIBODIES SPECIFIC FOR SAID PEPTIDES

(30) Priorité: 27.09.1990 FR 9011951; 26.10.1990 FR 9013324
(43) Date de publication de la demande: 14.07.1993
(60) Demande divisionnaire: 95112019.5
(73) Titulaire: INSTITUT PASTEUR, F-75724 Paris Cédex 15 (FR)
(72) Inventeur: MONTAGNIER, Luc, F-92350 Le Plessis-Robinson (FR); BERNEMAN, Danielle, F-75011 Paris (FR); GUETARD, Denise, F-75015 Paris (FR); BAHRAOUI, Elmostafa, F-78000 Versailles (FR); VAN RIETSCHOTEN, Jurphaas, F-13100 Aix-en-Provence (FR); CHAMARET, Solange, F-75015 Paris (FR); BLANCHARD, Alain, F-92129 Montrouge (FR)
(74) Mandataire: Benech, Frédéric
(86) Numéro de dépôt international: FR9100758
(87) Numéro de publication internationale: WO9206199

(56) Documents cités:
- WO-A-86/02383
- US-A- 4 945 041
- Infection and Immunity, vol. 57, no. 4, avril 1989; S.F. DALLO et al.
- International Journal of Peptide and Protein Research, vol. 35, no. 1, janvier 1990, (Munksgaard, DK), J.M. SABATIER et al.: "Large fragments of nef-protein and gp110 envelope gycoprotein from HIV-1", pages 63-72, voir page 64, séquence P3
- GENE, vol. 64, 1988, (Amsterdam, NL), J.M. INAMINE et al.: "Nucleotide sequence of the P1 attachment protein gene of Mycoplasma pneumoniae", pages 217-229, voir figure 4, acides aminées correspondantes à nucleotides 680-700,3870-3894, 4130-4190
- Comptes Rendus de l'Academie des Sciences, vol. 311, no. 12, 6 décembre 1990, (Paris, FR), L. MONTAGNIER et al.: "Inhibition de l'infectiosité de souches prototypes du VIH par des anticorps dirigés contre une séquence peptidique de mycoplasma", pages 425-430, voir le document en entier

## Description

L'invention concerne des peptides inhibant ou diminuant l'infectiosité de retrovirus du type HIV et des anticorps dirigés contre ces peptides. Dans ce qui suit l'expression "peptides" recouvre tout aussi bien des peptides ou polypeptides, naturels ou synthétiques, des fragments de protéines ou des polypeptides portant ou non des groupements de substitutions caractéristiques de ceux que l'on retrouve chez certaines protéines de structure, par exemple des groupes de glycosylation ou tous peptides pour lesquels une substitution en acides aminés dans la séquence ne modifie pas substantiellement l'activité du peptide telle que décrite dans l'invention.

Font partie de l'invention tous les peptides dont les séquences d'aminoacides (ou en acides aminés) sont issues des protéines de mycoplasmes ainsi que les séquences en aminoacides contenant lesdits peptides et qui sont susceptibles d'induire in vivo ou in vitro (pour les techniques de production in vitro d'anticorps on peut se référer aux travaux de PLUCKTHUN et al décrits dans **Science** 1988, 240 p 1038) la production d'anticorps protecteurs aptes à inhiber ou diminuer in vitro l'infectiosité de HIV, notamment à réduire le développement de l'infection par HIV de lymphocytes T4 par rapport au développement de l'infection observé dans des cultures témoins, en l'absence de ces peptides ou de leurs anticorps spécifiques, voire même à l'inhiber totalement dans les conditions expérimentales décrites plus loin. Cette réduction de l'infection est par exemple appréciée par la réduction correspondante des proportions dosées de transcriptase inverse ou de protéine p24/p25 gag synthétisée dans le cas d'une infection avec HIV-1.

La désignation HIV correspond à l'abréviation de l'expression anglaise "Human immuno-deficiency virus" (Virus d'Immuno-deficience Humaine ou "HIV") telle que définie dans **Nature**, 1988, Vo. 321, p. 10 (COFFIN, J. et al).

Plusieurs auteurs ont montré que les peptides de Mycoplasma pneumoniae étaient reconnus par des anticorps de patients atteints de pneumonies (J. Clin Microbiology, 1990, 28(6), p. 1194-1197, Jacobs E. et al) mais les séquences décrites n'indiquent aucune capacité des anticorps dirigés contre elles à inhiber l'infection à HIV.

Les peptides selon l'invention sont caractérisés par des séquences d'acides aminés correspondant à des séquences contenues dans des protéines de mycoplasmes ou dérivées de celles-ci, tout en conservant leur capacité à induire la synthèse d'anticorps inhibant in vitro l'infection, par HIV-1 ou HIV-2, de lymphocytes T ou d'autre types cellulaires permissifs au HIV.

De manière préférée les peptides présentent un pourcentage d'isologie (ou substitution conservative) d'au moins 60% avec des séquences d'acides aminés des protéines issues de HIV.

Des peptides préférés de l'invention contiennent de 5 à 25 acides aminés, notamment de 8 à 15 acides aminés, qui présentent préférentiellement le susdit pourcentage d'isologie avec des séquences d'acides aminés issus d'une protéine de HIV, notamment la protéine gag, les glycoprotéines d'enveloppe, ou encore de la protéine nef d'un HIV (Tableau I).

Compte-tenu de la variabilité génétique des HIV, il apparaît immédiatement que les peptides préférés sont ceux qui présentent un pourcentage d'isologie d'au moins 60%, voire de 70%, avec des régions conservées des glycoprotéines ou protéines de HIV, notamment HIV-1 et HIV-2. Par l'expression "région conservée" de glycoprotéines ou protéines ou peptides de HIV, on entend toutes séquences peptidiques d'au moins 10 acides aminés qui se correspondent mutuellement dans des variants de HIV et présentant un pourcentage d'homologie d'au moins 30% avec les protéines de mycoplasmes, conformément à la définition qui sera donnée de cette expression dans ce qui suit.

Parmi les peptides préférés de l'invention on citera ceux qui contiennent des séquences d'acides aminés correspondant à des protéines de structure de mycoplasme et, de manière préférentielle, les séquences qui contiennent des sites actifs d'adhésines de mycoplasme, en particulier de M. pneumoniae, de M. genitalium (souche de référence G37) ou de M. pirum, etc. Font également partie des peptides selon l'invention les peptides correspondant à des protéines de structure de mycoplasme telles que les protéines adhésines et qui présentent les pourcentages d'isologie sus-indiqués avec les séquences gp 160 env, gp 120 env, gp 41 env, p 27 nef et p 55 gag de HIV-1 ou gp 300 env, gp 140 env, p 31 nef, p 55 ou gp 36 env de HIV-2. On désigne ici sous l'expression "adhésine" un composant membranaire des mycoplasmes, responsable de l'adhésion de celui-ci à des supports inertes ou à des cellules.

On parle, dans cette demande de brevet, d'homologie ou d'isologie de séquence entre une séquence d'acides aminés issue d'une protéine de HIV et une séquence issue d'une protéine de mycoplasme lorsque, les représentations symboliques en lignes de séquences d'acides aminés issues de deux protéines ayant été placées l'une au dessous de l'autre, on voit apparaître - au besoin au prix d'un décalage de certaines sous-séquences au sein de l'une de ces séquences les unes vis à vis des autres, de façon à permettre des espacements entre ces sous-séquences qui, respectivement, n'excèdent pas la longueur (représentée par un "tiret" : " - " de l'emplacement correspondant à un acide aminé unique -,
- soit, dans le cas des homologies, une identité entre deux acides aminés placés au droit l'un de l'autre (ou l'un au-dessus de l'autre)
- soit dans le cas des isologies, une appartenance à la même famille, des deux acides aminés au droit l'un de l'autre dans les deux séquences ou sous-séquences respectives, cette famille étant choisie parmi l'une des sept familles suivantes (familles isologiques) :
   (1) R K
   (2) A G W
   (3) L F P M V I
   (4) H
   (5) S T Q N C
   (6) E D
   (7) Y.

Par exemple il y aura isologie lorsqu'au droit d'un acide aminé A dans la représentation symbolique de la première séquence (ou sous-séquence de cette première séquence) se trouve un acide aminé G ou W (famille (2) ci-dessus) dans la représentation symbolique de la seconde séquence.

Deux peptides respectivement issus d'une protéine de mycoplasme et d'une protéine de HIV sont dits présenter une "isologie" ou "homologie" de 30%, lorsque 30% de leurs acides aminés respectifs se trouvent dans la relation sus-définie d'isologie ou d'homologie.

D'autres peptides préférés seront encore indiqués dans la description d'exemples préférés qui suit.

Les peptides préférés sont d'une part ceux qui, issus d'une protéine de structure de mycoplasme ou dérivés de celles-ci, induisent la synthèse d'anticorps inhibant l'infection à HIV et d'autre part ceux dont la séquence comporte une homologie d'au moins 30%, de préférence même une homologie d'au moins 70% avec des séquences correspondantes issues de protéines de HIV. Il peut encore être noté que la longueur préférée de la séquence - au sein de celles qui répondent à la condition sus-indiquée d'induire des anticorps protecteurs contre un HIV - peut également être dictée par le pourcentage d'isologie et, le cas échéant, encore davantage par le pourcentage d'homologie.

Comme cela est rapporté plus loin, l'invention concerne plus particulièrement des séquences peptidiques courtes (comprises entre 5 et 25 acides aminés) de protéines de mycoplasmes qui présentent des pourcentages d'homologie supérieurs à 70% entre elles. Naturellement les peptides constitués par les séquences contenues dans les protéines correspondantes des HIV font également partie de l'invention et, à ce titre, doivent être considérés comme couverts par les revendications.

L'invention découle de deux hypothèses qui ont été faites l'une visant à perturber le contact entre cellules et mycoplasmes susceptibles d'agir en cofacteur du HIV lors d'une infection par ce virus d'une cellule et la seconde hypothèse étant basée sur l'interaction des mycoplasmes avec HIV-1 chez un sujet infecté qui pourrait mettre en jeu des séquences peptidiques de ces deux microorganismes présentant un certain degré d'isologie, sinon d'homologie. En effet, les infections à mycoplasmes sont très fréquentes. Plusieurs articles du groupe de LO ont indiqué la présence de mycoplasmes en localisation intracellulaire dans des tissus isolés de patients infectés par HIV-1 (1) (2) (3), en l'absence de toute correlation dans ces articles entre l'infection à HIV et la présence de mycoplasma fermentans à localisation intracellulaire. L'effet cytopathogène du virus peut être réduit in vitro par des antibiotiques actifs sur les mycoplasmes, d'où l'hypothèse d'une interaction voire d'une coopération entre HIV et une population de mycoplasmes lors de l'infection in vitro de cultures de lymphocytes T et d'autres cellules permissives au HIV (4), bien que l'utilisation de tels antibiotiques ne conduise pas à une éradication totale des mycoplasmes infectant des lymphocytes T.

La présente invention a conduit à la détermination de la structure de peptides issus de protéines de mycoplasmes susceptibles d'induire la synthèse d'anticorps inhibant une infection à HIV dans une culture de lymphocytes T ou d'autres cellules permissives à HIV. L'une des catégories de peptides selon l'invention est définie comme regroupant les peptides présentant outre leur capacité à induire la synthèse d'anticorps inhibant ou diminuant une infection à HIV, la capacité d'avoir une homologie ou une isologie avec des protéines de HIV. Pour cela une recherche d'homologie a été faite entre les séquences des sites actifs des adhésines de deux mycoplasmes (M. pneumoniae et M. genitalium) et les séquences d'acides aminés codées par les gènes env, nef et gag d'HIV-1 et HIV-2.

L'invention couvre également un procédé de préparation de "molécules hybrides" ou "peptides hybrides" caractérisé; en ce qu'il met en oeuvre la construction de molécules d'acides nucléiques constituées pour partie de séquences nucléotidiques codant pour au moins un peptide appartenant à HIV et, pour partie une séquence codant pour au moins un peptide de mycoplasme selon l'invention. L'invention couvre également les compositions de peptides hybrides liés entre eux de manière covalente ou constituées par un mélange non covalent de peptides de HIV et de peptides de mycoplasmes selon l'invention, non liés entre eux directement de manière covalente.

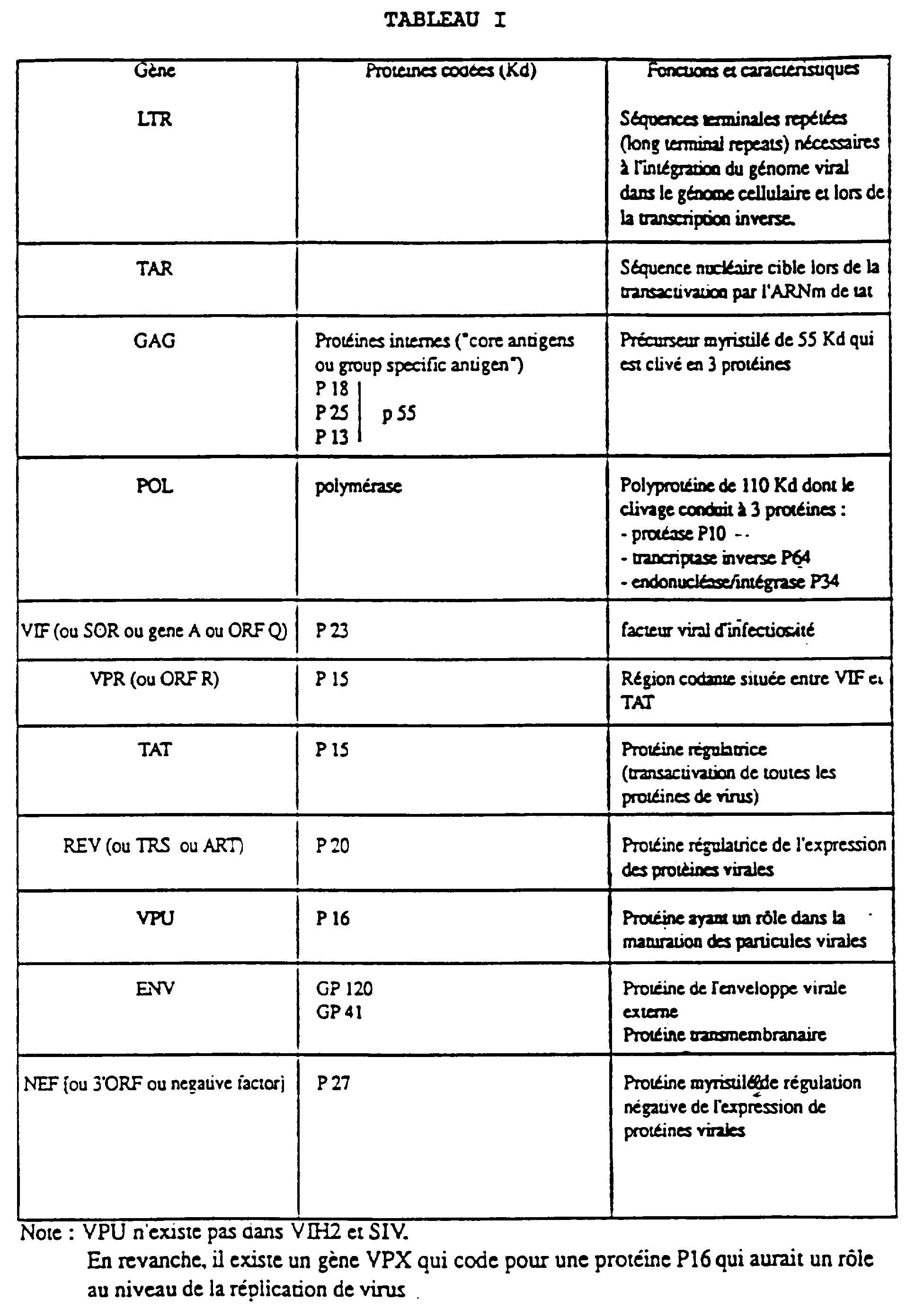

L'étude a porté plus particulièrement sur les protéines de structure et en particulier les protéines adhésines de Mycoplasma genitalium et de Mycoplasma pneumoniae.

La séquence nucléotidique du gène de la protéine P1 d'attachement à l'épithélium cilié respiratoire, de M.pneumoniae a été décrite par Inamine et al (6) et un site essentiel pour l'adhésion par Dallo, S.F. et al. (5).

La séquence du gène codant pour l'adhésine de M. genitalium a été décrite par Dallo et al (12) et il a été montré par Morrison-Plummer et al (7) que l'adhésine de M.genitalium partageait des déterminants immunologiques communs avec M.pneumoniae pour la fixation sur la cellule. Cette adhésine est dénommée "MgPa" pour M.genitalium.

Le tableau II présente l'homologie des séquences des adhésines au niveau des régions impliquées dans la cytoadhérence.

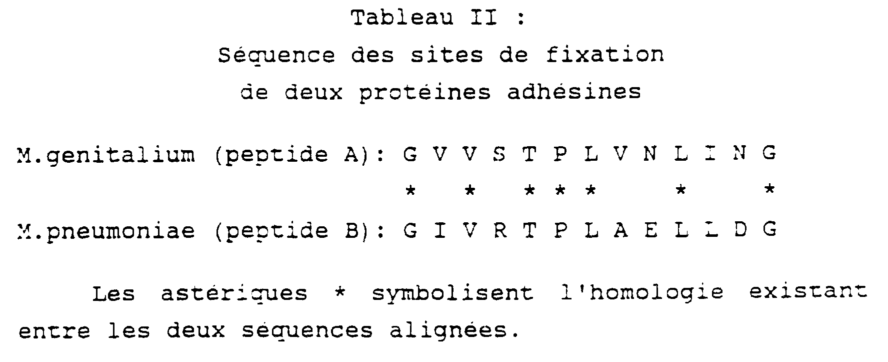

L'outil informatique mis en oeuvre était un ordinateur DATA GENERAL MV/8000.

Les séquences des adhésines de M. pneumoniae et de M. genitalium non accessibles directement sur banques de données ont été entrées manuellement.

Pour avoir accès aux séquences de protéines rétrovirales on peut également se référer à la source connue sous la désignation "Compilation and Analysis of nucleic acid and aminoacid sequences of human retroviruses and AIDS" (Compilation et analyse de séquences d'acides nucléiques et d'aminoacides de rétrovirus humains et SIDA), 1988-1989, éditée par Gerald Myers et al, Los Alamos, USA, "National Laboratory" (Laboratoire National).

### Critères de tri des données et alignement des séquences

L'algorithme de Lipman et Pearson a été utilisé (9, 10)
a) Pour les recherches d'homologies strictes on a pris comme critère de tri:
   - la présence de deux acides aminés consécutifs homologues ("K-tuple size" 2 ou taille minimum des séquences homologues à partir desquelles l'ordinateur commence sa recherche de séquences plus longues ayant un pourcentage d'homologie minimum choisi par l'opérateur),
   - une "lecture" de 20 en 20 acides aminés (taille de la fenêtre ou "window size"),
   - une pénalité d'espacement maximum entre sous-séquences ("gap penalty") de un.
b) Pour les alignements par isologie, le "K-tuple size" est de 3.

**TABLEAU III**

| Représentation symbolique des acides aminés | | |
|---|---|---|
| Alanine | Ala | A |
| Arginine | Arg | R |
| Asparagine | Asn | N |
| Aspartate | Asp | D |
| Asn+Asp | Asx | B |
| Cysteine | Cys | C |
| Glutamine | Gln | Q |
| Glutamate | Glu | E |
| Gln+Glu | Glx | Z |
| Glycine | Gly | G |
| Histidine | His | H |
| Isoleucine | Ile | I |
| Leucine | Leu | L |
| Lysine | Lys | K |
| Méthionine | Met | M |
| Phenylalanine | Phe | F |
| Proline | Pro | P |
| Sérine | Ser | S |
| Thréonine | Thr | T |
| Tryptophane | Trp | W |
| Tyrosine | Tyr | Y |
| Valine | Val | V |

### Recherches d'homologies entre les adhésines de M. peumoniae et de M. genitalium et les protéines d'envelope de HIV-1 (BRU) et P55 gag HIV-1 (BRU) et P27 nef HIV-1 (BRU) (résultats)

A titre de référence HIV1 (BRU) a été décrit comme étant HIV1 (LAI) Science, 1991, WAIN-HOBSON et al, 252, p. 961-965, nous avons commencé par effectuer un alignement direct entre la séquence de l'adhésine de M. pneumoniae (1624 acides aminés) et celle de M. genitalium (1444 acides aminés). Ce résultat a montré 723 acides aminés communs, soit 45% d'homologie. Dans l'approche par isologies entre M. pneumoniae et M. genitalium, le pourcentage est de 59% (957 acides aminés/1624).)

### A) Homologies gp 160 env (HIV-1 BRU) et adhésine P1 de M. pneumoniae

1) L'alignement de la séquence de P1 sur la séquence de la protéine de l'enveloppe montre 178 homologies ("matches" ou "coïncidences"). Ceci n'est pas significatif en raison de la large dispersion de ces homologies sur la séquence de ces 2 protéines. D'un point de vue "isologie", les résultats sont assez différents car le nombre de coïncidences est de 328 mais surtout certaines séquences présentent des coïncidences groupés et la dispersion est moins grande. En particulier plusieurs groupes de 8 à 10 coïncidences sont observés tels que : 
M. pneumoniae :                    S G Q N T S T
                                   *   * * * *
Glycoprotéine d'enveloppe HIV-1 : N A T N T N S S
ou :
M. pneumoniae :              T T T P V F G T S S G N
                             * * *   * * * *   * * *
Glycoprotéine d'en-          C T T A V P W N A S W S
veloppe de HIV-1 :
2) Alignement du site actif de fixation de P1 M.pneumoniae avec la séquence correspondante de la protéine d'enveloppe de HIV-1 ; aucun résultat significatif n'a été obtenu en homologie, du moins en ce qui concerne les séquences en acides aminés testées. En revanche, avec un alignement par substitution conservative ou isologique, on obtient :

env HIV-1 :                KVQKEY-AFFYKLDIIPIDND
                           *** *  ***  *    *** *
p1 (M.pneumoniae) :        KMNDDVDGIVRTPLAELLDGE
                           **    * *** ***   **  *
Mg Pa (M.genitalium) :     KMDGKLTGVVSTPLVNLINGQ
                           **       ***  * * * *
env HIV-1 :                KVQKEY-AFFYKLDIIPIDND

Les alignements montrent 9/21 acides aminés isologues entre HIV-1 et M. genitalium, 13/21 entre les 2 séquences des adhésines des mycoplasmes mentionnés ci-dessus.

Autre isologie retenue :

env HIV-1              :   WFNITNWLWYIKIFIMIVGGLV
                           ** ***         ***   *
site Pg Pa M.genitalium :  GV-VST---------PLVNLIN

Le site actif de fixation présumé de M.genitalium a 9 isologies communes avec une région de la partie transmembranaire de la protéine d'enveloppe du HIV1 (BRU)

### B) Homoloaies entre p27 nef de HIV-1 (BRU) et P1 de M. pneumoniae

Aucun résultat significatif par la méthode de tri utilisée n'a été obtenu pour les recherches d'homologies strictes et les isologies à partir des chaînes d'aminoacides plus particulièrement étudiées

### C) Homologies entre p55 gag de HIV-1 (BRU) et P1 de M.pneumoniae

Pas de résultat significatif avec la méthode de tri ulilisée.

### D) Homologies entre la gp160 de l'enveloppe de HIV-1 (BRU) et Mg Pa de M.genitalium

On observe 165 coïncidences dispersés sur toute la longueur. On remarque des séquences de 8 acides aminés, dont 6 acides aminés sont identiques, soit une homologie de 75%.

M.genitalium :          L I V T V P I V
                         * * * *     * *
gp 160 env HIV-1 :       L I V T R - I V

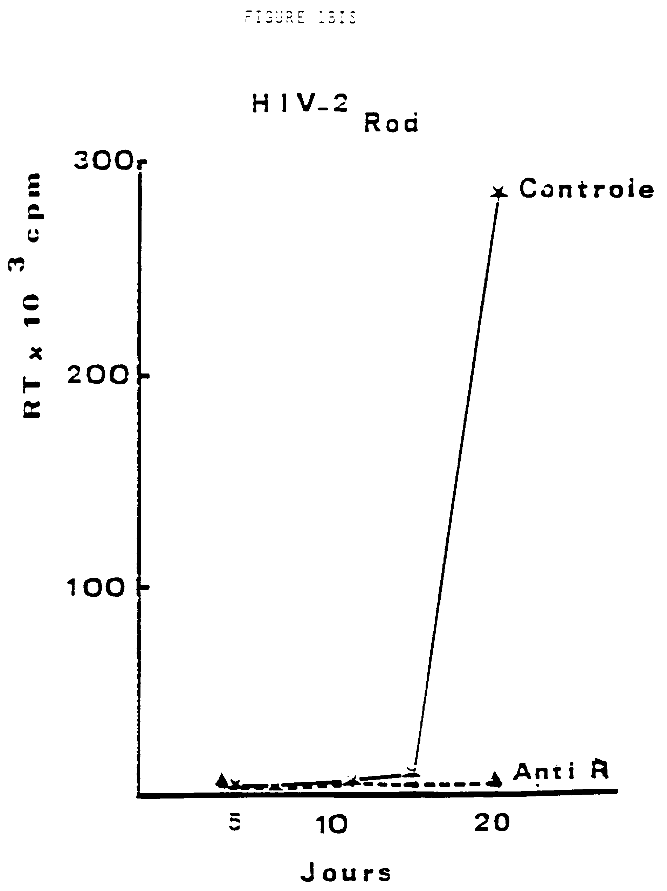

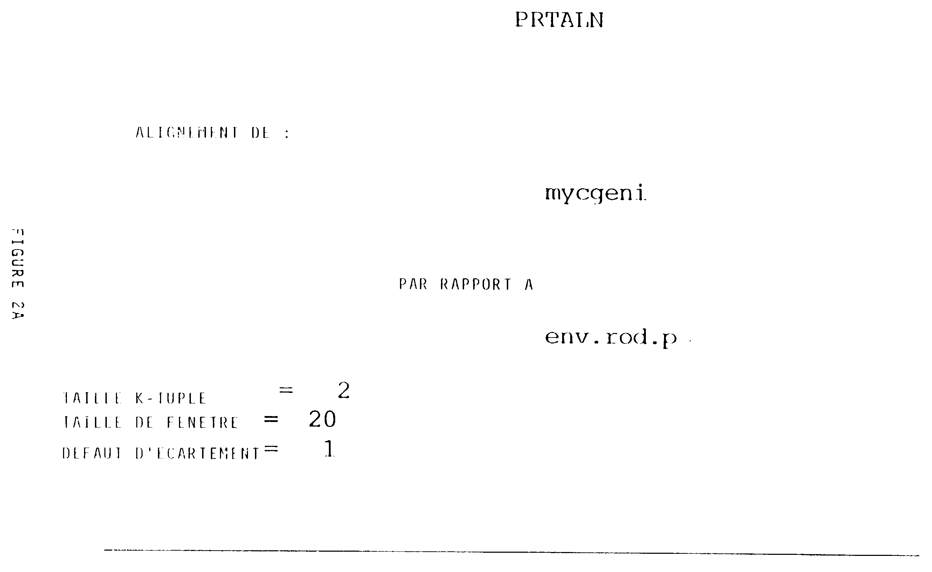

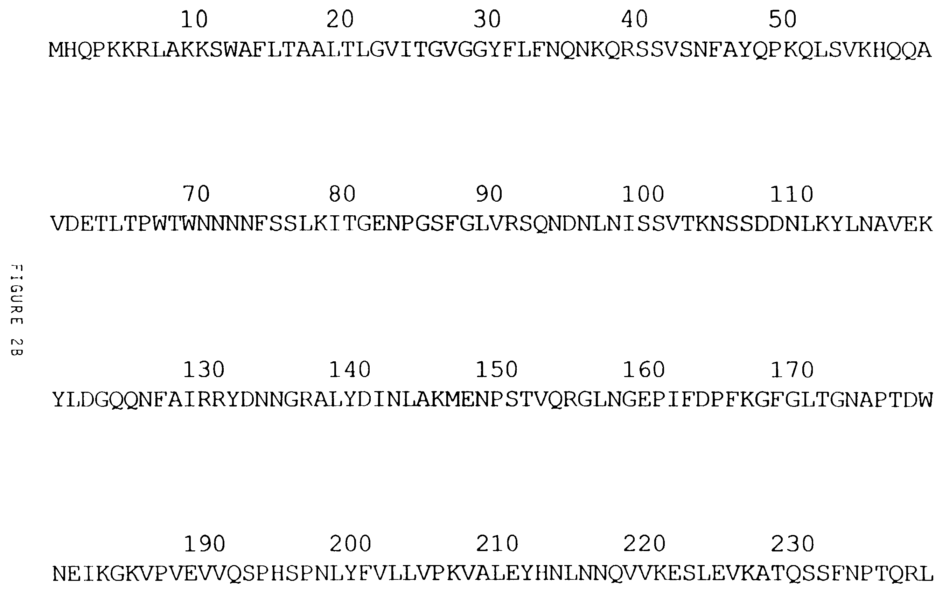

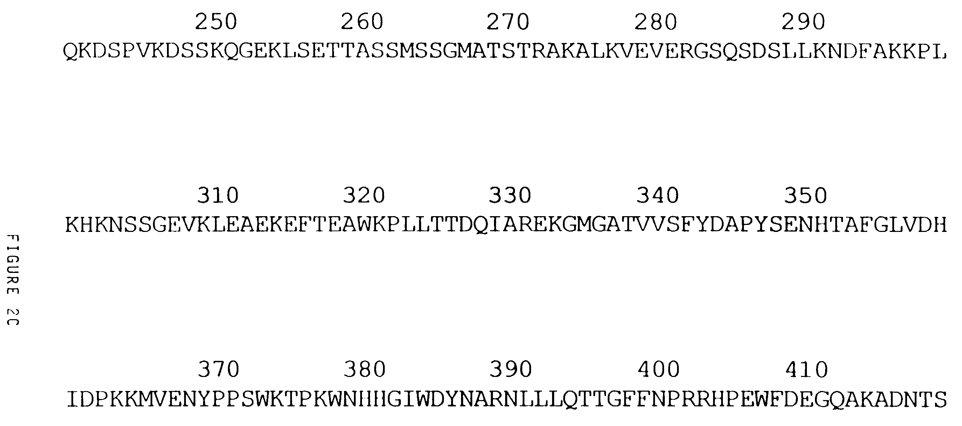

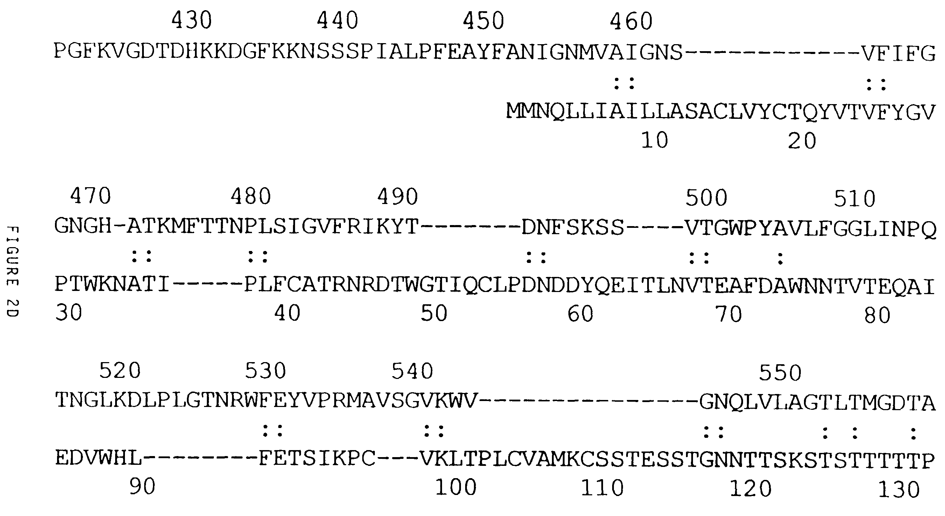

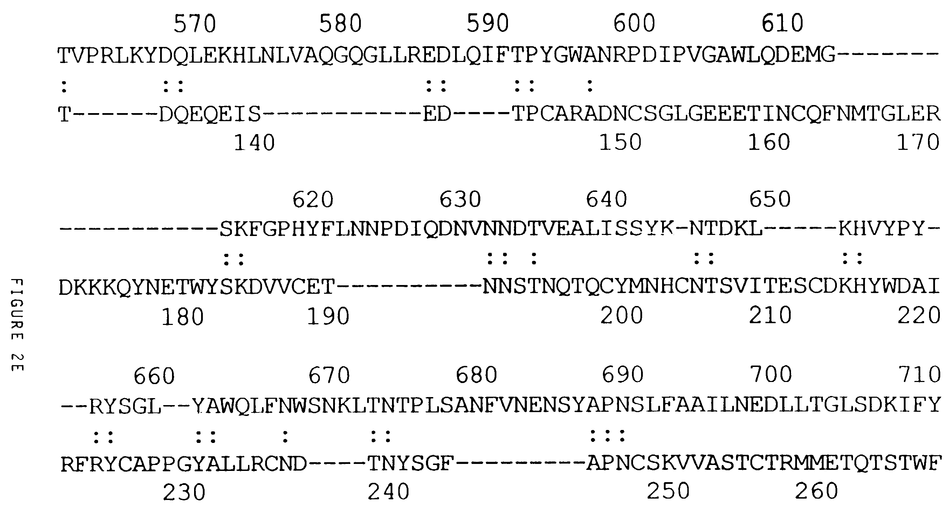

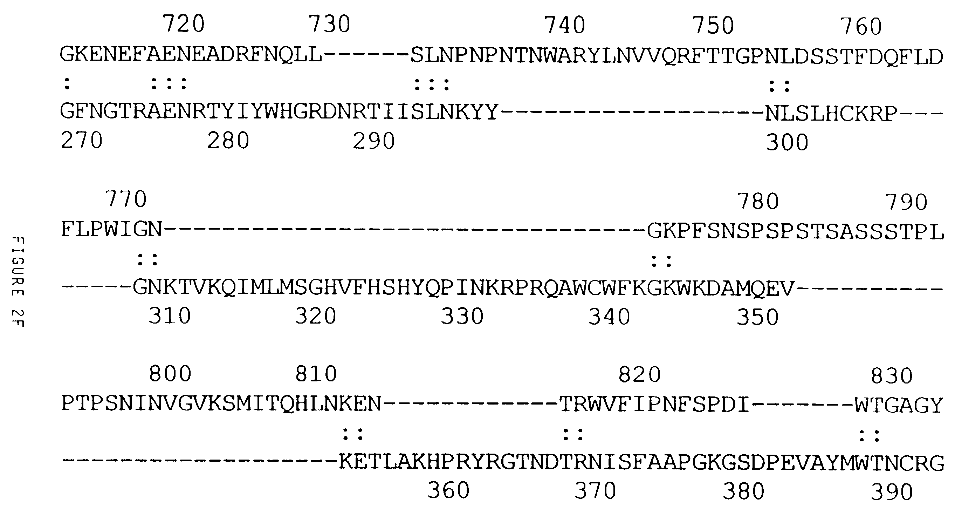

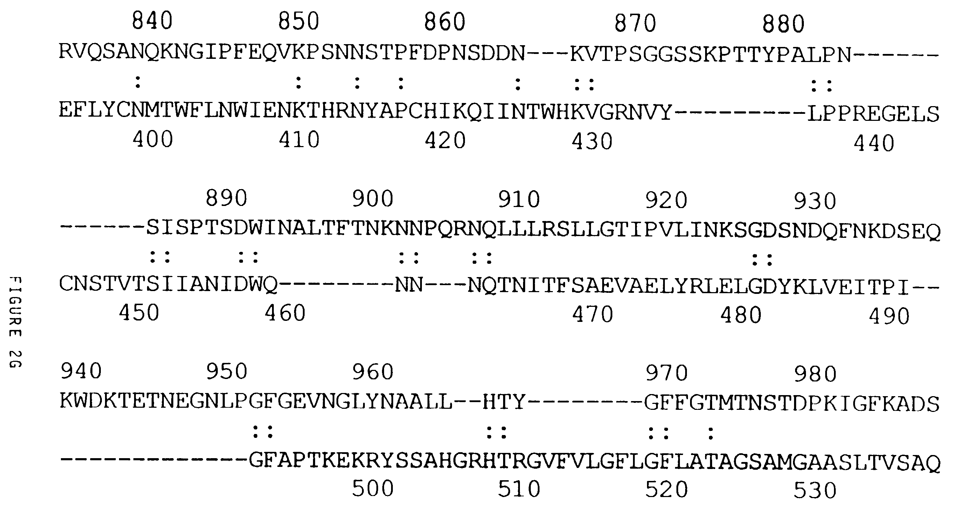

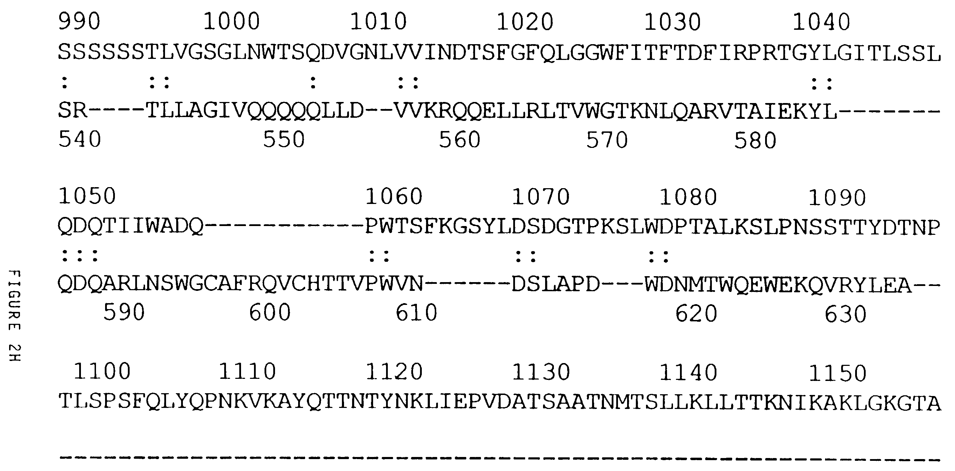

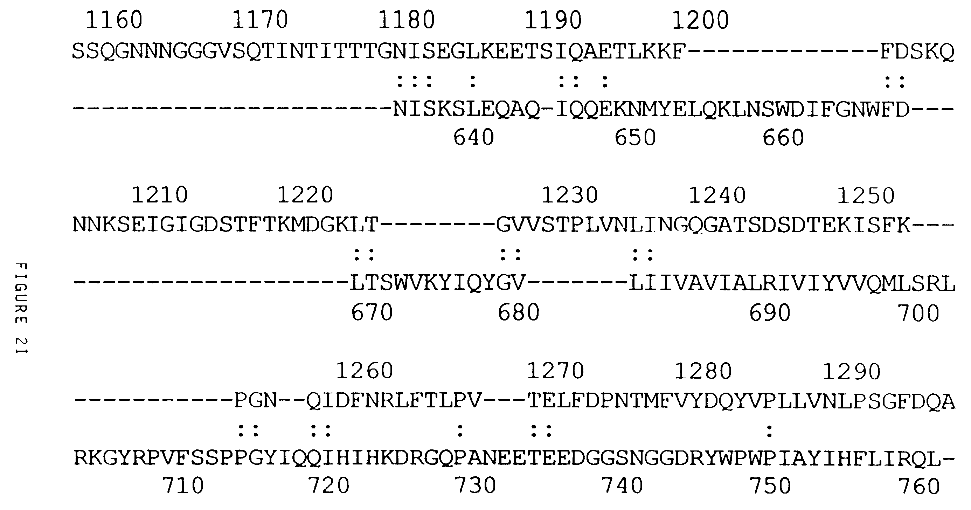

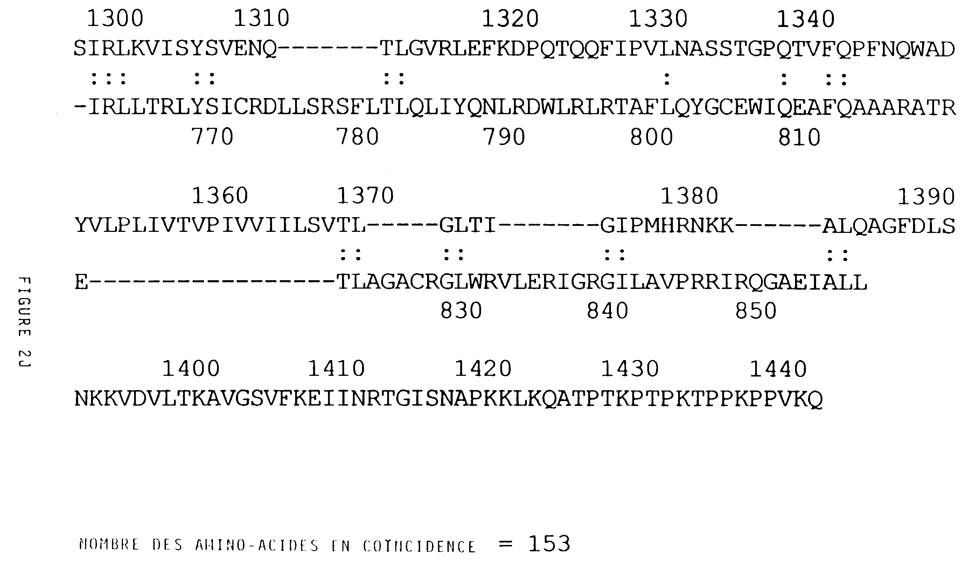

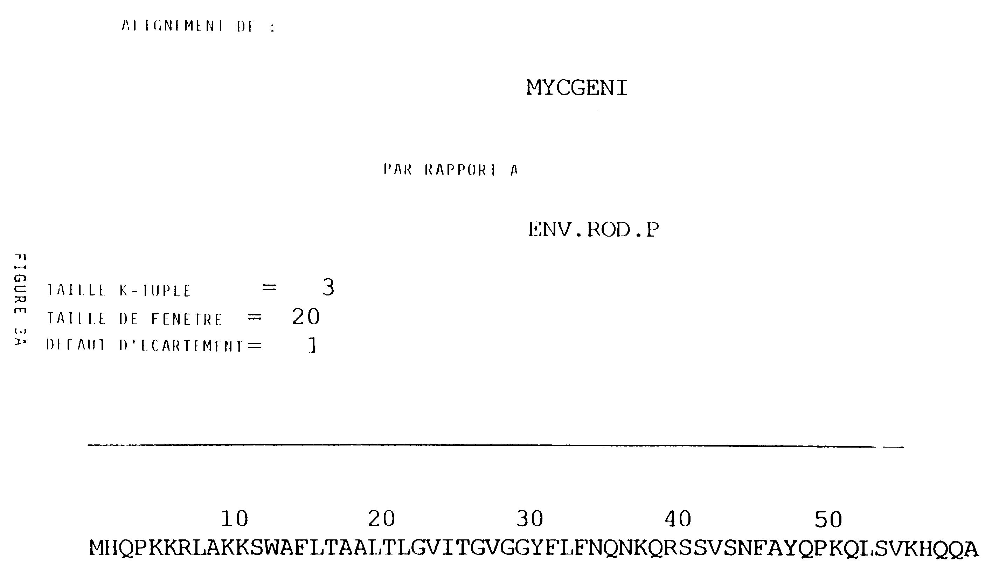

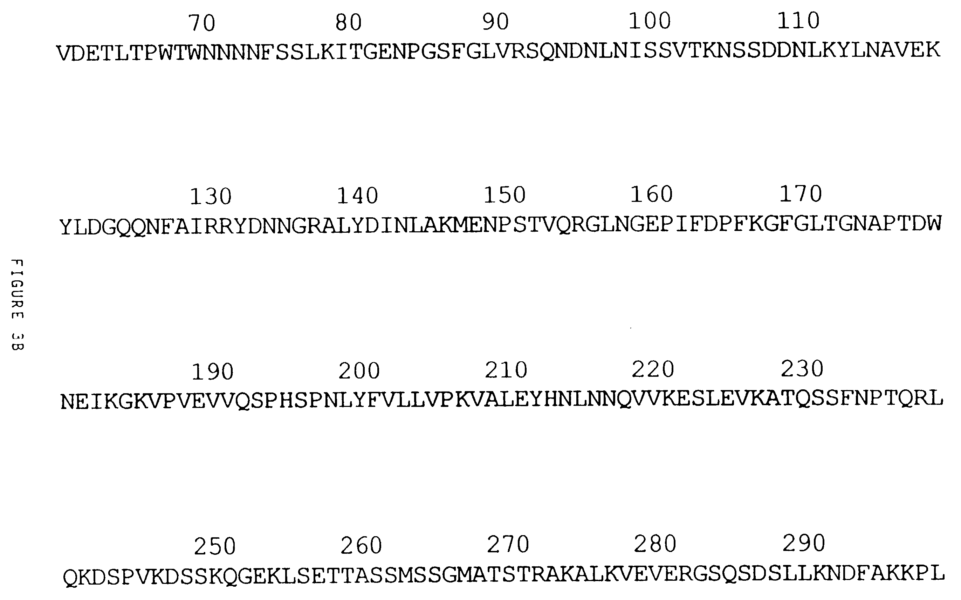

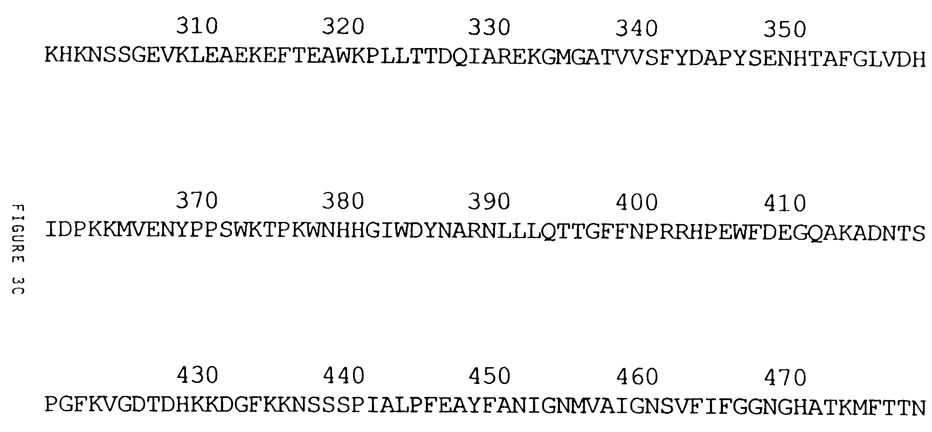

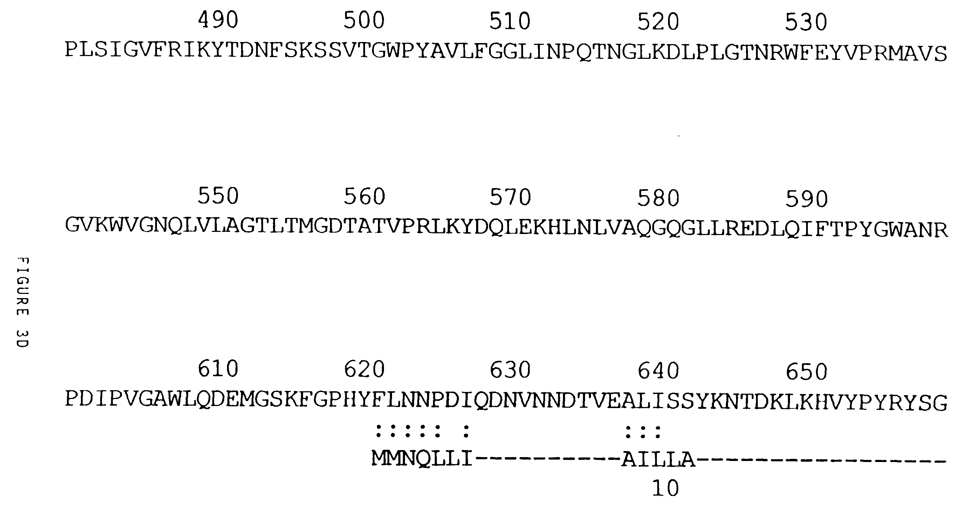

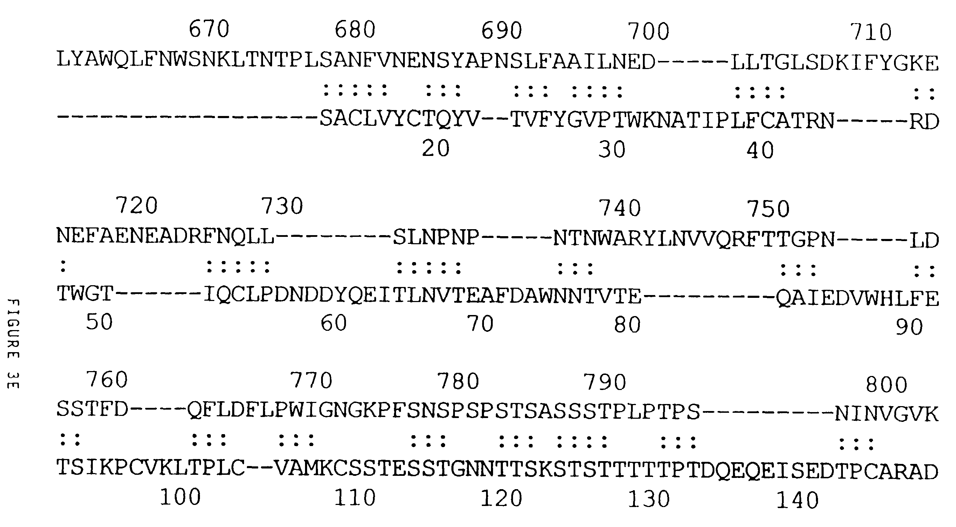

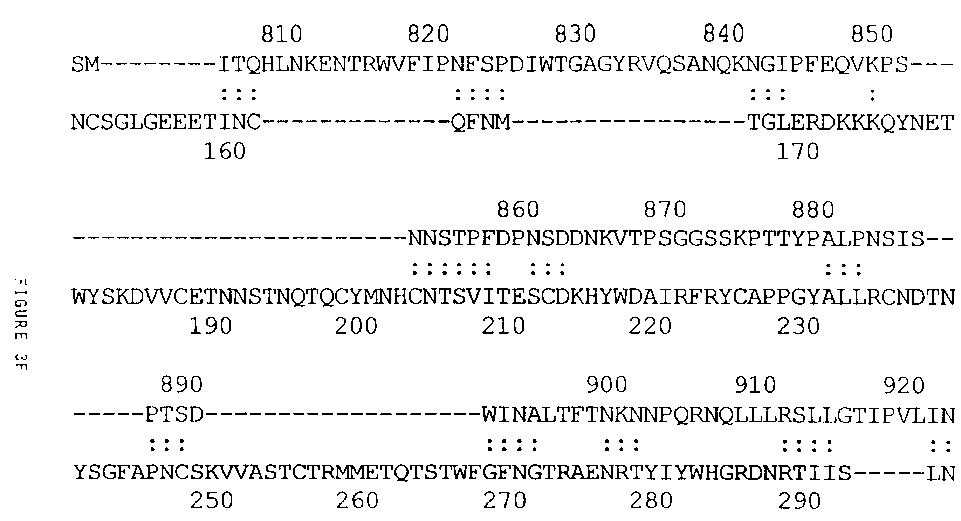

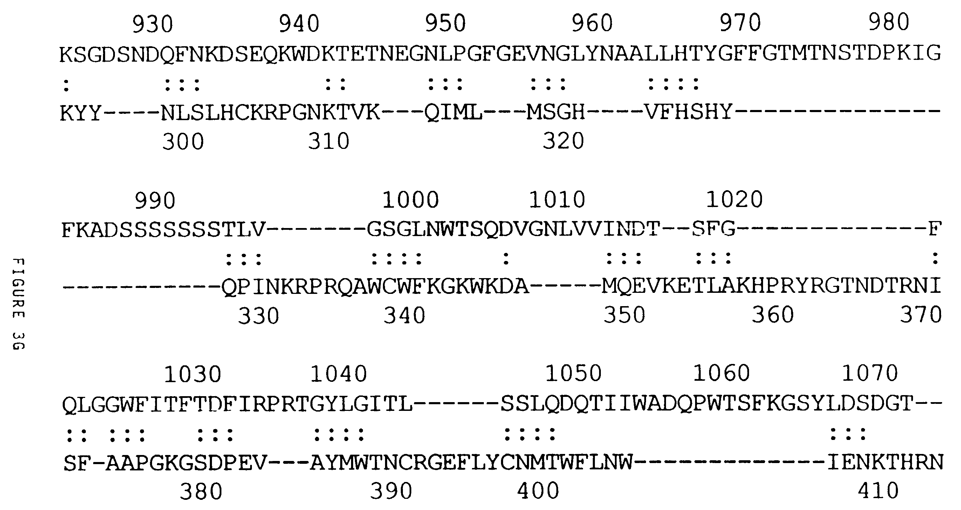

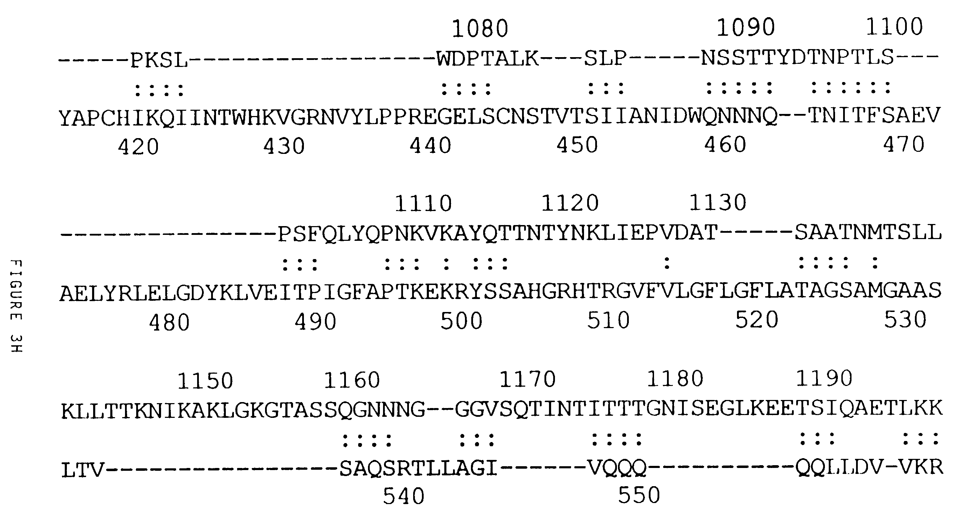

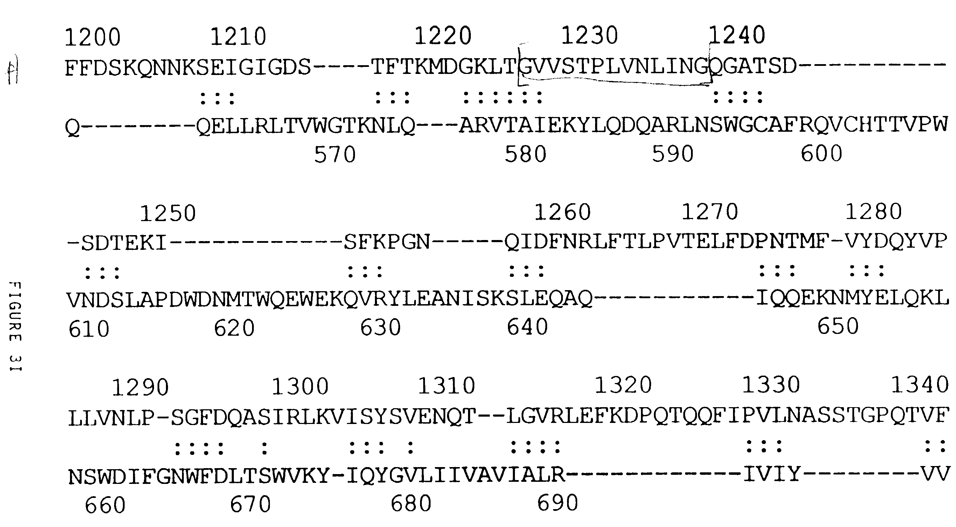

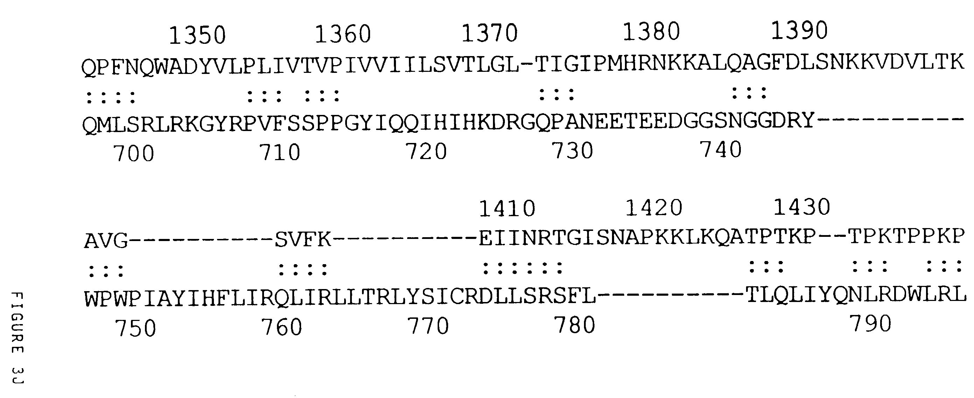

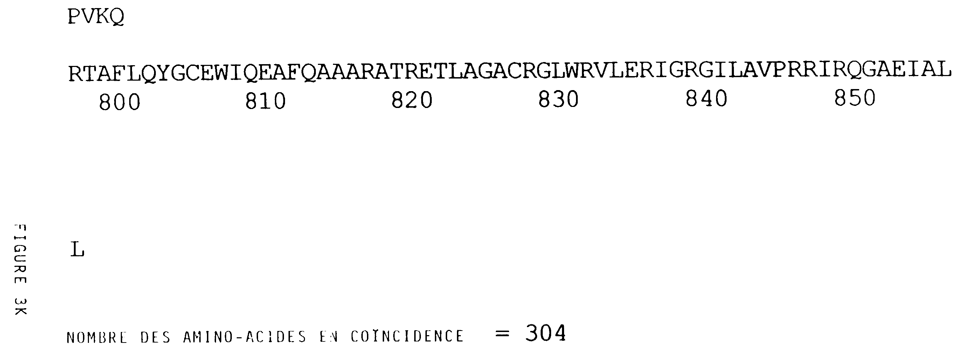

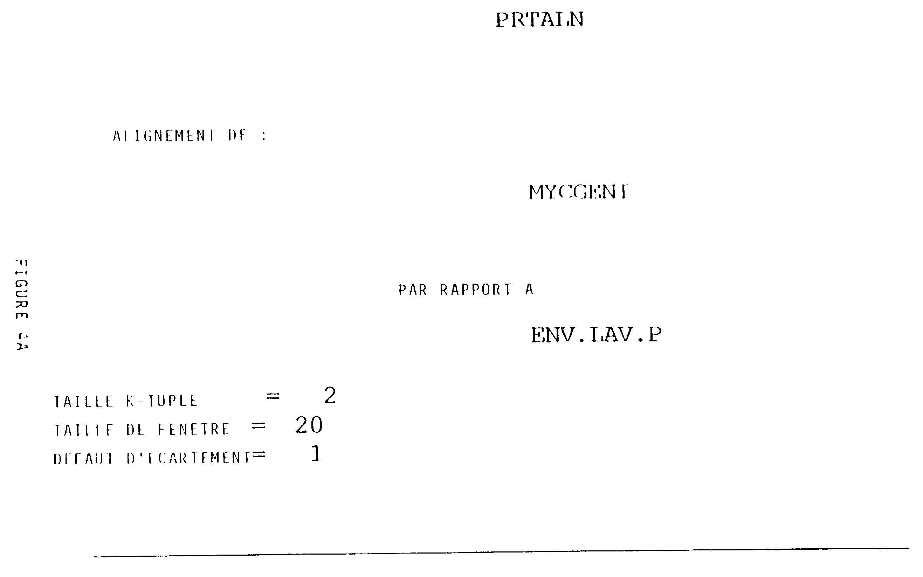

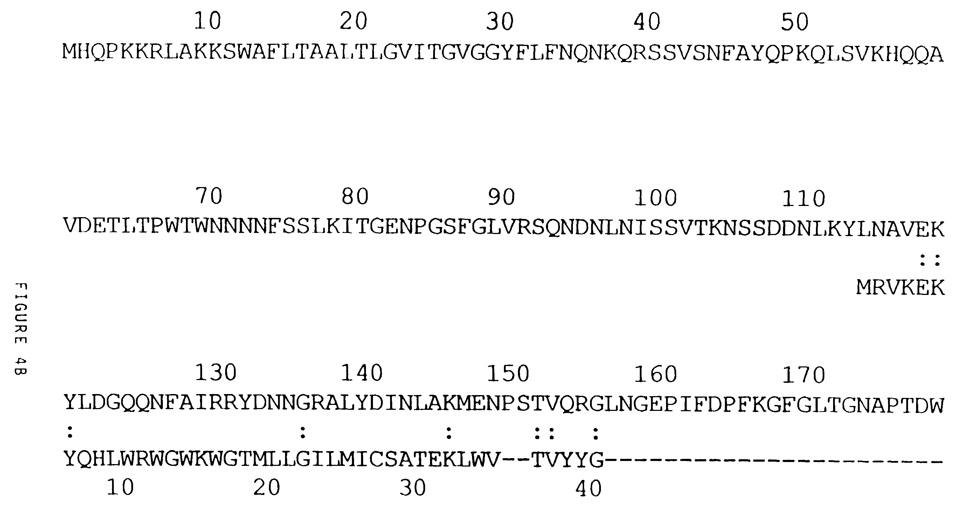

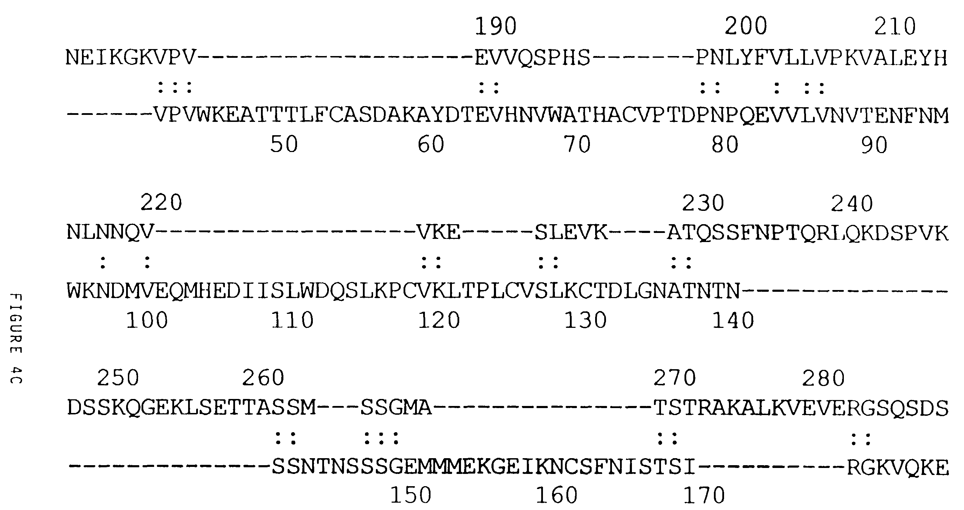

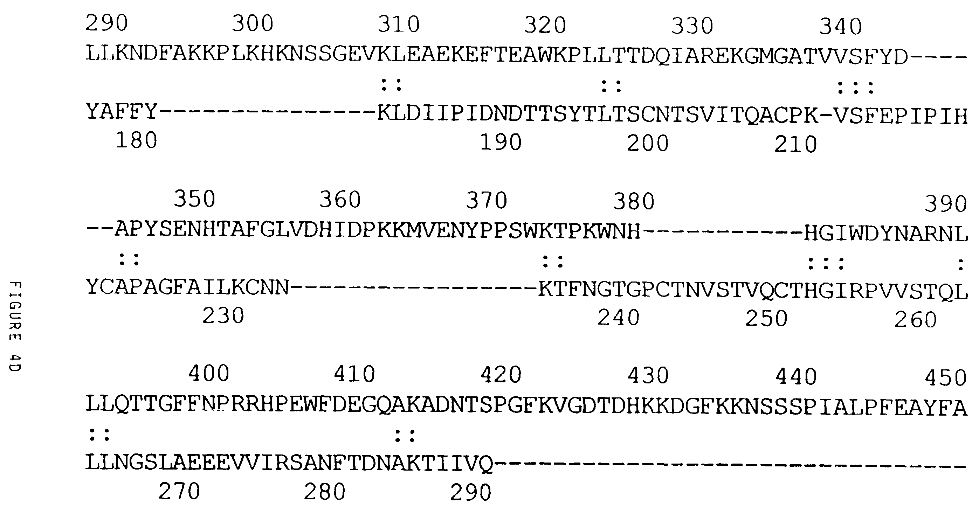

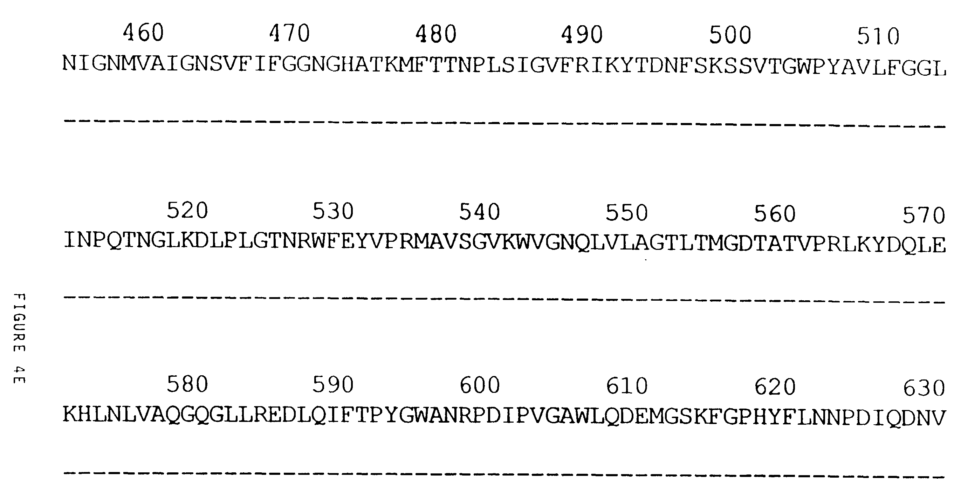

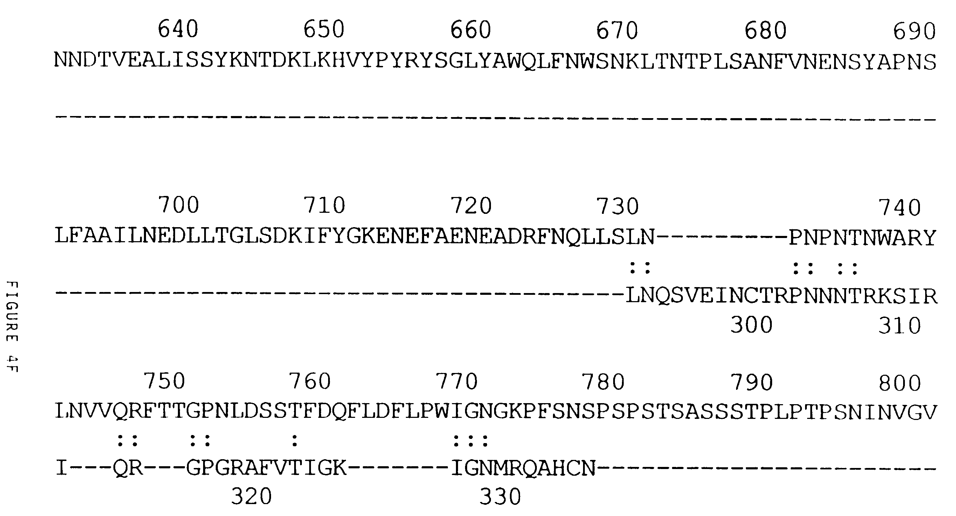

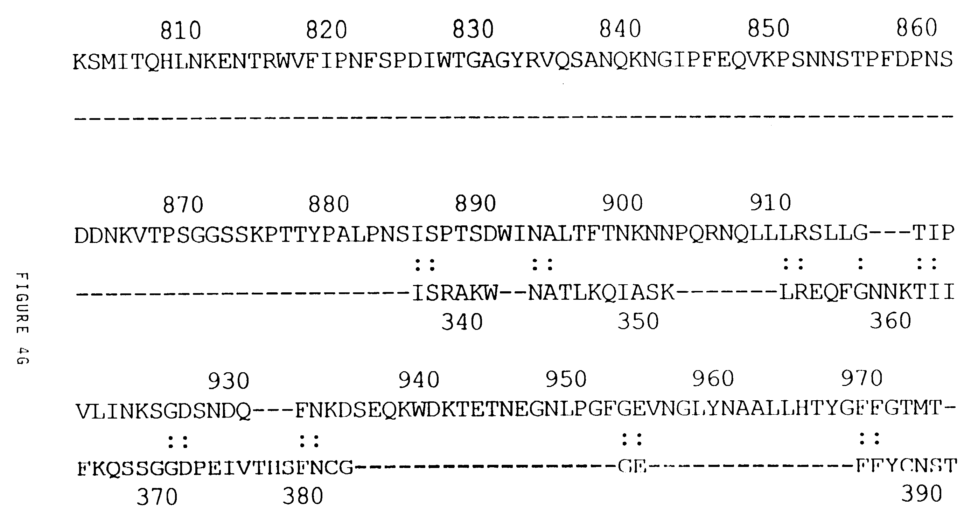

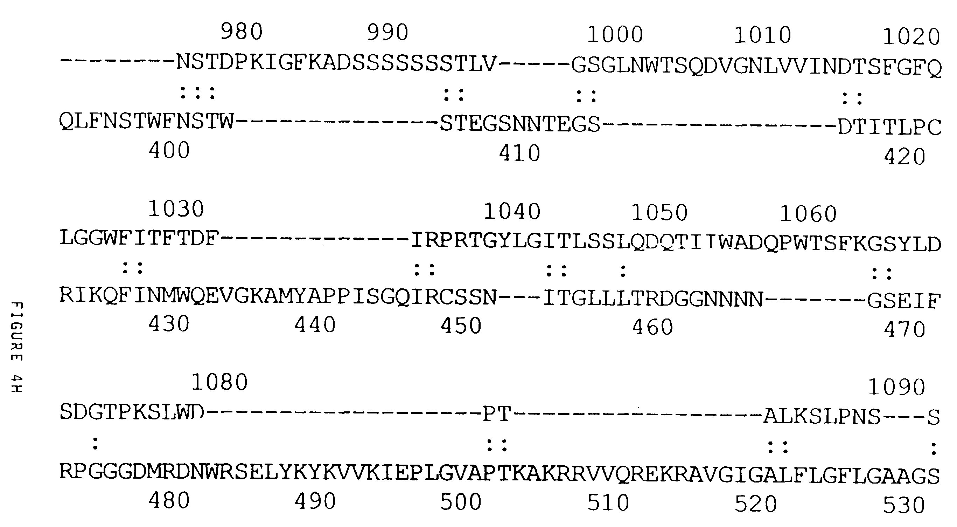

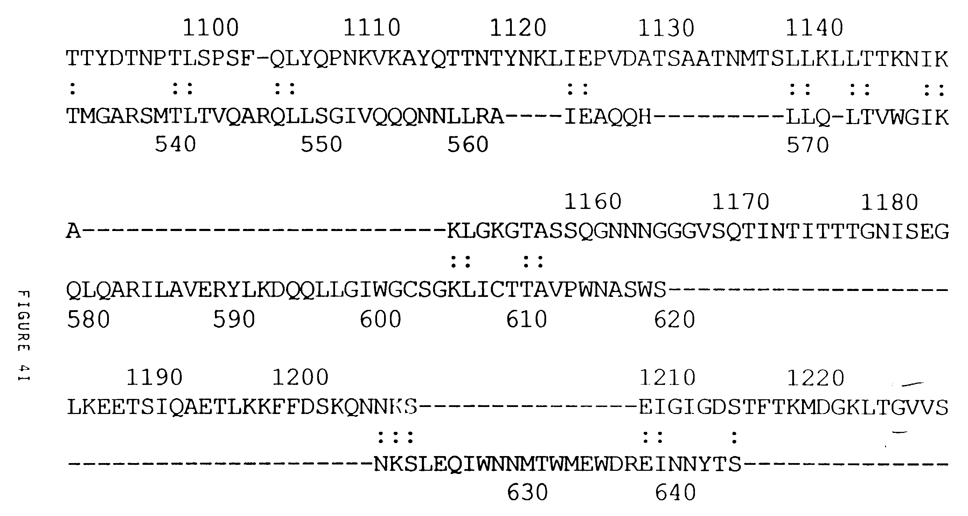

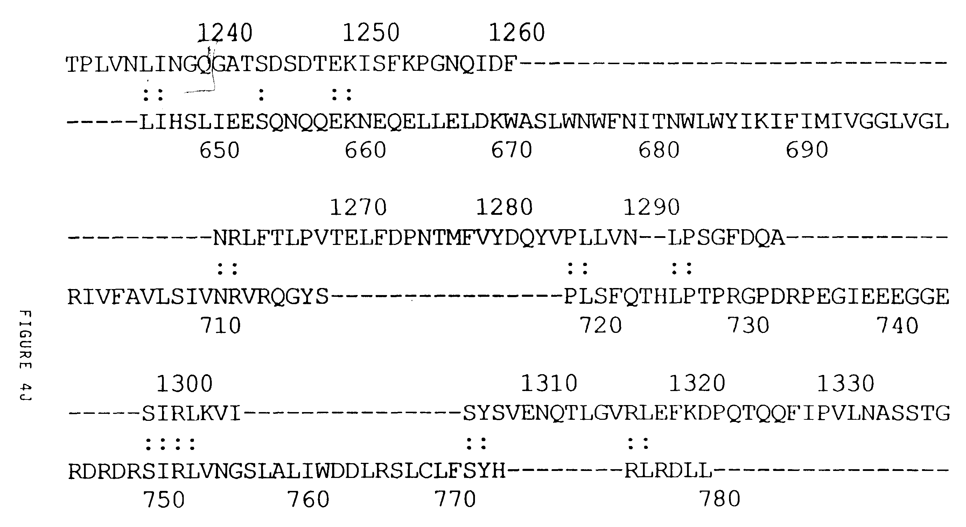

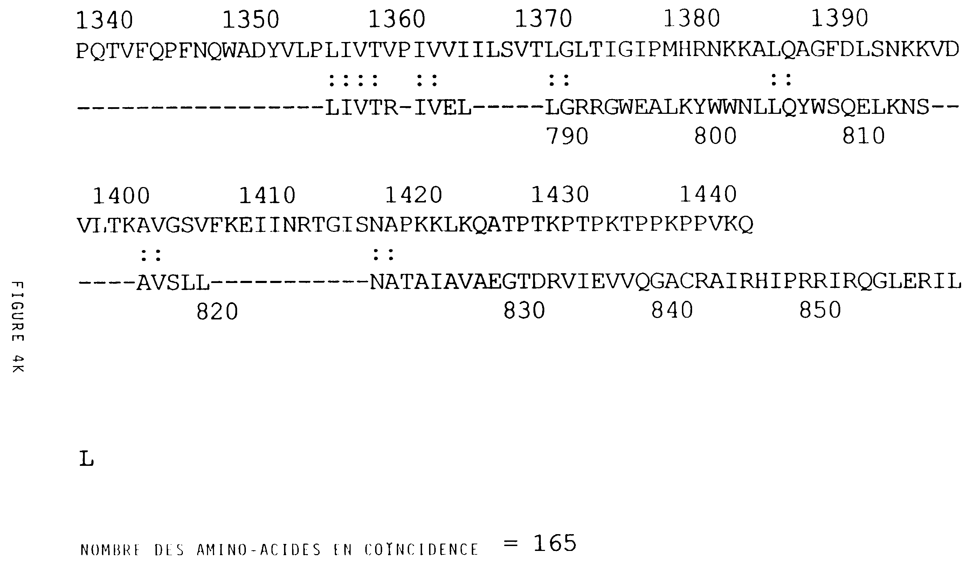

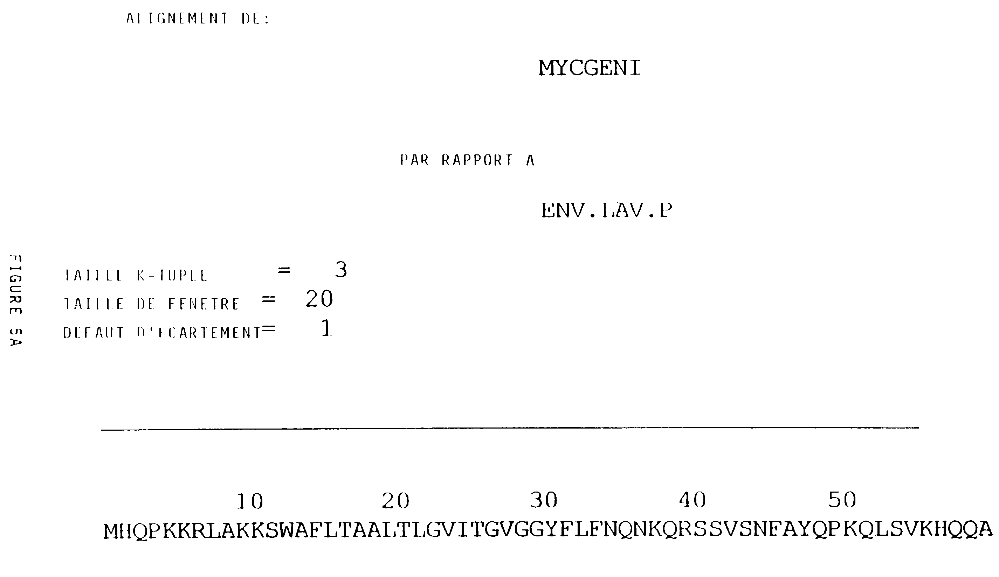

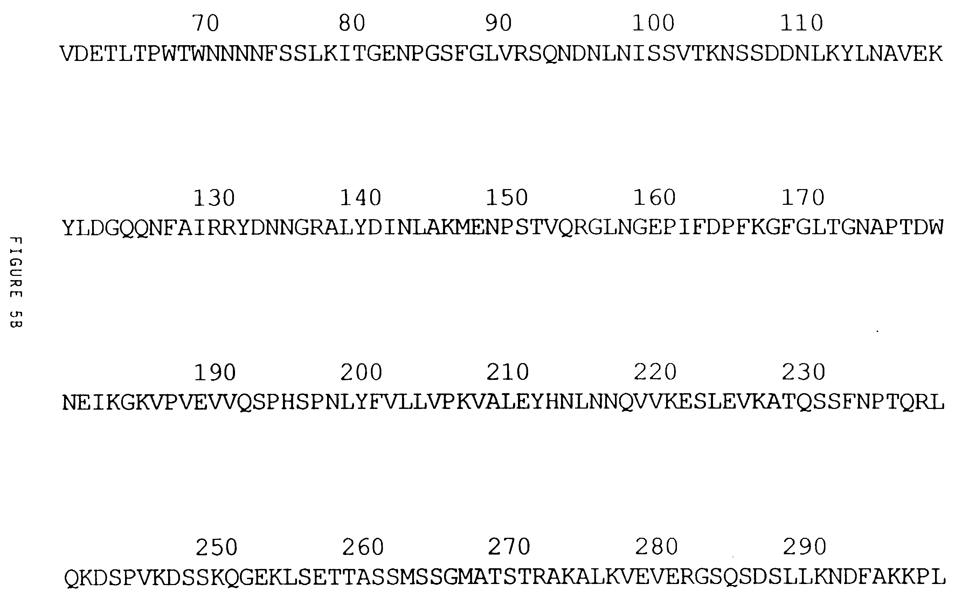

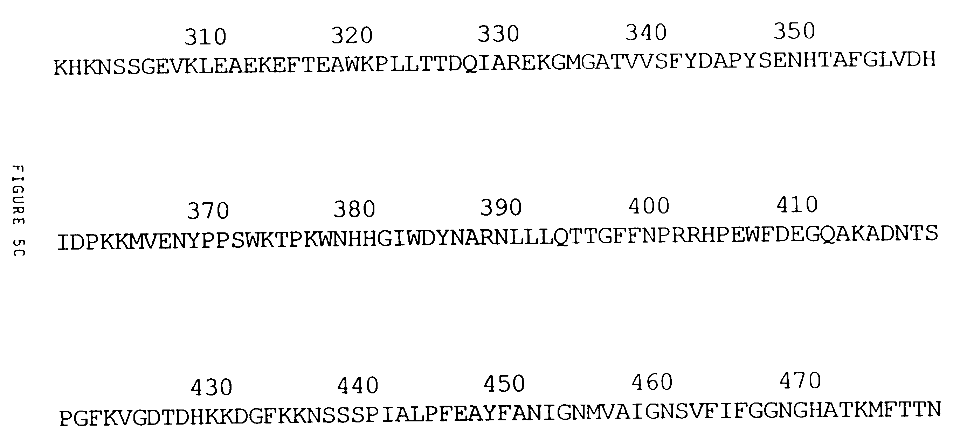

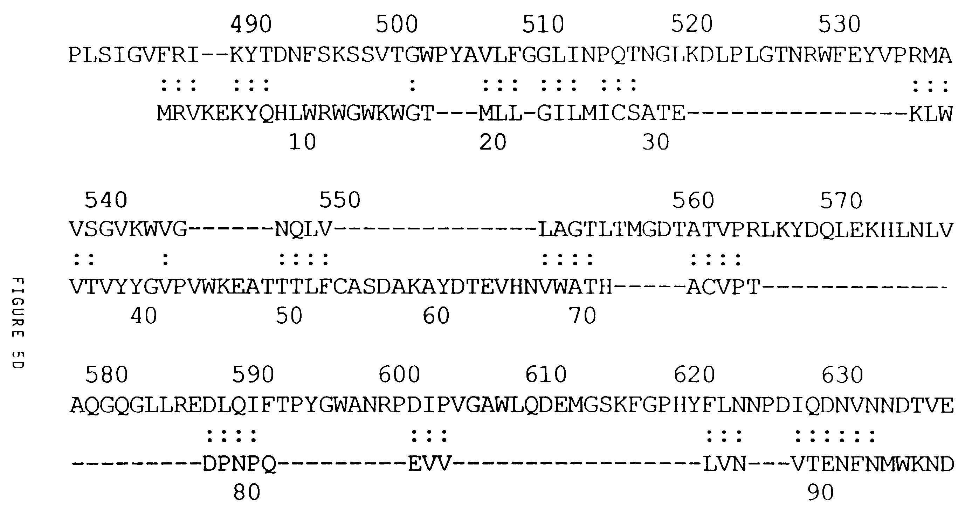

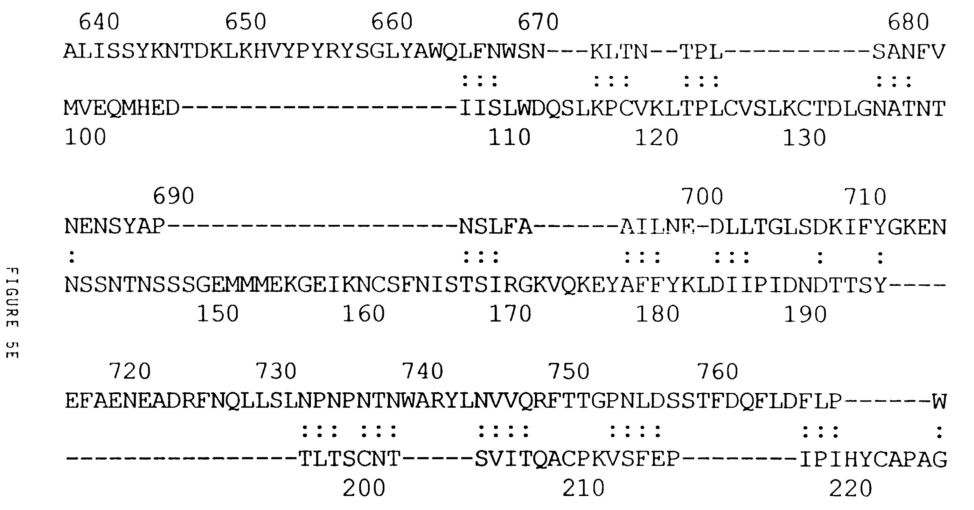

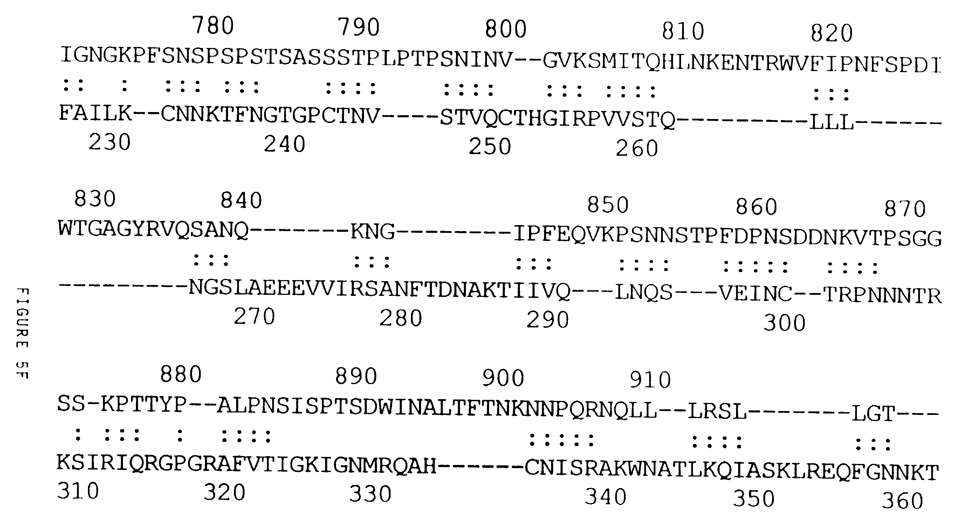

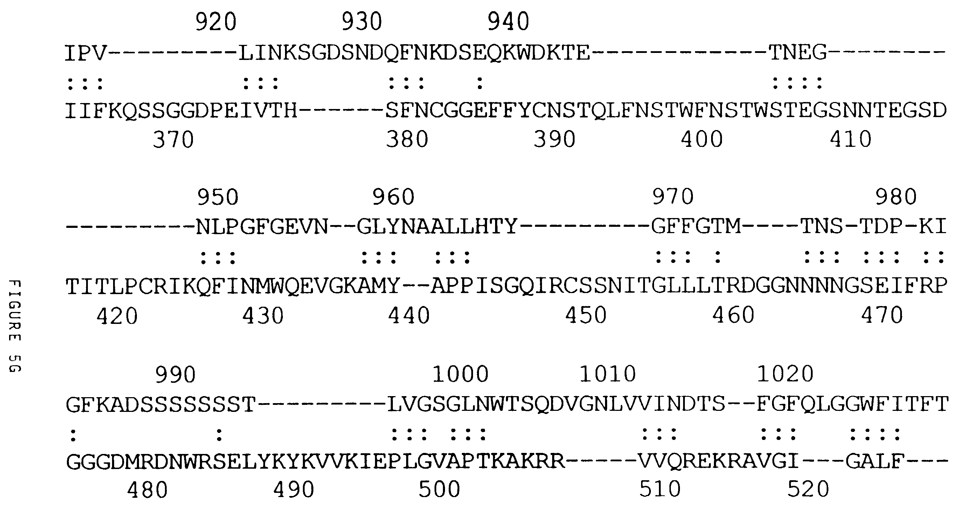

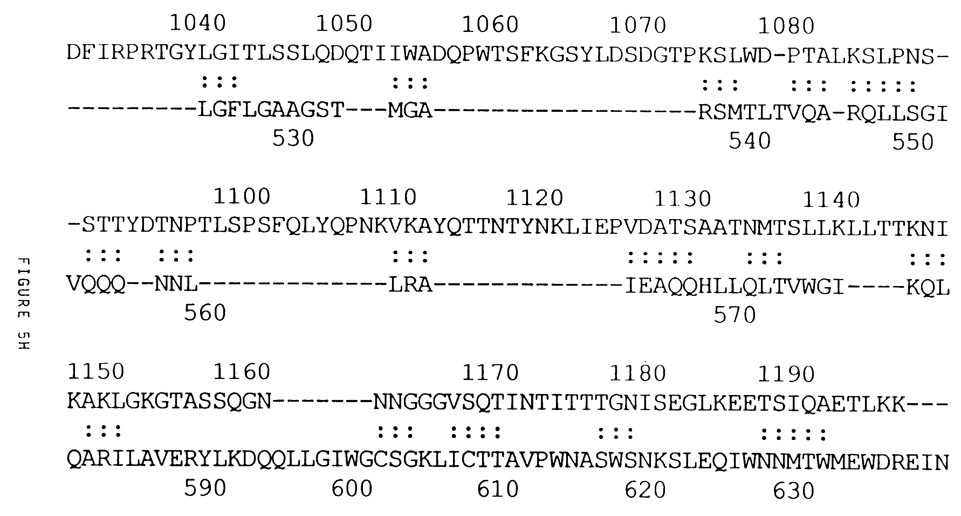

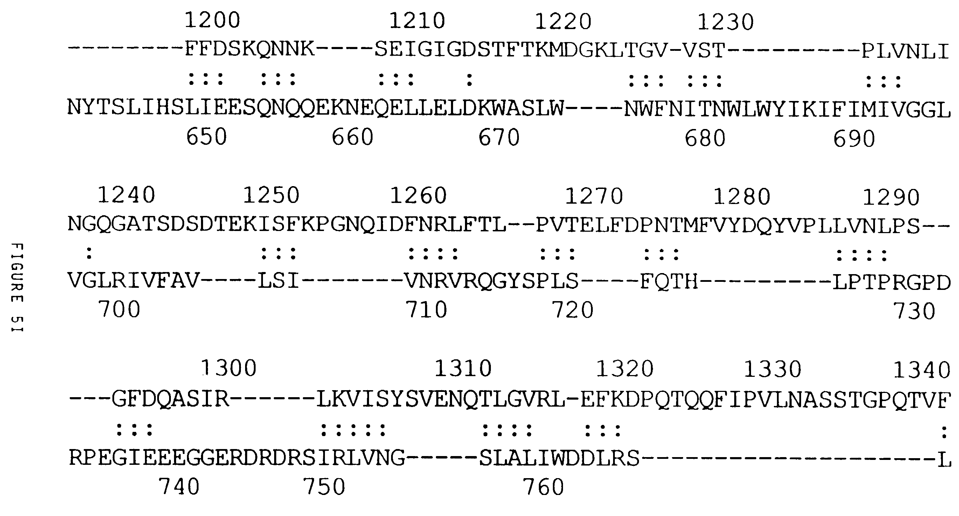

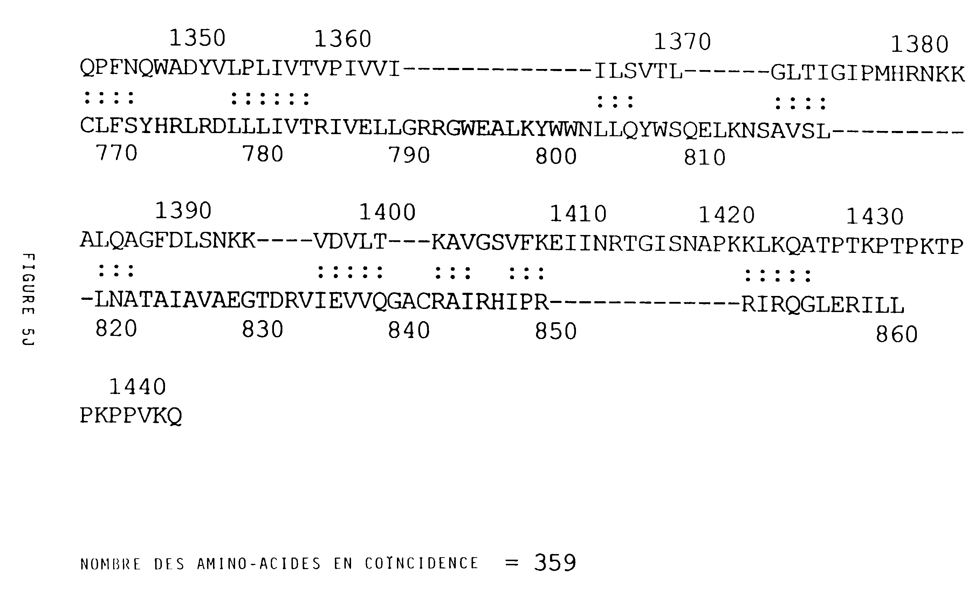

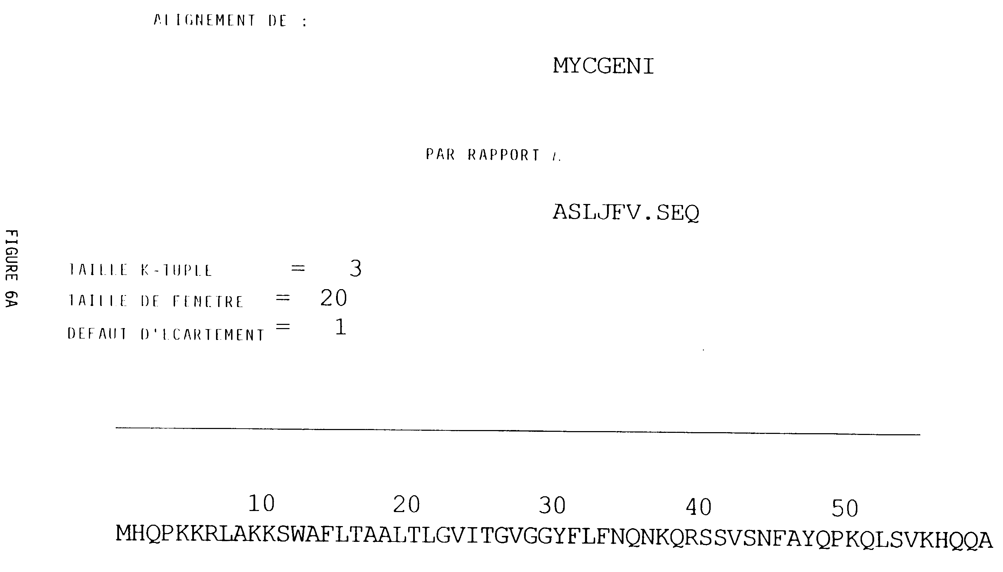

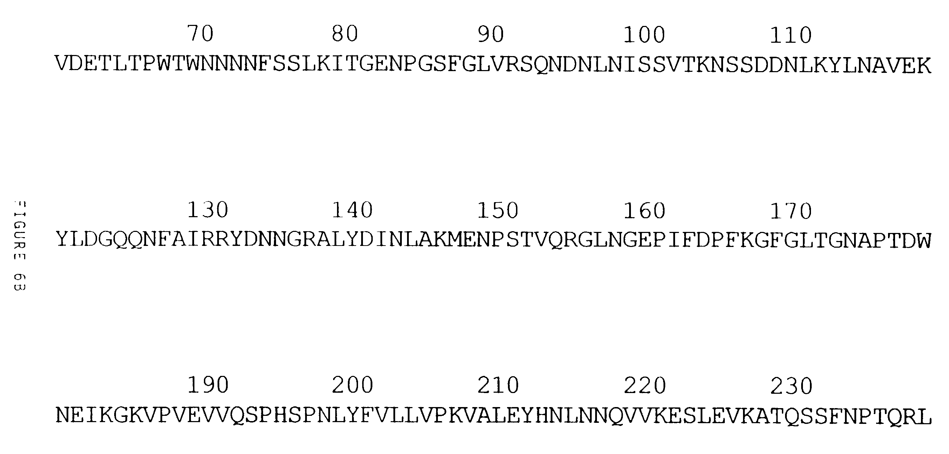

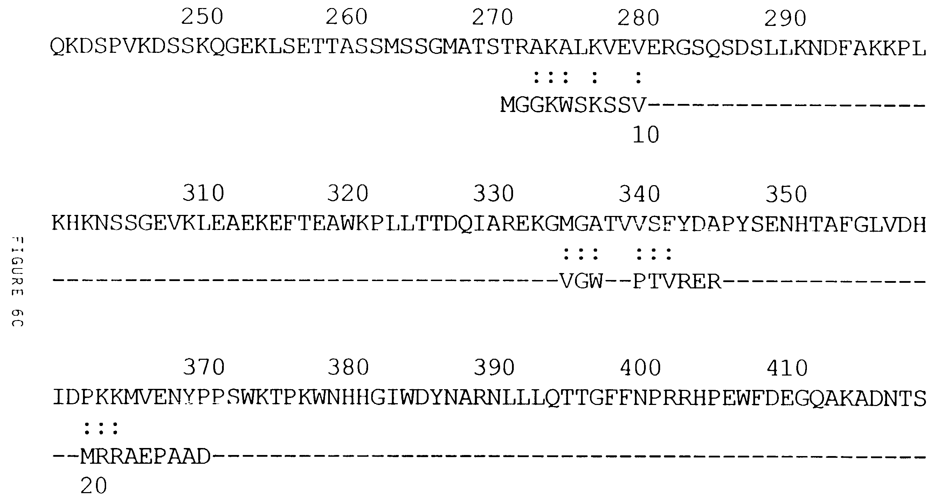

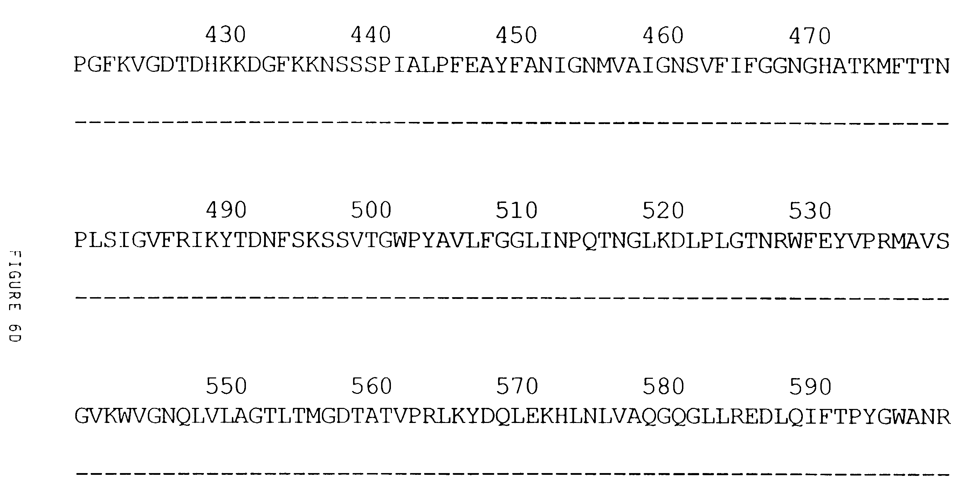

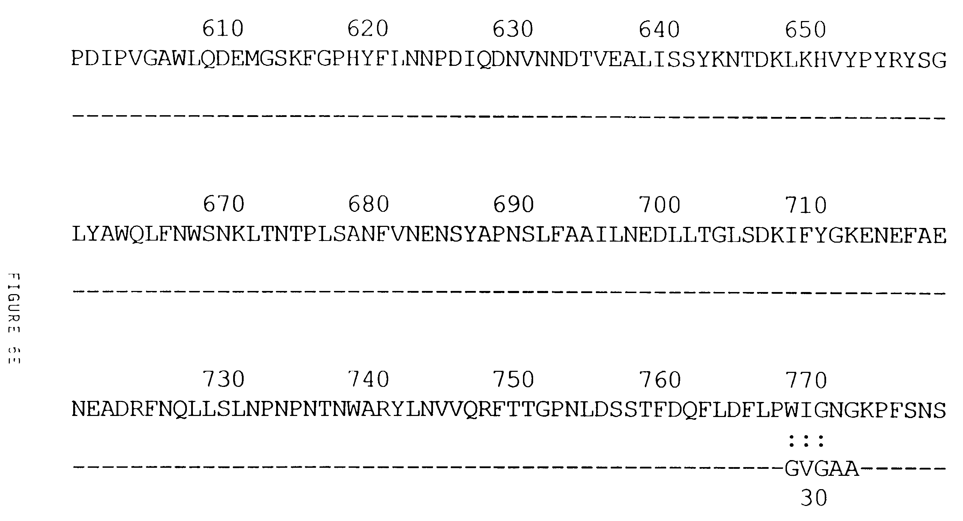

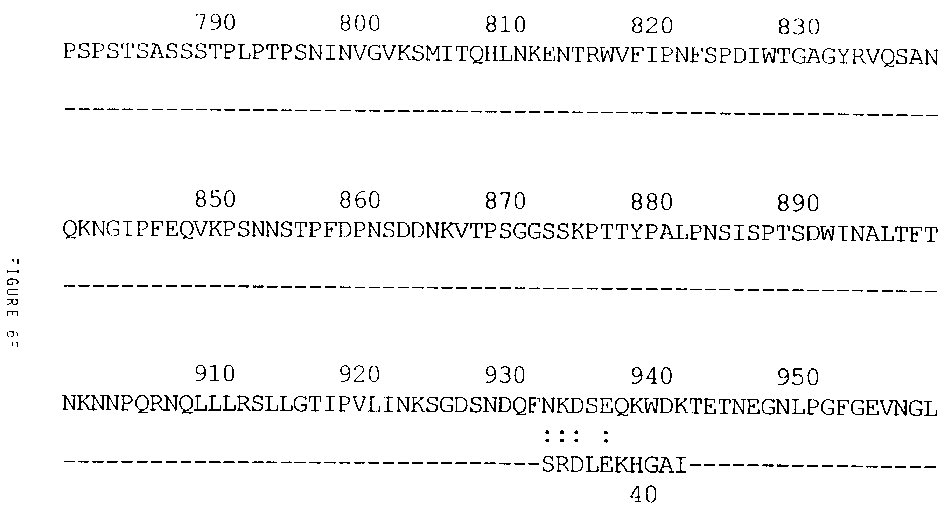

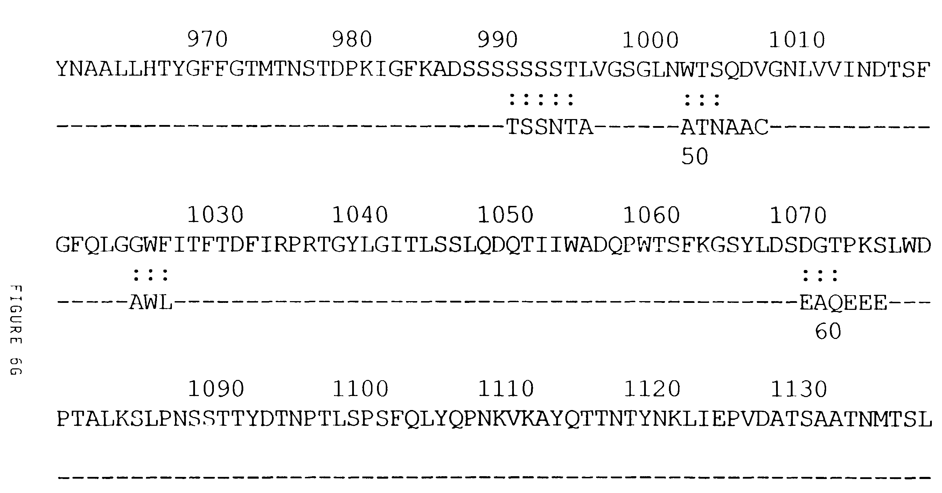

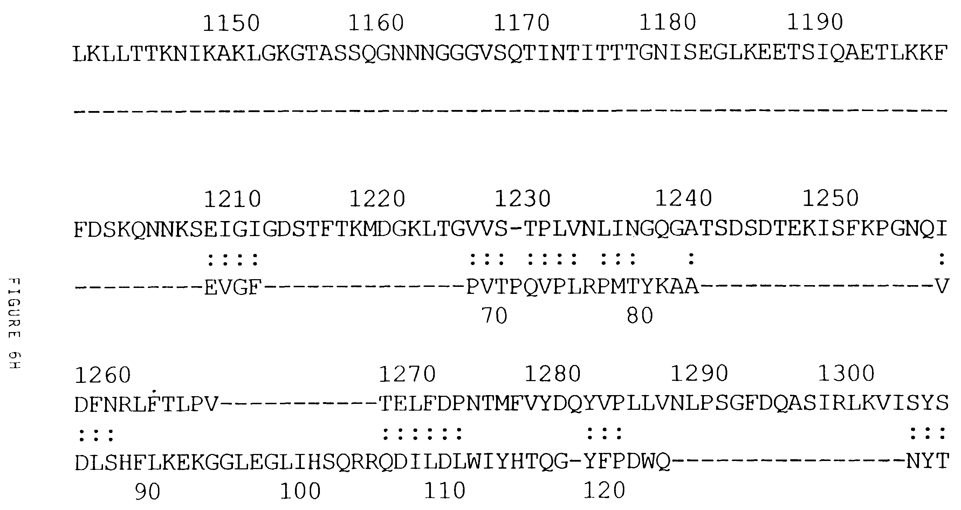

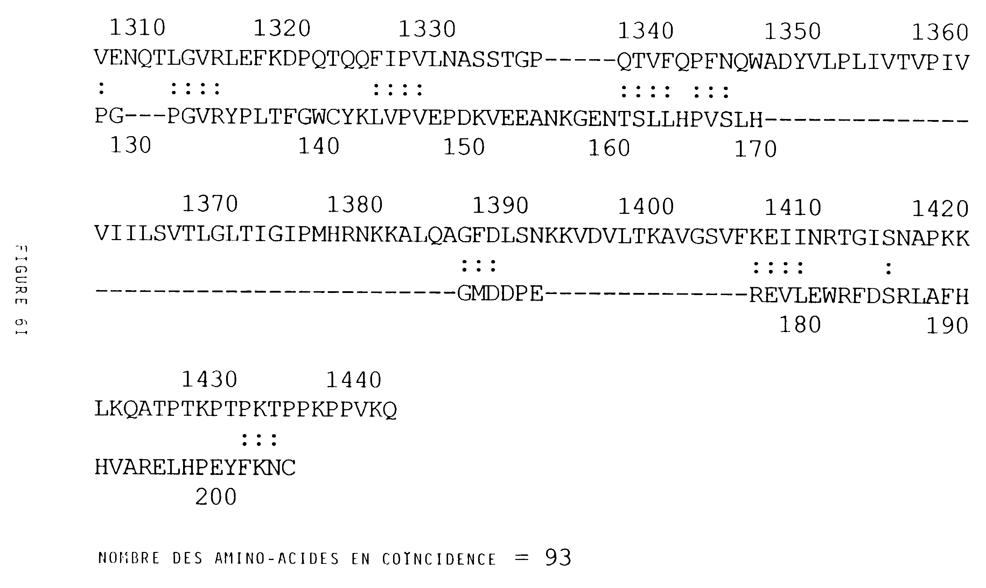

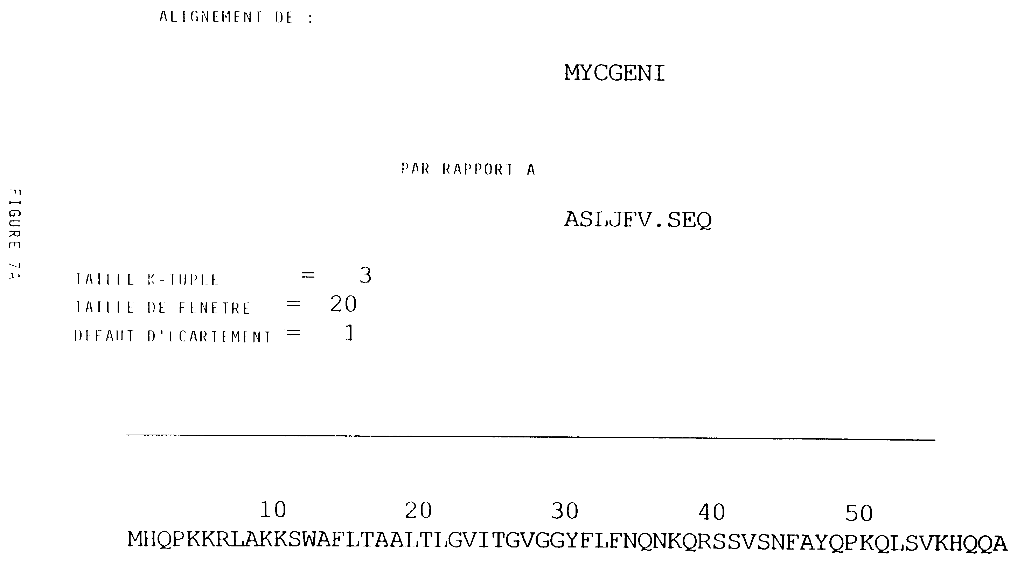

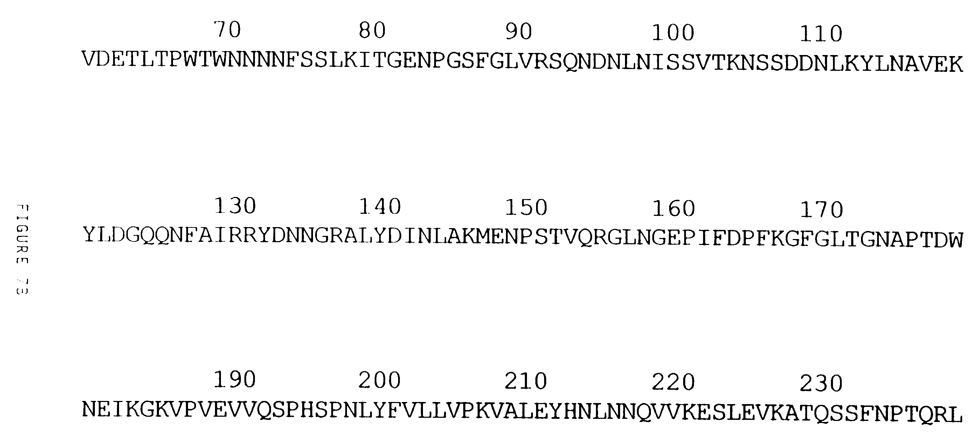

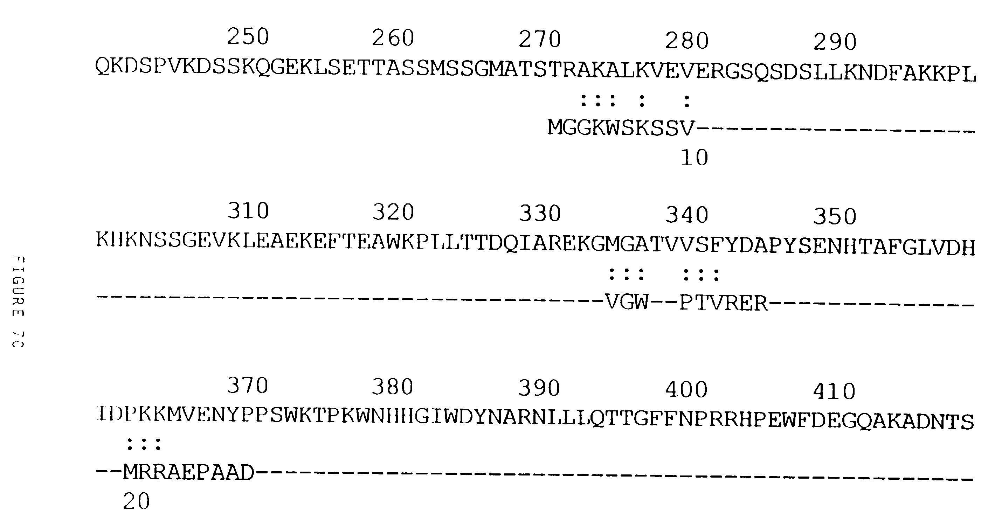

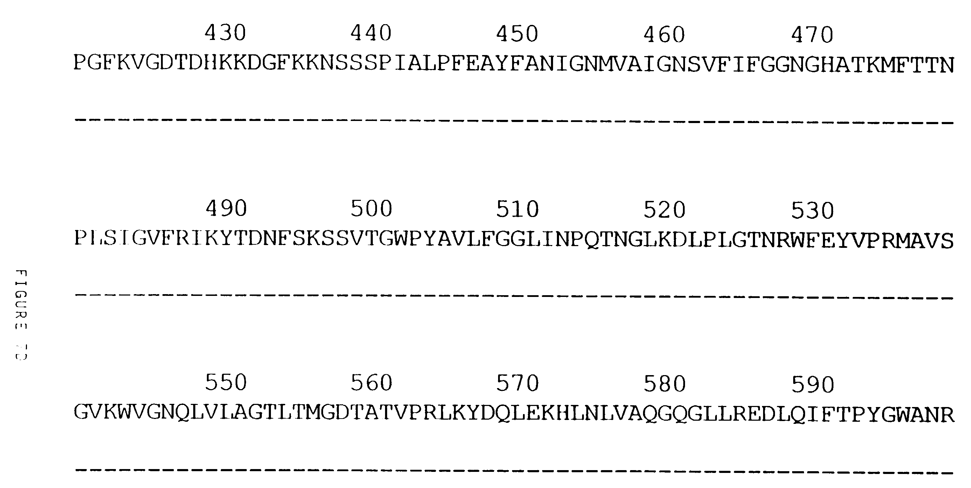

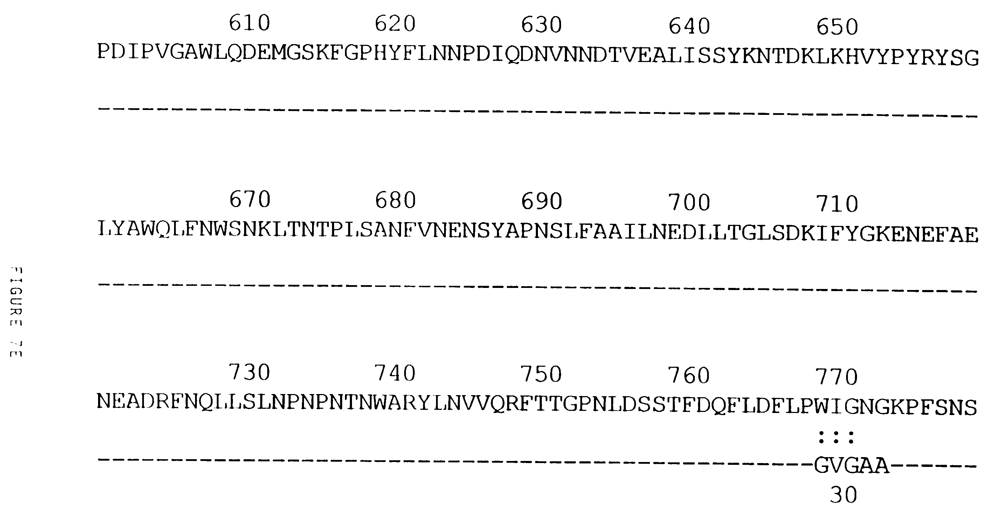

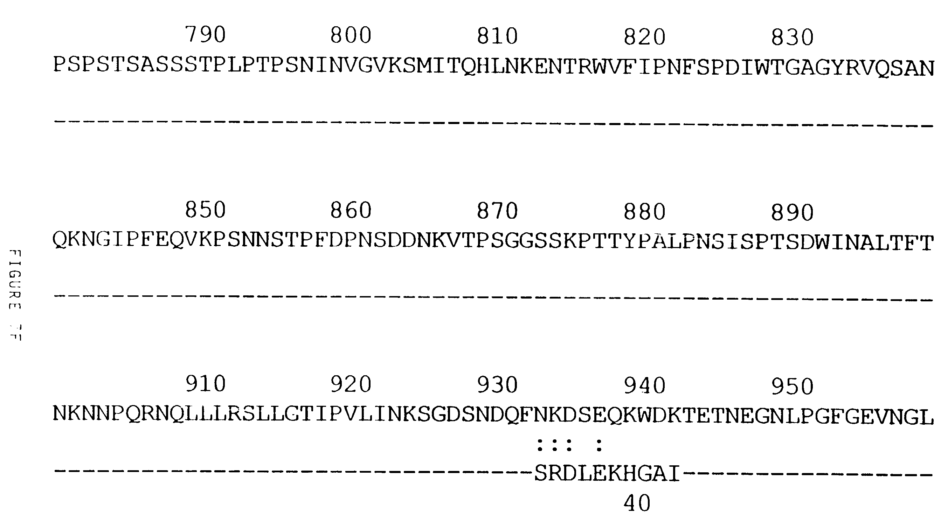

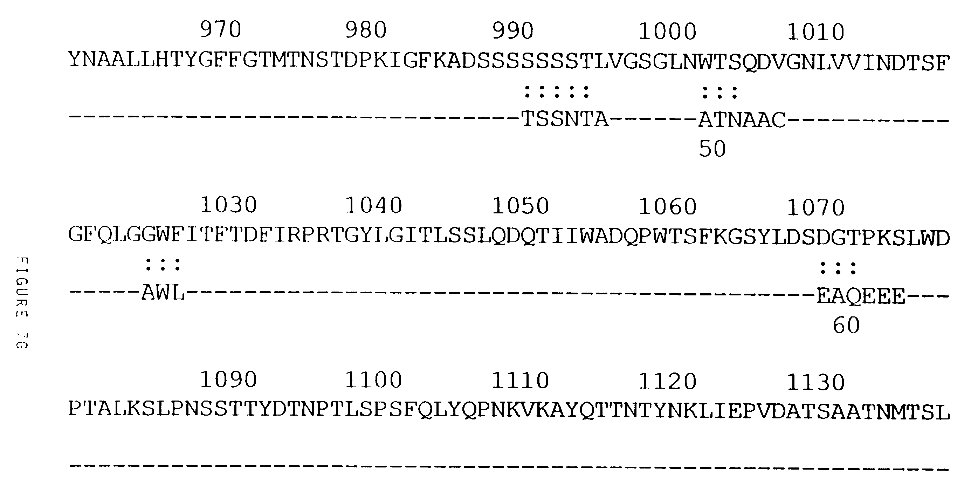

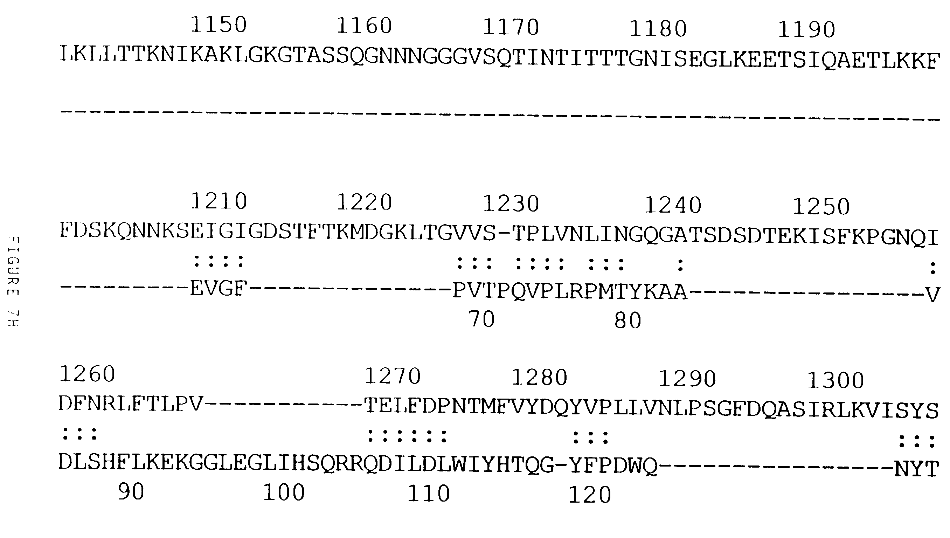

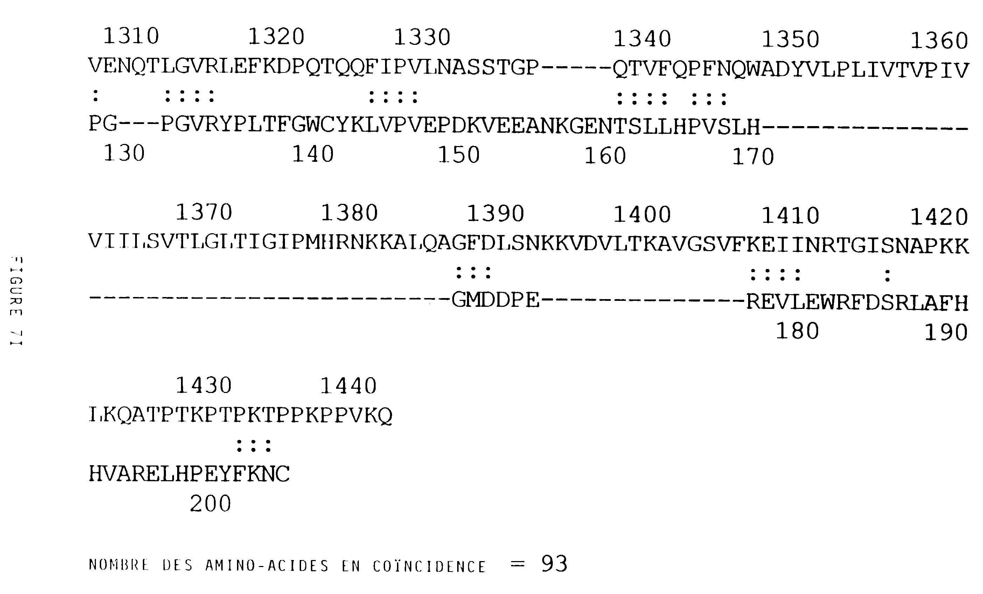

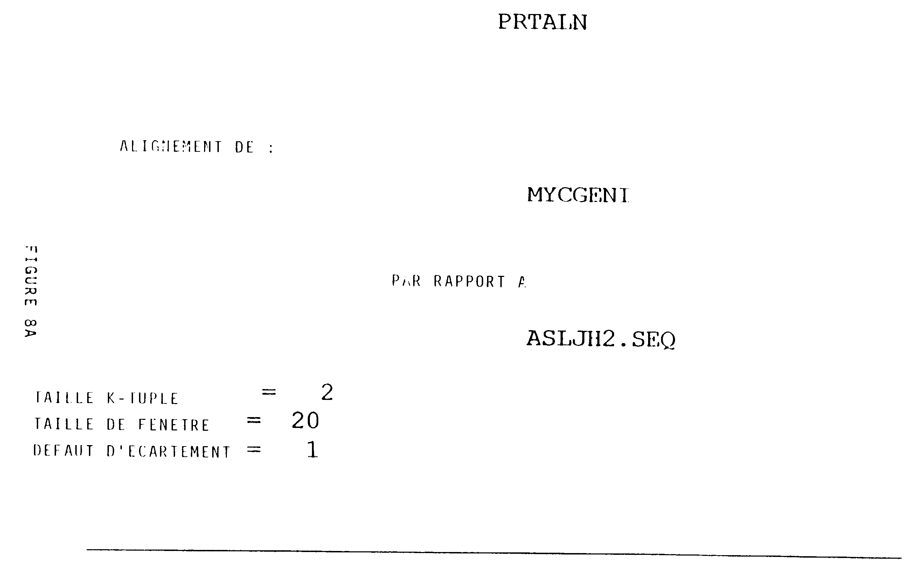

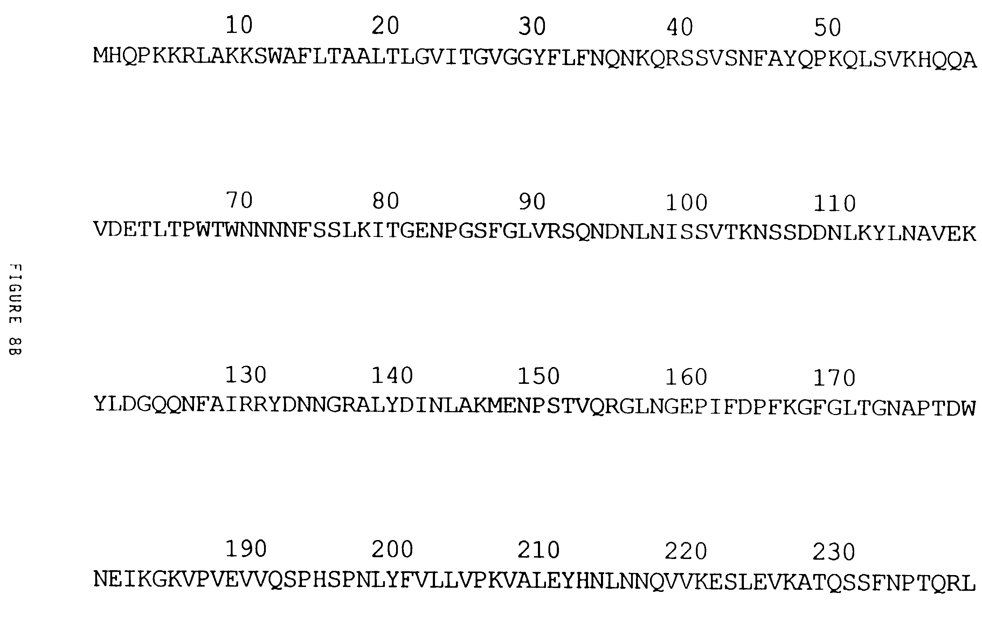

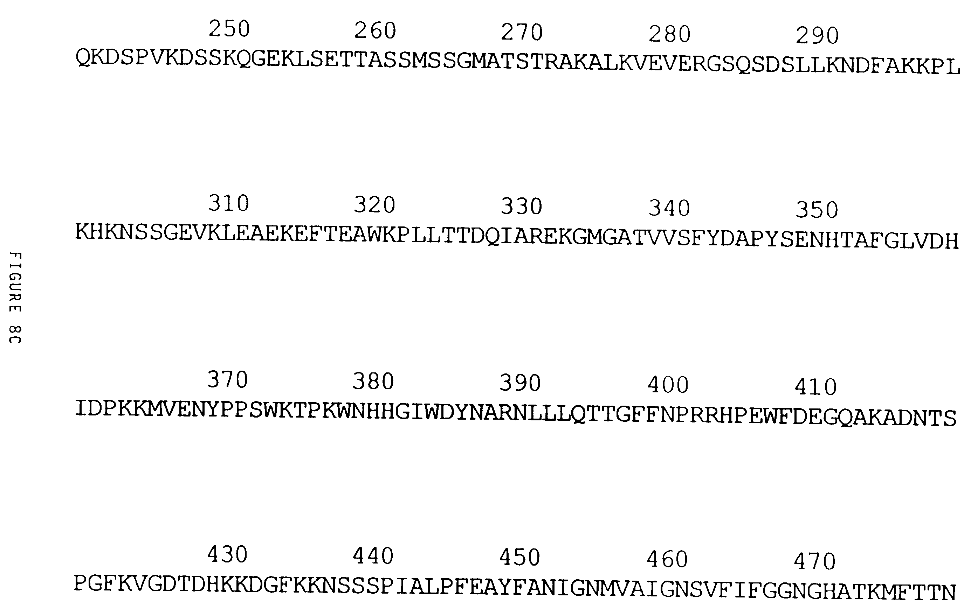

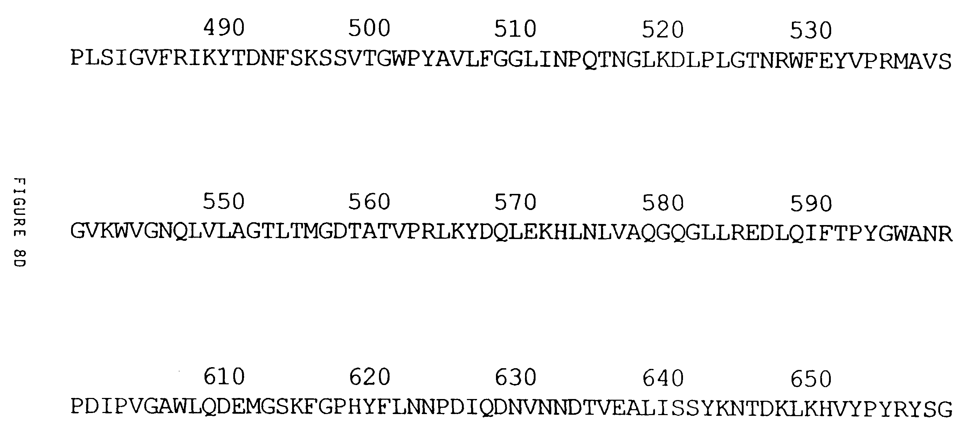

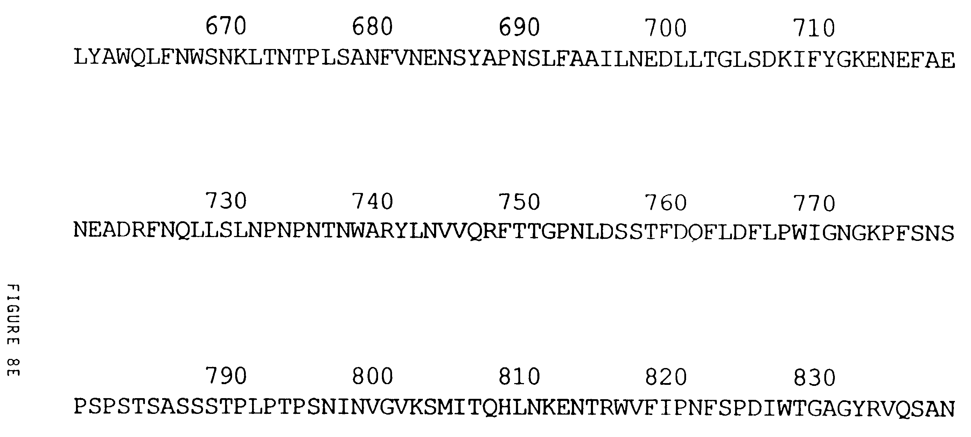

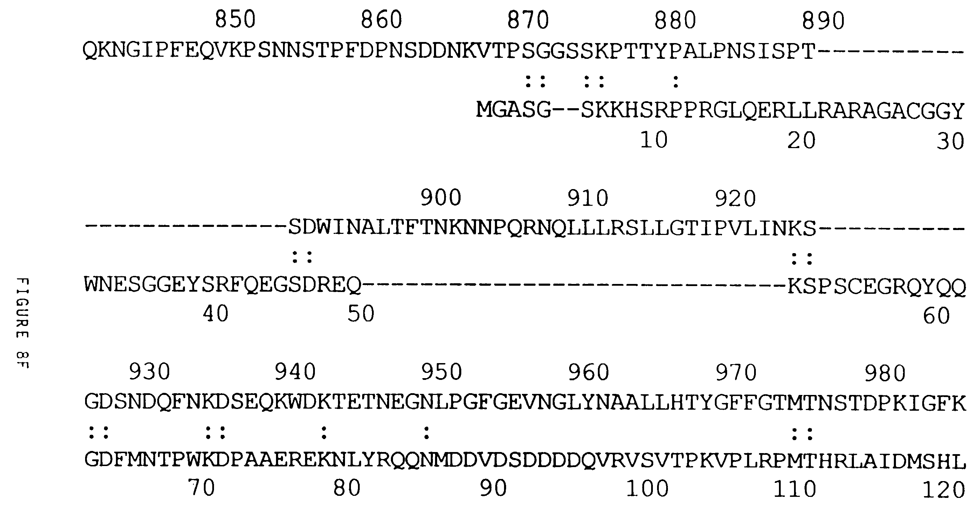

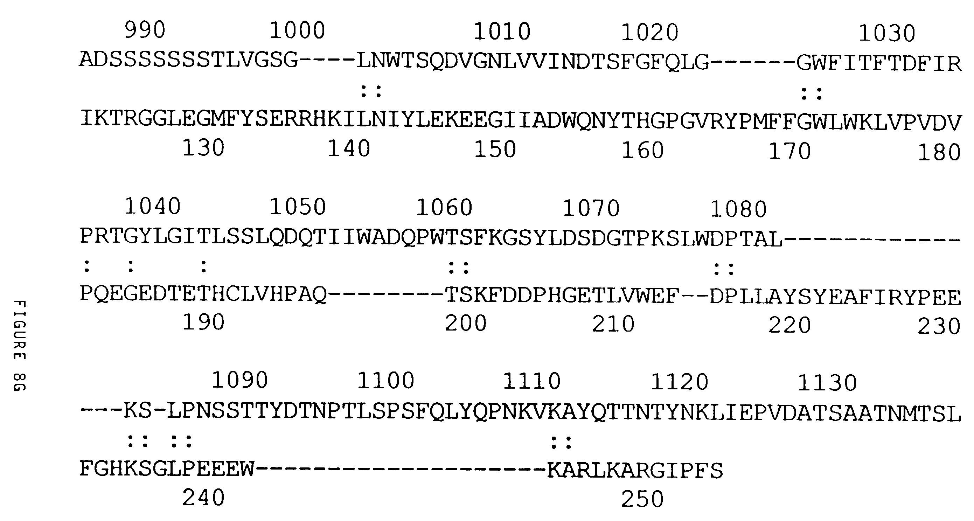

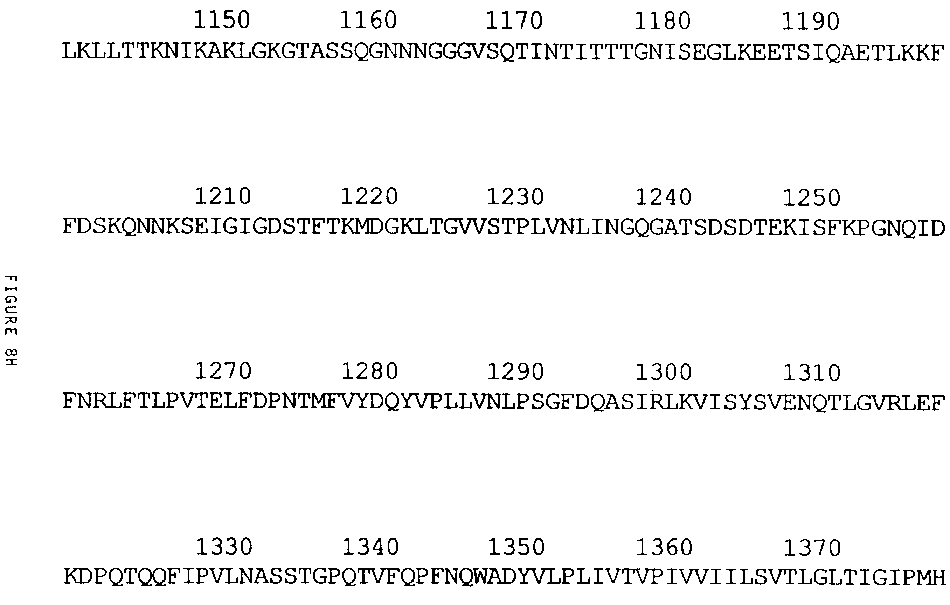

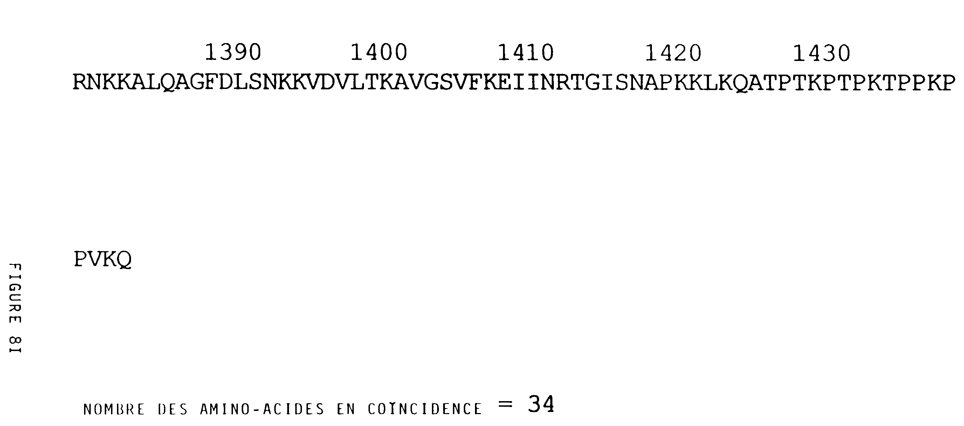

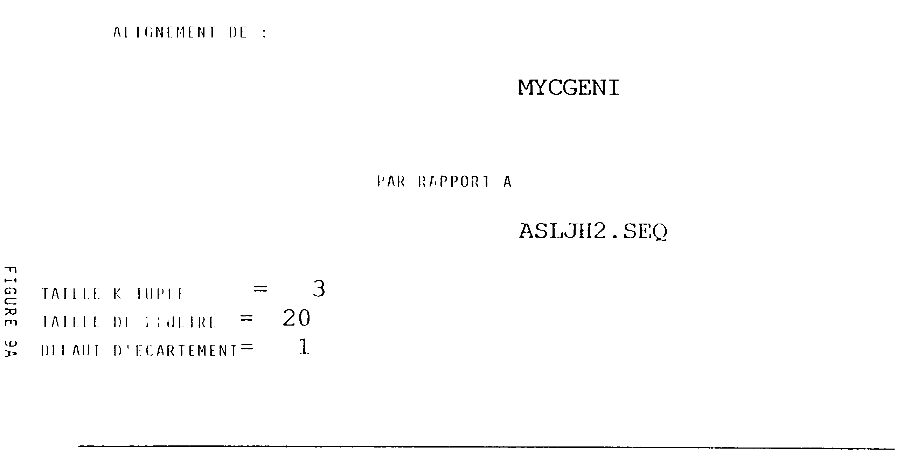

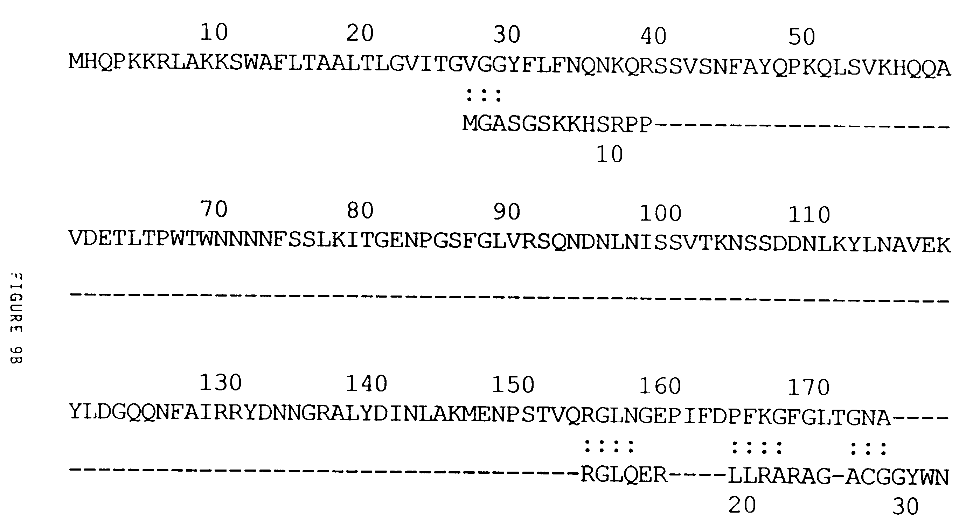

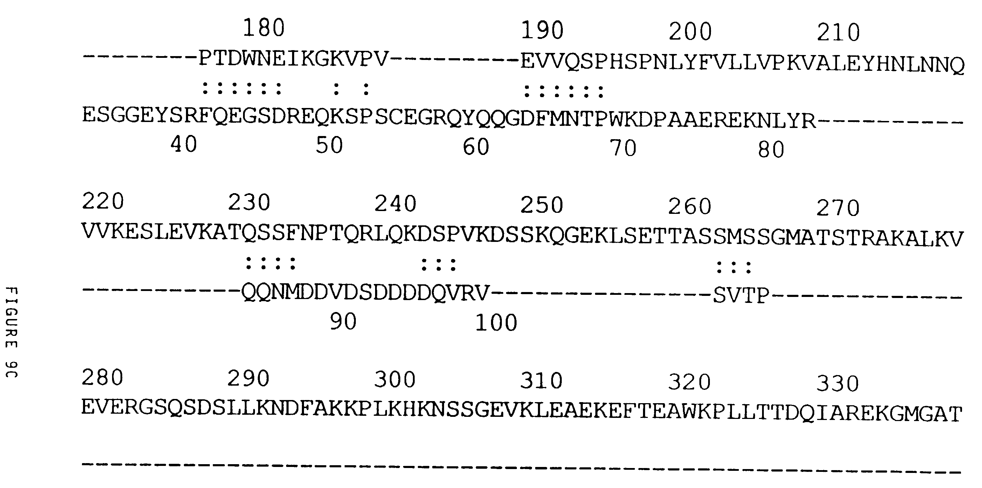

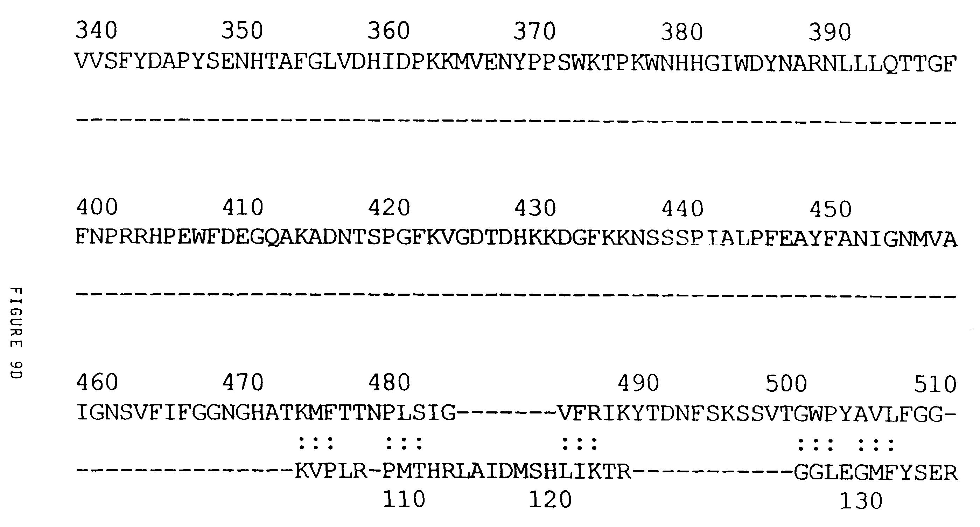

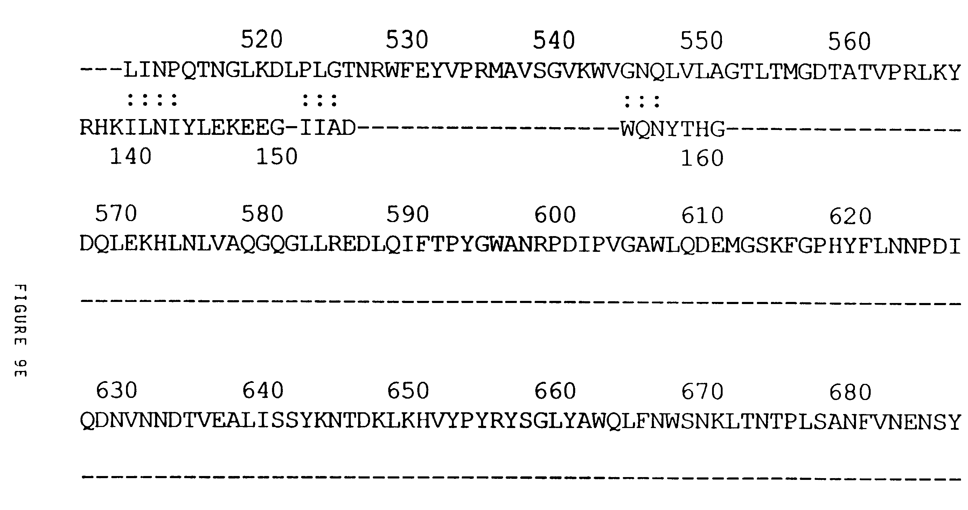

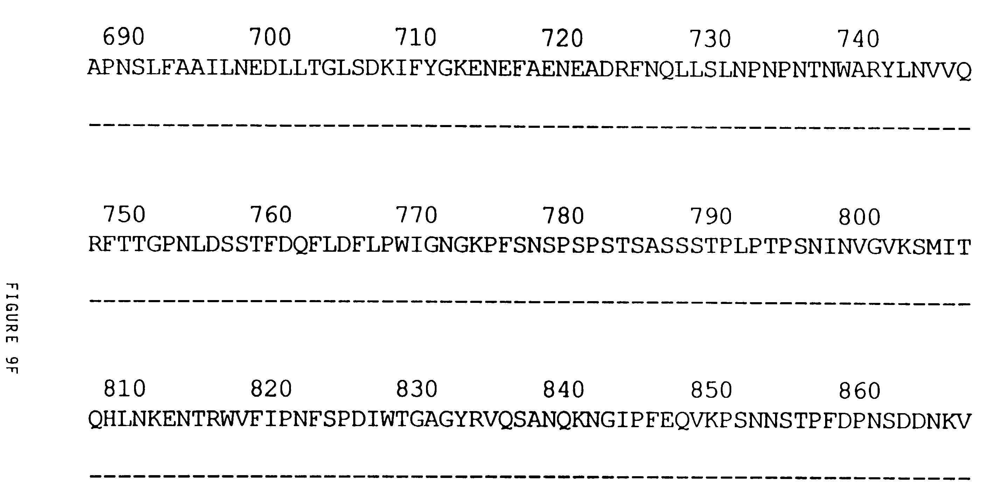

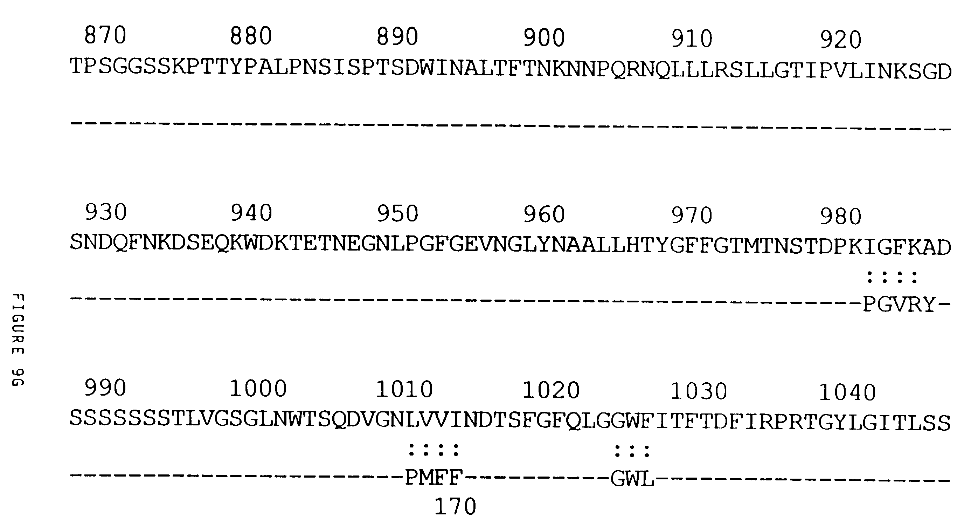

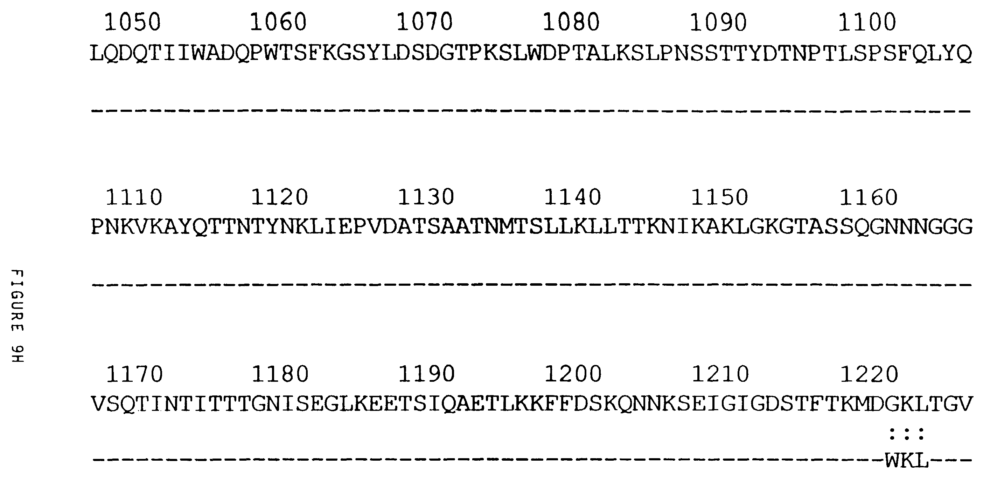

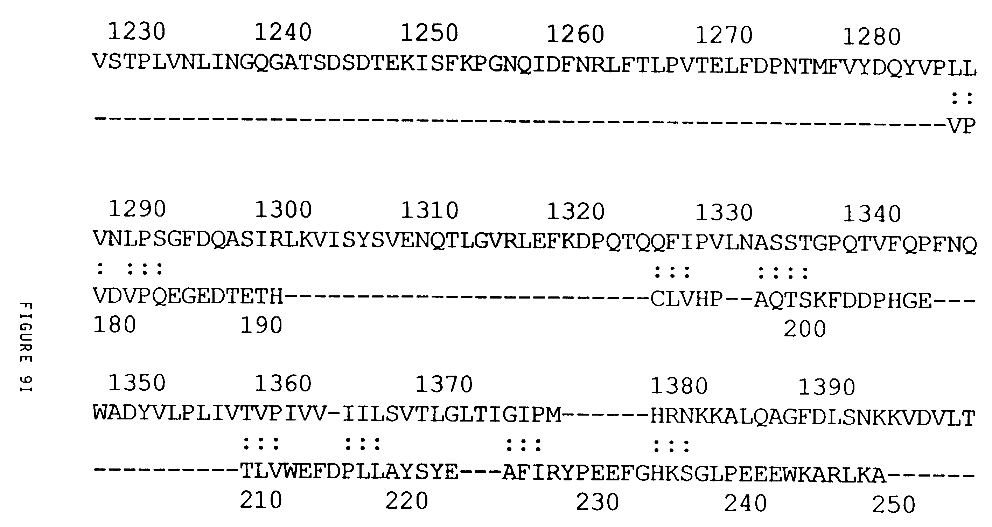

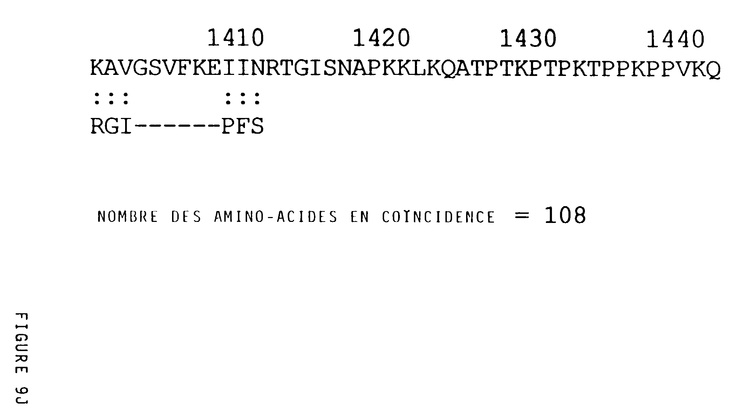

Les figures 2 à 9 regroupent les autres isologies et homologies obtenues pour HIV-1 et HIV-2.

### E) Homologies entre la p27 nef de HIV-1 et Mg Pa de M.genitalium

Une remarquable homologie de 90% existe pour une région de 9 acides aminés consécutifs conservés.

M.genitalium :     S K S S V T G W P
                    * * * * *   * * *
p 27 nef :          S K S S V V G W P

### F) Homologies entre la p55 gag de HIV-1 (BRU) et Mg Pa de M.genitalium

Aucune région d'homologie significative n'a pu être mise en évidence parmi les séquences étudiées.

L'invention concerne donc plus particulièrement les peptides ayant des séquences identiques à celles qui sont indiquées ci-dessus, qu'elles soient issues de HIV ou des mycoplasmes sus-indiqués. Elle concerne de même les peptides (plus longs ou plus courts) dont les séquences sont contenues dans celles des séquences les plus longues des susdits peptides, dès lors qu'ils correspondent aux susdites séquences mycoplasmiques, notamment celles issues de M. genitalium.

De même font encore partie de l'invention des peptides même encore plus courts, par exemple SKSSVTGWP dès lors qu'il sont eux-mêmes aptes
- soit (le cas échéant après leur couplage avec une molécule porteuse) à induire la production in vivo d'anticorps aptes à neutraliser la multiplication de retrovirus dans les cultures,
- soit à interférer eux-mêmes avec cette multiplication.

L'invention concerne encore les peptides qui se distinguent des précédents, par application des règles d'isologie dont il a été question plus haut, par exemple des peptides dans lesquels ceux des acides aminés présents qui appartiennent à des familles isologiques telles que définies plus haut peuvent être interchangés avec d'autres acides aminés appartenant respectivement aux mêmes familles isologiques.

Il résulte de ce qui précède - dans les limites des investigations qui ont été conduites à ce jour - que M.genitalium ou M.pirum ou de mycoplasmes apparentés sont une source préférée de séquences peptidiques utilisables pour les applications préférées de l'invention, notamment l'inhibition de la propagation de HIV dans les milieux biologiques qu'il sont susceptibles d'infecter.

Les peptides issus de protéines de mycoplasmes ou d'une autre source, dès lors qu'ils contiennent des séquences identiques ou semblables sont ceux qui induisent la synthèse d'anticorps inhibant in vitro l'infection à HIV quel que soit le degré d'homologie ou d'isologie à l'égard d'HIV.

L'invention concerne aussi les anticorps, polyclonaux ou monoclonaux, spécifiquement dirigés contre les susdits peptides. Des anticorps préférés sont ceux qui reconnaissent spécifiquement les séquences des régions d'adhésion des protéines adhésines de mycoplasme et en particulier pl de M.pneumoniae, de M.genitalium, ou de M.pirum.

Des anticorps formés contre de tels peptides, notamment contre le peptide A ont inhibé à 90% l'infection, par la souche prototype HIV-1 (BRU), d'une culture de cellules mono-nucléées de sang périphérique en majorité des lymphocytes T activés par la phytohémagglutinine (PHA). En présence de ces anticorps aucune formation de syncitia n'est observée. Des résultats identiques ont été obtenus avec HIV-2 (ROD). Ceci est un résultat intéressant pour la compréhension de la pathogenèse de l'infection à HIV dans la mesure où la prévention de cette infection n'impliquerait pas seulement le HIV-1 et HIV-2 mais également un éventuel co-facteur ou un agent opportuniste tel qu'un mycoplasme spécifique qui "faciliterait" le cas échéant l'infection par les HIV.

De telles préparations d'anticorps, dirigées contre une séquence peptidique correspondant probablement au site d'interaction de Mycoplasma genitalium ou d'un autre mycoplasme, par exemple M.pirum, avec les cellules eucaryotes, inhibent l'infection de lymphocytes T activés ou des cellules de la lignée CEM par la souche protype du HIV-1, BRU (11).

La séquence complète de l'adhésine Mg Pa de M. genitalium est indiquée sur la ligne supérieure figurant aux figures 2 à 9.

Ces résultats sont le mieux expliqués par l'effet neutralisant de l'anticorps ainsi obtenu sur la fixation cellulaire d'un mycoplasme accompagnant cette souche virale. Cela signifierait que ce mycoplasme jouerait un rôle-clé dans le déclenchement de la réplication du virus.

### 1°/ Choix d'une séquence immunisante préférée

Peu de séquences protéiques de mycoplasmes ont été déterminées à partir des gènes clonés et celles qui sont connues révèlent une grande variabilité d'une espèce à l'autre.

Cependant pour au moins deux espèces parasites de l'homme, M.pneumoniae et M.genitalium, des protéines d'adhésion (adhésine) de masse moléculaire élevée ont été caractérisées (12) et leurs séquences publiées (6) (15).

La comparaison de ces séquences en acides aminés révèle une certaine homologie (50%), notamment au niveau de la séquence supposée être impliquée directement dans la fixation de M.pneumoniae à des eucaryotes (12).

Cette séquence est la suivante (peptide B) pour M.pneumoniae:

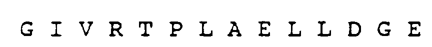

Nous avons, après alignement, retrouvé une séquence homologue (50%) sur la séquence publiée de M.genitalium (peptide A) :

G I V R T P L A E L L D G E
*   *   * * *     *     *
G V V S T P L V N L I N G Q (peptide A)

Deux variantes du peptique A consistent en ce qu'elles comportent les séquences suivantes :

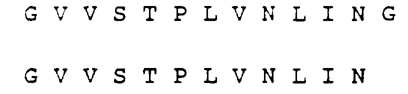

Un épitope particulier consiste en la séquence :

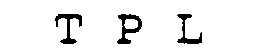

Ce peptide fait lui-même partie de l'invention. La conservation relative susdite suggère que les deux mycoplasmes, et peut-être d'autres espèces, utilisent cette séquence pour reconnaître une structure commune à la surface des cellules eucaryotes qu'ils parasitent.

Nous avons choisi préférentiellement la séquence de M.genitalium pour induire des anticorps spécifiques de cette séquence, du fait que ce mycoplasme est présent sur les muqueuses respiratoires, rectales et génitales (16), et que nous l'avons mis en évidence sur les globules rouges et les lymphocytes d'un malade atteint de SIDA. D'autres mycoplasmes ont été isolés du tractus intestinal chez l'homme.

Il a aussi été montré chez les patients immunodéprimés, qu'un mycoplasme, M.hominis, pouvait être isolé à partir d'organes, tissus et fluides différents. On se reportera par exemple à l'article de D. Mac Mahon et al. "Extragenital M.hominis infections in adults" publié en 1990, Vol. 89, p. 275 à 281 dans Am. Journal of Medicine.

Les protéines et peptides issus de ces différentes souches et qui répondent aux conditions qui ont été définies plus haut, entrent dans la définition des peptides selon l'invention. On peut en particulier se référer à l'article de GIEBEL J., BINDER A. et al "Isolation of Mycoplasmas from the intestine of rat, swine and man" (Zentralblatte für Bacteriologie, 1990, supplement N° 20, p. ).

A titre de contrôle, la séquence correspondant aux 25 acides aminés de l'extrêmité C terminale de la même protéine a été synthétisée (peptide C) :

A P K K L K Q A T P T K P T P K T P P K P P V K Q

### 2°/ Synthèse du peptide

La méthode développée par Merrifield (17) de synthèse en phase solide a été utilisée. L'assemblage des peptides a été mis en oeuvre de façon automatique sur un synthétiseur APPLIED BIOSYSTEMS modèle 430A.

Une partie (10 mg) a été couplée à une protéine porteuse, la "Keyhole limpet hemocyanin" (KLH, 10 mg) par la méthode à la glutaraldéhyde (18). L'efficacité de couplage était de 50 à 75%, d'après la détermination de la partie non couplée du peptide encore présent après passage sur une colonne de Séphadex G 25.

On peut également avoir recours (pour la production des peptides) à des techniques de recombinaison génétique, mettant par exemple en oeuvre des plasmides appropriés transfectant des hôtes cellulaires tels que bactéries, levures, cellules eucaryotes, etc... et contenant des séquences nucléotidiques codant pour les peptides selon l'invention, y inclus tout ou partie des séquences de protéine de mycoplasme les contenant ou d'autres séquences protéiques, dès lors que les produits d'expression correspondants induisent la production d'anticorps conservant leurs propriétés d'inhibition à l'égard d'une infection à HIV.

Comme on le verra plus loin, l'invention concerne également des associations ou combinaisons des peptides de l'invention -ou d'autres peptides, notamment des peptides présentant une activité immunogène en ce qu'il induisent in vivo la production d'anticorps aptes à neutraliser des infections par HIV, notamment HIV-1 et HIV-2.

L'invention concerne plus particulièrement des peptides recombinants ou hybrides contenant des séquences correspondant à l'un et à l'autre des peptides sus-indiqués. Ces peptides recombinants ou hybrides sont notamment accessibles par la technique de recombinaison génétique dont le principe a été rappelé plus haut, en mettant en oeuvre les plasmides ou les vecteurs viraux par exemple le virus de la vaccine, le baculovirus, etc... appropriés , contenant alors les séquences nucléotidiques qui codent pour ces peptides recombinants ou hybrides et dont l'expression peut être effectuée dans des hôtes cellulaires eucaryotes y inclus la levure (Saccharomyces cerevisiae, Pichia pastoris etc...) ou dans des bactéries.

A titre d'exemple, un recombinant préféré est constitué par une séquence nucléotidique codant pour la région V₃ de la gp 120 en particulier le peptide dont les acides aminés portent les numéros 307 à 321, du HIV-1 (Rusche J. et al., PNAS, 1988, 85, p. 3198-3202) associée à la séquence de la protéine adhésine de M.genitalium dont la séquence du gène a été décrite dans Gene, 1989, **82**, p.259-267 (Inamine et al) ou de l'adhésine de M.pirum ou de Mycoplasmes apparentés et de préférence la séquence nucléotidique correspondant à la séquence du peptide A. Un tel hybride exprimé dans un hôte cellulaire eucaryote y compris les levures par exemple Pichia, Kluyveromyces ou un hôte procaryote permet l'obtention d'une protéine ou d'un peptide hybride qui peut constituer l'un des composants d'une composition immunogène induisant des anticorps qui inhibent une infection à HIV in vitro. On peut se référer pour le choix des séquences d'HIV1 ou d'HIV2 exprimant une protéine à l'article de GUYADER et al Nature, 1987, 326, p. 662-669.

Ces mêmes peptides hybrides peuvent être utilisés à des fins de diagnostic in vitro. La méthodologie utilisée est celle décrite dans le brevet US n° 4.839.288.

### 3./ Préparation et caractérisation des anticorps :

Des lapins de Nouvelle Zélande ont été immunisés comme suit : 200 µg du peptide couplé à la KLH mélangé avec un volume égal d'adjuvant complet de Freund sont injectés par voie intradermique au jour O. Ce protocole a été ensuite répété par voie sous-cutanée en présence d'adjuvant incomplet de Freund aux jours 30, 60 et 90.

Les animaux sont saignés pour obtenir le sérum une semaine après chaque injection. Du sérum des mêmes animaux a été conservé avant le début de l'immunisation, à titre de témoins des effets biologiques des sérums.

Dans le cas où le peptide A est utilisé comme agent immunogène couplé à la KLH, les anticorps ainsi obtenus reconnaissent spécifiquement le peptide immunisant et aussi, en utilisant la technique du Western-Blot, une protéine de 140 KDa dans un extrait solubilisé de M.genitalium (G37) par une solution aqueuse de 1% (poids/volume) de sodium dodécyl sulfate. La solution utilisée est M.genitalium G37 (ATCC N° 33530).

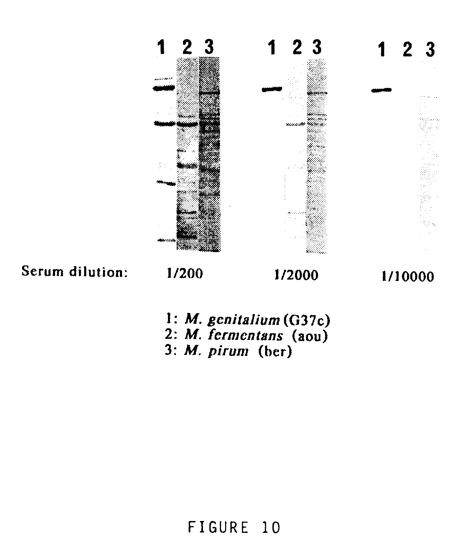

La protéine de 140KDa correspond à l'adhésine qui a été décrite dans la littérature et est reconnue par l'antisérum jusqu'à une dilution d'au moins 1/100.000 de ce dernier (Figure 10).

A une dilution plus faible (1/2000), l'anticorps anti-peptide A reconnait également une protéine de 130 KDa contenue dans un extrait lysé de Mycoplasma pirum (souche Ber) (déposée à la C.N.C.M. le 3 mai 1990 sous le numéro I-950 et à la N.C.I.N.B sous le N° 40283 le 17 mai 1990), un mycoplasme que l'on trouve également fréquemment chez les malades atteints du SIDA, et qui pourrait donc également contaminer les souches de HIV.

Les mesures de poids moléculaires des protéines susdites (obtenues à partir de lysats de mycoplasmes concervés dans du SDS) (dodecyl sulfate de sodium) à 1% ont été mesurées par comparaison de leurs distances de migration respectives avec celles de six protéines de référence, dans un gel d'électrophorèse, ayant des poids moléculaires indiquées ci-après (coffret d'analyse ou "kit" produit par Bio-Rad) :

| | |
|---|---|
| Lysozyme | 14.400 |
| Inhibiteur de trypsine de soja | 21.500 |
| Anhydrase carbonique | 31,000 |
| Ovalbumine | 45.000 |
| Serum albumine bovine | 66.200 |
| Phosphorylase B | 92.500 |

Ces mesures sont faites avec une précision de + ou-10%. La protéine de 130 KDa peut être utilisée pour réactif constituant l'un des éléments d'un coffret pour un test ELISA ou un test RIA ou encore comme réactif après marquage par un marqueur fluorescent par exemple. La protéine P130 KDa de M. pirum peut être également utilisée comme agent immunogène pour induire la synthèse d'anticorps actifs contre une infection à mycoplasme.

Des réactions antigéniques plus faibles sont observées dans les mêmes conditions avec le même anticorps anti peptide A, avec des protéines de masse moléculaire inférieures à 65 KDa, en particulier avec trois protéines de masse moléculaire de 64 KDa, 45 et 42 KDa de M. pirum.

On trouve également fréquemment, chez les malades atteints du SIDA, les anticorps dirigés contre la susdite protéine de 130 KDa, issue de M.pirum, ou reconnaissant des protéines ayant des propriétés antigéniques semblables et qui pourrait donc également contaminer les souches de HIV isolées des malades.

Les méthodes utilisées pour la préparation des peptides sélectionnés ou des anticorps correspondants peuvent naturellement être transposées à la fabrication d'autres peptides ou anticorps entrant dans le champs de la présente invention. Pour les anticorps monoclonaux, on utilise la méthode décrite par Köhler et Milstein (NATURE, 1975, 256, pages 495-497).

### 4°/ Effet des anticorps sur l'infection par HIV :

Des lymphocytes de donneurs HIV négatifs sont activés par la phytohémaglutinine pendant trois jours et cultivés en présence de protéine d'interleukine 2 en milieu RPMI 1640 avec 10% de sérum de veau fetal, 100 U/ml de pénicilline et 50 U/ml de streptomycine. Ils sont infectés par la souche prototype HIV-1 LAV_{BRU} à raison de 10⁴ cpm d'activité transcriptase inverse par 10⁶ cellules. La souche HIV-1 BRU est un échantillon de la souche CNCM I-232.

D'autres flacons de la même culture sont infectés avec la même suspension virale, préalablement incubée une heure à 37°C avec différentes concentrations d'antisérums dilués au 1/50ème ou au 1/200ème de lapins immunisés contre les peptides de l'adhésine selon le procédé décrit supra.

Ils ont été injectés à des lapins après couplage avec la KLH et adjonction d'adjuvant complet de Freund. Les anticorps obtenus ont été testés selon la technique décrite par Szmelcman et al (8) pour évaluer leurs effets, après mise en contact avec les lymphocytes T en culture puis infectés par la souche prototype HIV-1 (BRU). Le dosage de la transcriptase inverse et le dosage de la production de p25 ont été effectués pour déterminer la production du virus.

La production de HIV par les cultures est suivie par l'activité transcriptase inverse et celle de l'antigène p25 est mesurée par ELISA (Diagnostic Pasteur) dans les surnageants de culture. Cette production est aussi appréciée par l'effet cytopathogène (formation de syncitias et lyse des cellules isolées) induit par le virus.

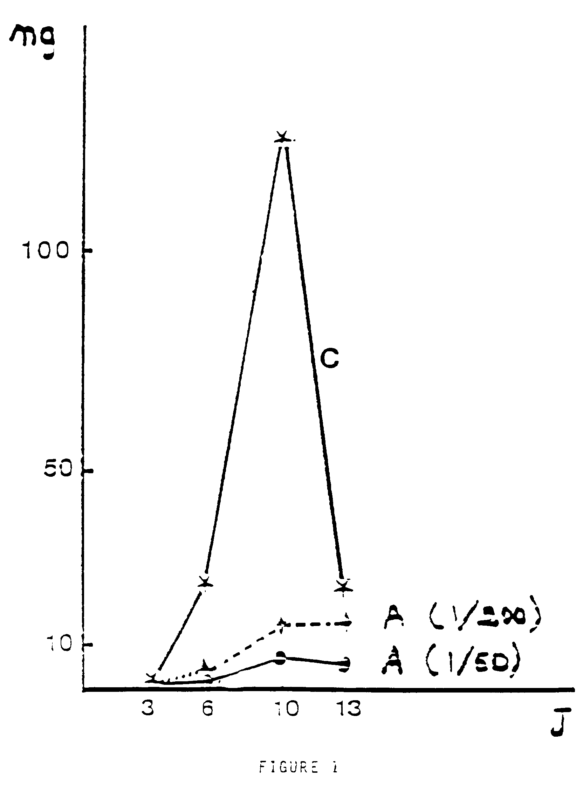

On constate que la production de p25, et par conséquent de virus est inhibée à plus de 90% par une dilution au 1/50^{ème} de l'antisérum contre le peptide A. Une moindre inhibition, mais encore notable, est obtenue au 1/200^{ème}. Les variations des taux d'inhibition de la quantité de p25 produite (p25) en ng sur l'axe des ordonnées, en fonction du temps (en jours (J) sur l'axe des abscisses) sont illustrées par les courbes de la figure 1. Les mesures ont été faites en présence d'anticorps contre le peptide A (aux dilutions de 1/50^{ème} et 1/200^{ème}) et du peptide C.

Les sérums de lapins prélevés avant immunisation n'ont pas d'effet, pas plus que les sérums des lapins immunisés contre le peptide C de la partie C-terminale de l'adhésine.

La spécificité de l'inhibition est montrée par le fait qu'elle est réduite par la préincubation du sérum anti-peptide A avec le peptide A en excès, l'inhibition résiduelle étant liée au peptide lui-même (voir plus loin).

L'inhibition par l'antisérum anti-peptide A est observée quand il est ajouté au moment de l'infection des lymphocytes T normaux par la préparation virale. Par "préparation virale" on entend un surnageant brut de culture de cellules lymphocytaires infectées par le virus HIV, ce surnageant pouvant contenir des mycoplasmes. Mais l'inhibition peut aussi être observée quand la préparation virale est préincubée pendant 1 heure à 37°C avec l'antisérum anti-peptide A, puis est ajoutée aux cellules pour l'infection. Dans ce cas, il n'est pas nécessaire, pendant la durée de l'expérience, de rajouter par la suite de l'antisérum au milieu de culture, pour obtenir l'effet d'inhibition maximum.

Des résultats similaires ont été obtenus avec la lignée cellulaire CEM clone 13, permissive à la souche HIV-1 BRU. Ces cellules sont préalablement propagées pendant trois semaines en présence d'antibiotiques ayant un large spectre d'action sur les mycoplasmes (MRA) avant d'être infectées par le virus, de façon à réduire la contamination endogène de cette lignée par des mycoplasmes.

On observe avec la lignée CEM infectée par une préparation virale d'HIV-1 (BRU) ou HIV-2 (ROD) traitée à l'antisérum anti-peptide A un retard dans la courbe de production du virus, mais pas une suppression totale de cette production, ainsi qu'un retard dans l'effet cytopathogène du virus. Cet effet cytopathogène se caractérise par la formation de grappes cellulaires, puis de syncitia ; on peut penser qu'il se produit secondairement une propagation de l'infection par contacts intercellulaires, ce mode d'infection n'étant plus sensible à l'antisérum anti-peptide A.

Une inhibition similaire par l'antisérum a été observée sur la souche HIV-2 ROD propagée sur les cellules CEM (figure 1 bis).

### 5°/ Effet du peptide A lui-même :

Si l'antisérum contre le peptide de A (ou peptide modèle du site de fixation du mycoplasme (Mycoplasma Binding Site Peptide)) inhibe la fixation aux cellules du mycoplasme contaminant le virus dans la préparation virale, il s'ensuit qu'on pourrait également neutraliser cette fixation par le peptide lui-même présent au moment où la préparation virus-mycoplasme est ajoutée aux cellules. Cette hypothèse a été vérifiée et on observe une inhibition de l'infectiosité du virus par le peptide à des concentrations relativement élevées (de l'ordre de 500 µg/ml).

En conclusion, nos résultats confirment qu'un mycoplasme de type M.genitalium ou M.pirum relativement proches de ces derniers, est impliqué dans une phase précoce de l'infection HIV-1_{BRU} (fixation, pénétration ou début de l'expression génomique dans les cellules T).

On peut donc envisager l'application de la présente invention à la préparation de compositions immunogènes, de vaccins ou de médicaments directement actifs, à la mise au point de nouvelles thérapeutiques, et à des méthodes diagnostiques :
1. Vaccination par le peptide A ou d'autres peptides efficaces conformes à l'invention, de sujets HIV-séronégatifs ou HIV séropositifs asymptomiques par le peptide A ou des peptides contenant la même séquence ou contenant des séquences permettant l'induction in vivo d'anticorps spécifiques ayant des propriétés identiques ou semblables contre l'infection à HIV, le cas échéant en présence d'un adjuvant approprié à l'homme, tel l'hydroxyde d'aluminium. Cette vaccination serait protectrice contre l'infection par HIV chez les sujets HIV-séronégatifs et protectrice de l'évolution vers le SIDA chez les sujets séropositifs asymptomatiques.
   Le cas échéant, les peptides contenus dans ces compositions vaccinantes sont eux-mêmes greffés sur des protéines ou polypeptides, porteurs aptes à renforcer l'immunogenicité recherchée desdits peptides.
   L'invention s'étend également à l'utilisation, en vaccination, des peptides selon l'invention, en association ou en combinaison avec un vaccin HIV distinct pour augmenter le pouvoir protecteur de ce dernier.
   L'invention concerne donc également des compositions immunogènes, plus particulièrement vaccinantes, à base de l'un au moins des peptides selon l'invention, soit pris seul, soit en combinaison avec des peptides immunogènes, plus particulièrement vaccinants, issus de HIV, ou avec des peptides qui leur sont apparentés (voir demandes de brevet européen rendues publiques au nom de l'lnstitut Pasteur, le cas échéant avec d'autres co-déposants, par exemple les demandes européennes publiées sous les N° EP 201540 (N° de dépôt 85.905513.9), et EP 283327 (N° de dépôt 88.400084.5). Un peptide issu de la glycoprotéine d'enveloppe gp 120 décrit par Rusche et al (PNAS 1988, 85, p 3198) est l'un des peptides préférés issus de HIV.
   On peut également utiliser comme peptide de HIV selon l'invention, la glycoprotéine d'enveloppe gp 120 ou des fragments de celle-ci dont l'activité a été décrite par BERMAN et al dans NATURE, 1990, Volume 345, page 622 à 625 (Protection of chimpanzees from infection by HIV-1 after vaccination with recombinant glycoprotein gp 120 but not gp 160) (Protection du chimpanzee contre l'infection par HIV) par vaccination avec une protéine recombinante gp 120, mais non gp 160). Ces compositions contiennent également, le cas échéant, les véhicules appropriés aux modes d'administration choisis, notamment par voie parentérale. On utilisera des doses immunogènes de peptide comprises entre 3 µg/kg et 10 µg/kg d'hôte recevant ces doses (Anderson, J. Inf. Dis., 1989, 160).
2. Thérapeutique chez les sujets immunodéprimés (en état de SIDA), soit par injection intraveineuse du peptide lui-même, soit par administration d'anticorps préalablement formés contre des peptides conformes à l'invention, en particulier des anticorps anti-peptide A préparés chez l'animal (immunothérapie passive) ou des anticorps monoclonaux. L'utilisation de fragments actifs purifiés, par exemple des fragments FAB de ces anticorps sera préconisée pour éviter les accidents d'hypersensibilité.
3. Diagnostic :
   Mesure chez les malades et les personnes asymptomatiques du taux d'anticorps contre le peptide A ou autres peptides conformes à l'invention, originaires de mycoplasmes et susceptibles d'induire des anticorps inhibant ou diminuant une infection à HIV in vitro ou in vivo.
   Application au diagnostic et au suivi de l'effet de médicaments actifs sur les mycoplasmes et dans le SIDA.

Les figures 2 à 9 qui suivent fournissent à la fois les séquences d'acides aminés de protéines issues de M. genitalium et de différentes protéines issues soit de HIV-1 ou de HIV-2.

Ces figures permettent d'identifier les peptides de l'invention qui, par application des règles d'isologies et d'homologies définies plus haut, font partie de M. genitalium et qui présentent des isologies, voire même des homologies, avec des régions correspondantes des différentes protéines de HIV-1 et HIV-2.

Plus particulièrement on note les séquences des acides aminés de la protéine p1 de M. genitalium qui se trouvent alignées sur :
Fig. 2A à 2J :
   la séquence en acides aminés de l'enveloppe (alignement par homologies directes) de HIV-2 ROD
Fig. 3A à 3K :
   la séquence de la protéine de l'enveloppe de HIV-2 ROD
Fig. 4A à 4K :
   la séquence de la protéine d'enveloppe de HIV-1 BRU
Fig. 5A à 5J :
   la protéine d'enveloppe de HIV-1 BRU
Fig. 6A à 6I :
   la séquence de p27 nef de HIV-1 BRU
Fig. 7A à 7I :
   la protéine p27 nef de HIV-1 BRU
Fig. 8A à 8I :
   des séquences de p31 nef de HIV-2 (ROD)
Fig. 9A à 9J :
   des séquences de p31 nef HIV-2 ROD.

L'attention est encore attirée :
- sur la figure lbis illustrant l'inhibition dans le temps (en jours sur l'axe des abscisses) de la production de reverse transcriptase virale (activité en ordonnées) dans des lymphocytes T placas en présence de HIV-2 et d'anti-peptides de A, par comparaison aux observations faites sur une culture "contrôle", en l'absence d'anticorps anti-peptide
- sur la figure 10 montrant les bandes d'électrophorèses observées aux dilutions indiquées dans des essais réalisés par analyse dide de "Western blot" et effectuées sur des sérums. On voit apparaître la présence d'une bande caractéristique du site de fixation du peptide A dans les essais mettant en oeuvre M. genitalium.

Dans le but de confirmer les résultats obtenus avec les anticorps de lapins immunisés avec le peptide A couplé à la KLH, le même peptide ainsi couplé a été utilisé pour immuniser 2 chevaux selon le calendrier suivant :

| CALENDRIER D'INJECTION ET DE PRELEVEMENTS | | |
|---|---|---|
| Période | Injection | Prélèvement |
| J0 | 1mg Pep P/CFA vol à vol. | 450 ml de sang stérile |
| J30 | 1 mg Pep P/IFA vol à vol. | 450 ml de sang stérile |
| J40 | 1 mg Pep P/IFA vol à vol. | 450 ml de sang stérile |
| J60 | 1 mg Pep P/IFA vol à vol. | 450 ml de sang stérile |
| J70 | 1 mg Pep P/IFA vol à vol. | 450 ml de sang stérile |
| J90 | 1 mg Pep P/IFA vol à vol. | 450 ml de sang stérile |
| J100 | 1 mg Pep P/IFA vol à vol. | 450 ml de sang stérile |
| J120 | 1 mg Pep P/IFA vol à vol. | 450 ml de sang stérile |
| J140 | 1 mg Pep P/IFA vol à vol. | 450 ml de sang stérile |
| J30 | 1 mg Pep P/IFA vol à vol. | 450 ml de sang stérile |
| J30 | 1 mg Pep P/IFA vol à vol. | 450 ml de sang stérile |

Les sérums déjà prélevés ont montré une forte immunoréactivité contre le peptide en ELISA (titre des sérums de l'ordre de 1/10⁶).

Des immunoglobulines sont en cours de purification afin de préparer les fragments Fab correspondant. Le sérum total, les immunoglobulines purifiées ainsi que les fragments Fab seront testés pour leur capacité à inhiber in vitro l'infection des cellules CD₄ ⁺ par le virus HIV.

### BIBLIOGRAPHIE

1. Lo S.C. et al.
   A.M.J. Trop. Med 1989, 41 (5)n 601-616
2. Saillard et al.
   Res. Virol., 1990, 141, 385-395
3. Chowohury I.H. et al.
   The Lancet 1990, Vol 336, 247-248
4. Lemaitre M et al.
   Res. Virol, 1990, 141, 5-16
5. Poch O. et al.
   The EMBO Journal, 1989, vol 8, N° 12, 3867-3874
6. Inamine et al.
   Gene, 1988, 64, 217-229.
7. Morrison-Plummer J. et al.
   Infection and Immunity, 1987, vol. 55, N° 1, 49-56
8. Scmelcman S. et al.
   J. of AIDS, 1990, vol. 3, N° 9, p. 859-887
9. Wibur W.J., Lipman D.
   Protc. Natl. Acad. Sci. USA, 1984, 80, 726-730
10. Lipman D., Pearson W.
   Science, 1935, vol. 227, 1435-1441
11. Barre-Sinoussi F. et al.
   Science, 1983, 220, 868-871.
12. Dallo S.F. et al.
   J. Exp. Med., 1988, 167, 718-723.
13. Wain Hobson et al.
   Cell, 1985, 40, 9-17.
14. Freed E.O. et al.
   Bull. Inst. Past. 1990, 88, p. 73-110
15. Dallo S.F.et al.
   Infection and Immunity, 1989, 57, 1059-1065.
16. Hooton T.M.
   Lancet, 1988, 1:266-268.
17. Merrifield R.B.
   J. AM. Chem. Soc., 1963, 85, 2149-2154.
18. Pfaff E.
   EMBO J., 1982, 1, 869-874.
19. Vaimus et al.
   Nature, Vol 333, p. 504.

## Revendications

1. Utilisation d'un peptide ou d'une séquence peptidique constitué par un fragment d'une protéine de structure d'un mycoplasme, pour la production d'une composition apte à induire une réponse immunitaire conduisant à la production d'anticorps capables d'inhiber l'infection par un HIV, ledit peptide présentant un pourcentage d'au moins 60% d'isologie ou, selon le cas, de 70% d'homologie avec une séquence peptidique, protéique ou glycoprotéique issue de HIV.

2. Utilisation selon la revendication 1 caractérisée en ce que la réaction immunitaire est une production in vivo d'anticorps aptes à inhiber l'infection par HIV.

3. Utilisation selon la revendication 1 ou 2, caractérisée en ce que la protéine de structure de mycoplasme est une adhésine de mycoplasme et que le fragment utilisé contient de préférence un site actif de ladite adhésine.

4. Utilisation selon les revendications 1 à 3, caractérisée en ce que le peptide utilisé présente un pourcentage d'au moins 60% d'isologie ou, selon le cas, de 70% d'homologie avec une séquence d'acides aminés d'une gp 160 env. ou p 27 nef de HIV.

5. Utilisation du peptide selon l'une des revendications 1 à 4 caractérisé en ce qu'il contient de 5 à 25 acides aminés, notamment de 8 à 15 acides aminés, possédant l'une des séquences suivantes d'acides aminés: 
G V V S T P L V N L I N G Q
G V V S T P L V N L I N
K M D G K L T G V V S T P L V N L I N G Q
G I V R T P L A E L L D G E
 ou une séquence soit plus longue, soit plus courte mais comprenant le site qui confère au peptide correspondant la capacité d'induire in vivo la production d'anticorps ayant la propriété d'inhiber une infection de lymphocytes T par HIV.

6. Peptide caractérisé par le fait que sa séquence est issue d'une adhésine du mycoplasme, et contenant un site ayant la capacité d'induire in vivo la production d'anticorps ayant la propriété d'inhiber une infection de lymphocytes T par HIV, ledit peptide présentant un pourcentage d'au moins 60% d'isologie ou, selon le cas, de 70% d'homologie avec une séquence peptidique, protéique ou glycoprotéique issue de HIV.

7. Peptide selon la revendication 6, caractérisé par le fait qu'il est issu d'une adhésine de mycoplasme choisi notamment parmi M. pneumoniae, M. genitalium ou M. pirum.

8. Peptide, selon l'une des revendications 6 ou 7, caractérisé en ce qu'il présente un pourcentage au moins de 60% d'isologie ou, selon le cas, de 70% d'homologie avec une séquence d'acides aminés d'une gp 160 env., ou p27 nef de HIV, notamment HIV-1 ou HIV-2.

9. Peptide, selon l'une quelconque des revendications 6 à 8, caractérisé en ce qu'il présente le susdit pourcentage d'isologie ou, selon le cas, d'homologie avec une séquence d'acides aminés issue, soit d'une protéine gag, soit d'une glycoprotéine d'enveloppe, soit encore de la protéine nef d'un HIV.

10. Peptide selon l'une quelconque des revendications 6 à 9, caractérisé en ce qu'il contient de 5 à 25 acides aminés, notamment de 8 à 15 acides aminés, possédant l'une des séquences suivantes d'acides aminés : 
G V V S T P L V N L I N G Q
G V V S T P L V N L I N
K M D G K L T G V V S T P L V N L I N G Q
G I V R T P L A E L L D G E
 ou une séquence soit plus longue, soit plus courte mais comprenant le site qui confère au peptide correspondant la capacité d'induire in vivo la production d'anticorps ayant la propriété d'inhiber une infection de lymphocytes T par HIV.

11. Peptide selon les revendications 8 à 10, caractérisé en ce qu'il possède un épitope particulier contenant la séquence TPL.

12. Peptide selon la revendication 6 ou 7, caractérisé en ce qu'il est dérivé de M. genitalium, qu'il présente les susdites homologie ou isologie avec la gp 160 de HIV1 et que soit il comprend la séquence suivante d'acides aminés : 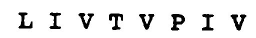 soit sa séquence en acides aminés est plus courte, incluse dans la séquence identifiée ci-dessus et comprend le site qui confère au peptide correspondant la capacité d'induire in vivo la production d'anticorps ayant la propriété d'inhiber une infection de lymphocytes T par HIV.

13. Peptide selon la revendication 6 ou 7, caractérisé en ce qu'il est dérivé de M. genitalium, qu'il présente une homologie de séquence avec la protéine nef de HIV1 et que soit il comprend la séquence suivante d'acides aminés : 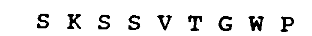 soit sa séquence en acides aminés est plus courte, incluse dans la séquence identifiée ci-dessus et comprend le site qui confère au peptide correspondant la capacité d'induire in vivo la production d'anticorps ayant la propriété d'inhiber une infection de lymphocytes T par HIV.

14. Peptide issu d'une protéine gp 160 d'un HIV et présentant au moins 60% d'isologie de séquence avec la séquence d'un peptide selon la revendication 6, et dont la séquence d'acides aminés est l'une des suivantes :

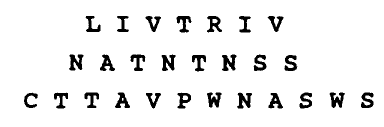

15. Peptide issu d'une protéine nef d'un HIV et présentant une isologie d'au moins 60% ou une homologie d'au moins 70% avec un peptide de la revendication 6 dont la séquence d'acides aminés est la suivante:

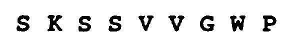

16. Peptide selon la revendication 8 ou 9, caractérisé en ce qu'il est dérivé de M. pneumoniae, qu'il présente un pourcentage d'au moins 60% d'isologie ou, selon le cas de 70% d'homologie avec la gp 160 de HIV-1 et qu'il contient l'une des séquences suivantes d'acides aminés:

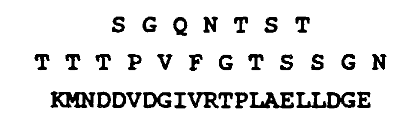

17. Peptide recombinant ou hybride contenant une séquence peptidique de l'un des peptides selon l'une quelconque des revendications 6 à 16 et une séquence d'un peptide immunogène capable d'induire in vivo la production d'anticorps aptes à neutraliser une infection par HIV, notamment HIV-1 et HIV-2.

18. Peptide recombinant ou hybride selon la revendication 17, caractérisé en ce que la séquence du peptide immunogène capable d'induire in vivo des anticorps aptes à neutraliser une infection par HIV est la séquence codant pour la région V₃ de la gp 120 de HIV-1.

19. Peptide selon l'une quelconque des revendications 6 à 18, caractérisé en ce qu'il est lui-même greffé sur une protéine ou polypeptide porteur, apte à renforcer l'immunogénicité audit peptide.

20. Protéine purifiée de M. pirum ayant un poids moléculaire de 130 kDa correspondant à l'adhésine, cette protéine étant reconnue par des anticorps formés contre le peptide 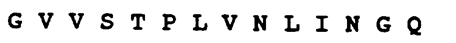 de la revendication 10.

21. Protéine purifiée de M. pirum ayant un poids moléculaire inférieur à 65 kDa, notamment de l'ordre de 64 kDa, 45 ou 42 kDa, laquelle protéine est reconnue par des anticorps formés contre le peptide : 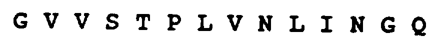 de la revendication 10, ladite protéine étant issue de la souche de M. pirum, déposée le 3 mai 1990 auprès de la C.N.C.M., sous le n.I-950 et le 17 mai 1990 à la N.C.I.M.B., sous le n. 40.283.

22. Anticorps monoclonal ou polyclonal contre l'un des peptides ou protéines selon l'une quelconque des revendications 6 à 21.

23. Anticorps selon la revendication 22 contre ceux des peptides qui contiennent le site actif de fixation de l'adhésine de M. genitalium et qui inhibent eux-mêmes l'infection par HIV de lymphocytes T en culture.

24. Anticorps selon la revendication 22 contre ceux des peptides qui contiennt le site actif de fixation de l'adhésine de M. pirum et qui inhibent eux-mêmes l'infection par HIV de lymphocytes T en culture.

25. Utilisation pour la production d'un médicament apte à induire in vivo des anticorps aptes à inhiber l'infection de cellules permissives à HIV, tel que HIV1 ou HIV2,
- soit d'un peptide constitué par un fragment d'une protéine de structure d'un mycoplasme et présentant un pourcentage d'au moins 60% d'isologie ou, selon le cas, de 70% d'homologie avec une séquence peptidique, protéique ou glycoprotéique issue de HIV,
- soit d'un peptide selon l'une quelconque des revendications 14 à 17,
- soit d'un peptide selon l'une quelconque des revendications 18 et 19, associé à un véhicule pharmaceutique.

26. Utilisation selon la revendication 25 caractérisé en ce que le peptide utilisé est issu d'une adhésine de mycoplasme.

27. Utilisation selon l'une des revendications 25 ou 26 caractérisé en ce que le peptide est issu d'une adhésine de M. pneumoniae, M. genitalium, M. pirum.

28. Utilisation selon l'une des revendications 25 à 26 caractérisé en ce que le peptide utilisé est l'un de ceux définis dans les revendications 6 à 13 et dans la revendication 18.

29. Utilisation pour la production d'un médicament apte à induire in vivo des anticorps aptes à inhiber l'infection de cellules permissives à HIV, tel que HIV1 ou HIV2, d'une protéine selon la revendication 20 ou la revendication 21.

30. Composition immunogène contre l'infection par HIV contenant l'un des peptides selon l'une quelconque des revendications 6 à 19 ou une protéine selon la revendication 20 ou la revendication 21.

31. Composition immunogène selon la revendication 30, caractérisée en ce qu'elle contient en outre un peptide distinct vaccinant contre HIV.

32. Composition selon la revendication 31, caractérisée en ce que le peptide distinct, capable d'induire in vivo la production d'anticorps aptes à neutraliser une infection par HIV, est un peptide contenu dans la région V₃ de la gp 120 de HIV-1.

33. Vaccin contre une infection à HIV contenant d'une part, l'un au moins des peptides selon les revendications 6 à 20 ou une protéine selon la revendication 20 ou la revendication 21 et, d'autre part un peptide issu d'au moins HIV-1 ou HIV-2.

34. Composition immunogène apte à induire in vivo la production d'anticorps actifs contre des mycoplasmes, caractérisée en ce qu'elle contient la protéine ou fragment de protéine selon la revendication 20 ou la revendication 21.

35. Procédé de détection dans un échantillon biologique provenant d'une personne asymptomatique ou d'un malade, d'anticorps contre des peptides de mycoplasmes, ledit procédé étant caractérisé par la mise en contact de cet échantillon biologique avec un peptide susceptible d'induire in vivo la production d'anticorps aptes à inhiber ou diminuer l'infection par HIV in vitro, ledit peptide présentant un pourcentage d'au moins 60% d'isologie ou, selon le cas, de 70% d'homologie avec une séquence peptidique, protéique ou glycoprotéique issue de HIV.

36. Procédé de détection d'anticorps dans un échantillon biologique provenant d'une personne susceptible d'être infectée par HIV, par des mycoplasmes, ou par les deux à la fois, caractérisé par la mise en contact de cet échantillon avec un peptide selon l'une quelconque des revendications 6 à 19.

37. Procédé de détection dans un échantillon biologique provenant d 'une personne asymptomatique ou d'un malade, d'anticorps contre des peptides de mycoplasmes et susceptibles d'induire des anticorps inhibant ou diminuant une infection par HIV in vitro ou in vivo, caractérisé par la mise en contact de cet échantillon biologique avec une protéine ou un fragment de protéine selon la revendication 20 ou la revendication 21.

38. Procédé de détection dans un échantillon biologique provenant d'une personne asymptomatique ou d'un malade, d'anticorps spécifiques de M. genitalium caractérisé par la mise en contact de cet échantillon biologique avec un peptide contenant la région V₃ de la gp 120, en particulier le peptide dont les acides aminés portent les numéros 307 à 321 du HIV1 ou le peptide de la revendication 10.

39. Procédé pour la détection d'anticorps spécifiques de M. genitalium dans un échantillon biologique, caractérisé par la mise en contact de cet échantillon avec un peptide selon l'une quelconque des revendications 6 à 9, contenant de 5 à 25 acides aminés, de préférence de 8 à 15 acides aminés, possédant l'une des séquences suivantes d'acides aminés: 
G V V S T P L V N L I N G Q
G V V S T P L V N L I N
K M D G K L T G V V S T P L V N L I N G Q
G I V R T P L A E L L D G E
 ou une séquence soit plus longue, soit plus courte mais comprenant le site qui confère au peptide correspondant la capacité d'induire in vivo la production d'anticorps ayant la propriété d'inhiber une infection de lymphocytes T par HIV.

40. Procédé de préparation de "molécules hybrides" ou "peptides hybrides" caractérisé par :
- la transformation d'un organisme susceptible de les exprimer avec un acide nucléique recombinant constitué pour partie d'une séquence nucléotidique codant pour au moins un peptide appartenant à HIV et pour partie d'une séquence codant pour au moins un peptide selon l"une des revendications 6 à 19,
- par la culture des organismes ainsi transformés et,
- la récupération desdits "molécules hybrides ou "peptides hybrides" parmi les produits exprimés.

41. Composition de peptides hybrides caractérisée en ce qu'elle contient des peptides appartenant à HIV et pour partie un peptide de mycoplasme selon l'une des revendications 6 à 19.

42. Composition selon la revendication 41, caractérisée en ce que les peptides hybrides sont liés entre eux de manière covalente.

43. Composition caractérisée en ce qu'elle est constituée par un mélange d'un peptide de HIV et d'un peptide de mycoplasme, non liés entre eux de manière covalente, les peptides de mycoplasmes présentant un pourcentage d'au moins 60% d'isologie ou, selon le cas, de 70% d'homologie avec une séquence peptidique, protéique ou glycoprotéique issue de HIV.

44. Composition caractérisée en ce qu'elle est constituée par un mélange d'un peptide de HIV et d'une protéine selon la revendication 20 ou la revendication 21.

45. Utilisation d'une composition selon l'une des revendications 41 à 44, pour la production d'un vaccin contre une infection à HIV.

46. Utilisation d'une composition comprenant un peptide selon l'une quelconque des revendications 6 à 19 ou une séquence constituée par un fragment d'une protéine de structure d'un mycoplasme selon la revendication 20 ou 21 pour la production d'une composition apte à induire une production in vivo d'anticorps capables d'inhiber l'infection par un HIV, et un peptide ou un polypeptide d'HIV.

## Patentansprüche

1. Verwendung eines Peptids oder einer Peptidsequenz, umfassend ein Fragment eines Strukturproteins eines Mycoplasma, für die Herstellung einer Zusammensetzung, die geeignet ist, eine auf die Produktion von Antikörpern, die die Infektion durch ein HIV inhibieren können, gerichtete Immunantwort zu induzieren, wobei das Peptid einen Prozentsatz von mindestens 60 % Isologie oder, nach dem Umständen, 70 % Homologie zu einer aus HIV stammenden Peptid-, Protein- oder Glykoproteinsequenz aufweist.

2. Verwendung nach Anspruch 1,
dadurch gekennzeichnet, daß die Immunreaktion eine in vivo-Produktion von zur Inhibierung der Infektion durch HIV geeigneten Antikörpern ist.

3. Verwendung nach Anspruch 1 oder 2,
dadurch gekennzeichnet, daß das Strukturprotein eines Mycoplasma ein Adhäsin eines Mycoplasma ist und daß das verwendete Fragment vorzugsweise eine aktive Stelle dieses Adhäsins enthält.

4. Verwendung nach den Ansprüchen 1 bis 3,
dadurch gekennzeichnet, daß das verwendete Peptid einen Prozentsatz von mindestens 60 % Isologie oder, nach den Umständen, 70 % Homologie zu einer Aminosäuresequenz eines gp 160 env. oder p 27 nef von HIV hat.

5. Verwendung des Peptids nach einem der Ansprüche 1 bis 4,
dadurch gekennzeichnet, daß es 5 bis 25 Aminosäuren, insbesondere 8 bis 15 Aminosäuren, umfaßt, wobei es eine der folgenden Aminosäuresequenzen aufweist: 
G V V S T P L V N L I N G Q
G V V S T P L V N L I N
K M D G K L T G V V S T P L V N L I N G Q
G I V R T P L A E L L D G E
 oder eine entweder längere oder kürzere Sequenz, die aber die Stelle umfaßt, die dem entsprechenden Peptid die Fähigkeit verleiht, in vivo die Produktion von Antikörpern zu induzieren, die die Eigenschaft haben, eine Infektion von T-Lymphozyten durch HIV zu inhibieren.

6. Peptid,
gekennzeichnet dadurch, daß seine Sequenz von einem Adhäsin aus Mycoplasma abstammt und eine Stelle enthält, die die Fähigkeit hat, in vivo die Produktion von Antikörpern zu induzieren, die die Eigenschaft haben, eine Infektion von T-Lymphozyten durch HIV zu inhibieren, wobei das Peptid einen Prozentsatz von mindestens 60 % Isologie oder, nach den Umständen, 70 % Homologie zu einer aus HIV stammenden Peptid-, Protein- oder Glykoproteinsequenz aufweist.

7. Peptid nach Anspruch 6,
gekennzeichnet dadurch, daß es von einem Adhäsin eines Mycoplasma, ausgewählt insbesondere unter M. pneumoniae, M. genitalium oder M. pirum, abstammt.

8. Peptid nach einem der Ansprüche 6 oder 7,
dadurch gekennzeichnet, daß es einen Prozentsatz mindestens von 60 % Isologie oder, nach den Umständen, 70 % Homologie zu einer Aminosäuresequenz eines gp 160 env. oder p27 nef aus HIV, insbesondere HIV-1 oder HIV-2, aufweist.

9. Peptid nach einem der Ansprüche 6 bis 8,
dadurch gekennzeichnet, daß es den obengenannten Prozentsatz an Isologie oder, nach den Umständen, Homologie zu einer Aminosäuresequenz, die entweder aus einem Protein gag, einem Glykoprotein der Hülle oder weiters dem nef-Protein eines HIV stammt, aufweist.

10. Peptid nach einem der Ansprüche 6 bis 9, dadurch gekennzeichnet, daß es 5 bis 25 Aminosäuren, insbesondere 8 bis 15 Aminosäuren, umfaßt, wobei es eine der folgenden Aminosäuresequenzen aufweist: 
G V V S T P L V N L I N G Q
G V V S T P L V N L I N
K M D G K L T G V V S T P L V N L I N G Q
G I V R T P L A E L L D G E
 oder eine entweder längere oder kürzere Sequenz, die aber die Stelle umfaßt, die dem entsprechenden Peptid die Fähigkeit verleiht, in vivo die Produktion von Antikörpern zu induzieren, die die Eigenschaft haben, eine Infektion von T-Lymphozyten durch HIV zu inhibieren.

11. Peptid nach den Ansprüchen 8 bis 10, dadurch gekennzeichnet, daß es ein besonderes Epitop, das die Sequenz TPL enthält, aufweist.

12. Peptid nach Anspruch 6 oder 7,
dadurch gekennzeichnet, daß es von M. genitalium abgeleitet ist, die obengenannte Homologie oder Isologie zu dem gp 160 aus HIV-1 aufweist und entweder die folgende Aminosäuresequenz enthält: 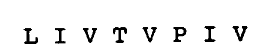 oder daß seine Aminosäuresequenz kürzer ist, in der vorstehend angegebenen Sequenz enthalten ist und die Stelle enthält, die dem entsprechenden Peptid die Fähigkeit verleiht, in vivo die Produktion von Antikörpern zu induzieren, die die Eigenschaft haben, eine Infektion von T-Lymphozyten durch HIV zu inhibieren.

13. Peptid nach Anspruch 6 oder 7, dadurch gekennzeichnet, daß es von M. genitalium abgeleitet ist, eine Sequenzhomologie zu dem nef-Protein aus HIV-1 aufweist und entweder die folgende Aminosäuresequenz enthält: 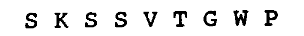 oder daß seine Aminosäuresequenz kürzer ist, in der vorstehend angegebenen Sequenz enthalten ist und die Stelle enthält, die dem entsprechenden Peptid die Fähigkeit verleiht, in vivo die Produktion von Antikörpern zu induzieren, die die Eigenschaft haben, eine Infektion von T-Lymphozyten durch HIV zu inhibieren.

14. Peptid, das aus einem gp 160-Protein eines HIV stammt und mindestens 60 % Sequenzisologie zu einer Sequenz eines Peptids nach Anspruch 6 aufweist und dessen Aminosäuresequenz eine der folgenden ist:

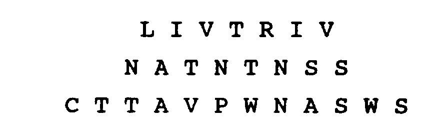

15. Peptid, das aus einem nef-Protein eines HIV stammt und eine Isologie von mindestens 60 % oder eine Homologie von mindestens 70 % zu einem Peptid des Anspruchs 6 aufweist, wobei dessen Aminosäuresequenz die folgende ist:

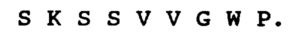

16. Peptid nach Anspruch 8 oder 9,
dadurch gekennzeichnet, daß es von M. pneumoniae abgeleitet ist, einen Prozentsatz von mindestens 60 % Isologie oder, nach den Umständen, 70 % Homologie zu dem gp 160 aus HIV-1 aufweist und eine der folgenden Aminosäuresequenzen enthält

S G Q N T S T
T T T P V F G T S S G N
K M N D D V D G I V R T P L A E L L D G E

17. Rekombinantes oder hybrides Peptid, das eine Peptidsequenz eines der Peptide nach einem der Ansprüche 6 bis 16 und eine immunogene Peptidsequenz, die in vivo die Produktion von zur Neutralisierung einer Infektion durch HIV, insbesondere durch HIV-1 und HIV-2, geeigneten Antikörpern induzieren kann, umfaßt.

18. Rekombinantes oder hybrides Peptid nach Anspruch 17, dadurch gekennzeichnet, daß die immunogene Peptidsequenz, die in vivo zur Neutralisierung einer Infektion durch HIV geeignete Antikörper induzieren kann, die für den Abschnitt V₃ des gp 120 aus HIV-1 kodierende Sequenz ist.

19. Peptid nach einem der Ansprüche 6 bis 18, dadurch gekennzeichnet, daß es mit einem Trägerprotein oder Trägerpolypeptid, die geeignet sind, die Immunogenität des Peptids zu erhöhen, gekoppelt ist.

20. Gereinigtes Protein aus M. pirum mit einem Molekulargewicht von 130 kDa entsprechend dem Adhäsin, wobei dieses Protein durch Antikörper, die gegen das Peptid 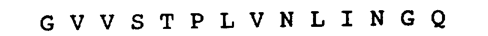 des Anspruchs 10 gebildet werden, erkannt wird.

21. Gereinigtes Protein aus M. pirum mit einem Molekulargewicht unter 65 kDa, insbesondere von einer Größenordnung von 64 kDa, 45 oder 42 kDa, wobei dieses Protein durch Antikörper, die gegen das Peptid 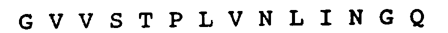 des Anspruchs 10 gebildet werden, erkannt wird, wobei das Protein aus dem Stamm von M. pirum stammt, der am 03. Mai 1990 bei der C.N.C.M. unter der Nr. I-950 und am 17. Mai 1990 bei der N.C.I.M.B. unter der Nr. 40.283 hinterlegt worden ist.

22. Monoklonaler oder polyklonaler Antikörper gegen eines der Peptide oder Proteine nach einem der Ansprüche 6 bis 21.

23. Antikörper nach Anspruch 22 gegen diejenigen der Peptide, die die aktive Anheftungsstelle des Adhäsins aus M. genitalium umfassen und die selbst die Infektion von T-Lymphozyten in Kultur durch HIV inhibieren.

24. Antikörper nach Anspruch 22 gegen diejenigen der Peptide, die die aktive Anheftungsstelle des Adhäsins aus M. pirum umfassen und die selbst die Infektion von T-Lymphozyten in Kultur durch HIV inhibieren.

25. Verwendung zur Herstellung eines Arzneimittels, das in vivo zur Inhibierung der Infektion für HIV, wie HIV-1 oder HIV-2, permissiver Zellen geeignete Antikörper induzieren kann, von
- entweder einem Peptid, das aus einem Fragment eines Strukturproteins eines Mycoplasma besteht und einen Prozentsatz von mindestens 60 % Isologie oder, nach den Umständen, 70 % Homologie zu einer aus HIV stammenden Peptid-, Protein- oder Glykoproteinsequenz aufweist
- oder einem Peptid nach einem der Ansprüche 14 bis 17
- oder einem Peptid nach einem der Ansprüche 18 und 19 in Verbindung mit einem pharmazeutischen Träger.

26. Verwendung nach Anspruch 25,
dadurch gekennzeichnet, daß das verwendete Peptid von einem Adhäsin eines Mycoplasma stammt.

27. Verwendung nach einem der Ansprüche 25 oder 26,
dadurch gekennzeichnet, daß das Peptid von einem Adhäsin aus M. pneumoniae, M. genitalium, M. pirum stammt.

28. Verwendung nach einem der Ansprüche 25 bis 26,
dadurch gekennzeichnet, daß das verwendete Peptid eines derjenigen ist, die in den Ansprüchen 6 bis 13 und in Anspruch 18 definiert wurden.

29. Verwendung zur Herstellung eines Arzneimittels, das in vivo zur Inhibierung der Infektion für HIV, wie HIV-1 oder HIV-2, permissiver Zellen geeignete Antikörper induzieren kann, von einem Protein nach Anspruch 20 oder Anspruch 21.

30. Immunogene Zusammensetzung gegen die Infektion durch HIV, das eines der Peptide nach einem der Ansprüche 6 bis 19 oder ein Protein nach Anspruch 20 oder Anspruch 21 enthält.

31. Immunogene Zusammensetzung nach Anspruch 30, dadurch gekennzeichnet, daß sie darüberhinaus ein unterschiedliches Peptid für eine Impfung gegen HIV enthält.

32. Zusammensetzung nach Anspruch 31,
dadurch gekennzeichnet, daß das unterschiedliche Peptid, das in vivo die Produktion von Antikörpern, die eine Infektion durch HIV neutralisieren können, induzieren kann, ein Peptid ist, das in dem Abschnitt V₃ des gp 120 aus HIV-1 enthalten ist.

33. Impfstoff gegen eine Infektion mit HIV, der einerseits mindestens eines der Peptide nach den Ansprüchen 6 bis 20 oder ein Protein nach Anspruch 20 oder Anspruch 21 und andererseits ein mindestens aus HIV-1 oder HIV-2 stammendes Peptid enthält.

34. Immunogene Zusammensetzung, die in vivo die Produktion von aktiven Antikörpern gegen Mycoplasmen induzieren kann, dadurch gekennzeichnet, daß sie das Protein oder Proteinfragment nach Anspruch 20 oder Anspruch 21 enthält.

35. Verfahren zum Nachweis in einer biologischen Probe, die von einer Person ohne Symptome oder einem Kranken stammt, von Antikörpern gegen Peptide von Mycoplasmen, wobei das Verfahren dadurch gekennzeichnet ist, daß diese biologische Probe mit einem Peptid in Kontakt gebracht wird, das in vivo die Produktion von Antikörpern, die die Infektion durch HIV in vitro inhibieren oder verringern können, induzieren kann, wobei das Peptid einen Prozentsatz von mindestens 60 % Isologie oder, nach den Umständen, 70 % Homologie zu einer aus HIV stammenden Peptid-, Protein- oder Glykoproteinsequenz aufweist.

36. Verfahren zum Nachweis von Antikörpern in einer biologischen Probe, die von einer Person stammt, die mit HIV, mit Mycoplasmen oder mit beiden zugleich infiziert sein könnte, gekennzeichnet dadurch, daß diese Probe mit einem Peptid nach einem der Ansprüche 6 bis 19 in Kontakt gebracht wird.

37. Verfahren zum Nachweis in einer biologischen Probe, die von einer Person ohne Symptome oder einem Kranken stammt, von Antikörpern gegen Peptide von Mycoplasmen, die Antikörper induzieren können, die eine Infektion durch HIV in vitro oder in vivo inhibieren oder verringern können, gekennzeichnet dadurch, daß diese biologische Probe mit einem Protein oder einem Proteinfragment nach Anspruch 20 oder Anspruch 21 in Kontakt gebracht wird.

38. Verfahren zum Nachweis in einer biologischen Probe, die von einer Person ohne Symptome oder einem Kranken stammt, von für M. genitalium spezifischen Antikörpern, gekennzeichnet dadurch, daß diese biologische Probe mit einem Peptid, das den Abschnitt V₃ des gp 120 enthält, insbesondere dem Peptid, dessen Aminosäuren die Nummern 307 bis 321 aus HIV-1 tragen, oder dem Peptid des Anspruch 10, in Kontakt gebracht wird.

39. Verfahren zum Nachweis von für M. genitalium spezifischen Antikörpern in einer biologischen Probe, gekennzeichnet dadurch, daß diese Probe mit einem Peptid nach einem der Ansprüche 6 bis 9, das 5 bis 25 Aminosäuren, vorzugsweise 8 bis 15 Aminosäuren, umfaßt und eine der folgenden Aminosäuresequenzen aufweist: 
G V V S T P L V N L I N G Q
G V V S T P L V N L I N
K M D G K L T G V V S T P L V N L I N G Q
G I V R T P L A E L L D G E
 oder eine entweder längere oder kürzere Sequenz aufweist, die aber die Stelle enthält, die dem entsprechenden Peptid die Fähigkeit verleiht, in vivo die Produktion von Antikörpern zu induzieren, die die Eigenschaft haben, eine Infektion von T-Lymphozyten durch HIV zu inhibieren, in Kontakt gebracht wird.

40. Verfahren zur Herstellung von "hybriden Molekülen" oder "hybriden Peptiden",
gekennzeichnet durch:
- die Transformation eines Organismus, der zur Exprimierung derselben fähig ist, mit einer rekombinanten Nukleinsäure, die zu einem Teil aus einer Nukleotidsequenz, die für mindestens ein zu HIV gehörendes Peptid kodiert, und zu einem Teil aus einer Sequenz, die für mindestens ein Peptid nach einem der Ansprüche 6 bis 19 kodiert, besteht,
- die Kultur der so transformierten Organismen und
- die Gewinnung der "hybriden Moleküle" oder "hybriden Peptide" aus den exprimierten Produkten.

41. Zusammensetzung von hybriden Peptiden,
dadurch gekennzeichnet, daß sie zu HIV gehörige Peptide und zu einem Teil ein Peptid eines Mycoplasma nach einem der Ansprüche 6 bis 19 enthält.

42. Zusammensetzung nach Anspruch 41,
dadurch gekennzeichnet, daß die hybriden Peptide miteinander auf kovalente Weise verbunden sind.

43. Zusammensetzung,
dadurch gekennzeichnet, daß sie eine Mischung eines Peptids von HIV und eines Peptid eines Mycoplasma umfaßt, die miteinander nicht auf kovalente Weise verbunden sind, wobei die Peptide von Mycoplasmen einen Prozentsatz von mindestens 60 % Isologie oder, nach den Umständen, 70 % Homologie zu einer aus HIV stammenden Peptid-, Protein- oder Glykoproteinsequenz aufweisen.

44. Zusammensetzung,
dadurch gekennzeichnet, daß sie eine Mischung eines Peptids von HIV und eines Proteins nach Anspruch 20 oder Anspruch 21 umfaßt.

45. Verwendung einer Zusammensetzung nach einem der Ansprüche 41 bis 44 zur Herstellung eines Impfstoffs gegen eine Infektion durch HIV.

46. Verwendung einer Zusammensetzung, enthaltend ein Peptid nach einem der Ansprüche 6 bis 19 oder eine Sequenz, umfassend ein Fragment eines Strukturproteins eines Mycoplasma nach Anspruch 20 oder 21, zur Herstellung einer Zusammensetzung, die eine in vivo-Produktion von Antikörpern induzieren kann, die die Infektion durch ein HIV inhibieren können, und ein Peptid oder ein Polypeptid von HIV.

## Claims

1. Use of a peptide or peptide sequence consisting of a fragment of a structural protein of a mycoplasma, for the production of a composition capable of inducing an immune response leading to the production of antibodies capable of inhibiting infection by an HIV, the said peptide displaying a percentage of at least 60% isology or, depending on the case, of 70% homology with a peptide, protein or glycoprotein sequence originating from HIV.

2. Use according to Claim 1, characterized in that the immune reaction is an in vivo production of antibodies capable of inhibiting HIV infection.

3. Use according to Claim 1 or 2, characterized in that the mycoplasma structural protein is a mycoplasma adhesin, and in that the fragment used preferably contains an active site of the said adhesin.

4. Use according to Claims 1 to 3, characterized in that the peptide used displays a percentage of at least 60% isology or, depending on the case, of 70% homology with an amino acid sequence of an HIV env gp 160 or nef p 27.

5. Use of the peptide according to one of Claims 1 to 4, characterized in that it contains from 5 to 25 amino acids, and in particular from 8 to 15 amino acids, possessing one of the following amino acid sequences: 
G V V S T P L V N L I N G Q
G V V S T P L V N L I N
K M D G K L T G V V S T P L V N L I N G Q
G I V R T P L A E L L D G E
 or a sequence which is either longer or shorter but which comprises the site which confers on the corresponding peptide the capacity to induce in vivo the production of antibodies having the property of inhibiting an infection of T lymphocytes by HIV.

6. Peptide, characterized in that its sequence originates from a mycoplasma adhesin, and containing a site having the capacity to induce in vivo the production of antibodies having the property of inhibiting an infection of T lymphocytes by HIV, the said peptide displaying a percentage of at least 60% isology or, depending on the case, of 70% homology with a peptide, protein or glycoprotein sequence originating from HIV.

7. Peptide according to Claim 6, characterized in that it originates from an adhesin of mycoplasma chosen, in particular, from M. pneumoniae, M. genitalium or M. pirum.

8. Peptide according to either of Claims 6 and 7, characterized in that it displays a percentage at least of 60% isology or, depending on the case, of 70% homology with an amino acid sequence of a env gp 160 or nef p 27 of an HIV, in particular HIV-1 or HIV-2.

9. Peptide according to any one of Claims 6 to 8, characterized in that it displays the abovementioned percentage isology or, depending on the case, homology with an amino acid sequence originating either from a gag protein, or from an envelope glycoprotein, or alternatively from the nef protein of an HIV.

10. Peptide according to any one of Claims 6 to 9, characterized in that it contains from 5 to 25 amino acids, and in particular from 8 to 15 amino acids, possessing one of the following amino acid sequences: 
G V V S T P L V N L I N G Q
G V V S T P L V N L I N
K M D G K L T G V V S T P L V N L I N G Q
G I V R T P L A E L L D G E
 or a sequence which is either longer or shorter but which comprises the site which confers on the corresponding peptide the capacity to induce in vivo the production of antibodies having the property of inhibiting an infection of T lymphocytes by HIV.

11. Peptide according to Claims 8 to 10, characterized in that it possesses a particular epitope containing the sequence TPL.

12. Peptide according to Claim 6 or 7, characterized in that it is derived from M. genitalium, in that it displays the abovementioned homology or isology with the HIV-1 gp 160, and in that either it comprises the following amino acid sequence: 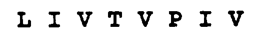 or its amino acid sequence is shorter, is included in the sequence identified above and comprises the site which confers on the corresponding peptide the capacity to induce in vivo the production of antibodies having the property of inhibiting an infection of T lymphocytes by HIV.

13. Peptide according to Claim 6 or 7, characterized in that it is derived from M. genitalium, in that it displays a sequence homology with the HIV-1 nef protein, and in that either it comprises the following amino acid sequence: 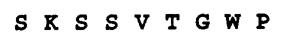 or its amino acid sequence is shorter, is included in the sequence identified above and comprises the site which confers on the corresponding peptide the capacity to induce in vivo the production of antibodies having the property of inhibiting an infection of T lymphocytes by HIV.

14. Peptide originating from a gp 160 protein of an HIV and displaying at least 60% sequence isology with the sequence of a peptide according to Claim 6, and whose amino acid sequence is one of the following:

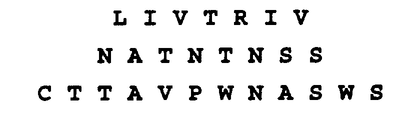

15. Peptide originating from a nef protein of an HIV and displaying at least 60% isology or at least 70% homology with a peptide of Claim 6, whose amino acid sequence is as follows:

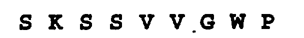

16. Peptide according to Claim 8 or 9, characterized in that it is derived from M. pneumoniae, in that it displays a percentage of at least 60% isology or, depending on the case, of 70% homology with the HIV-1 gp 160 and in that it contains one of the following amino acid sequences

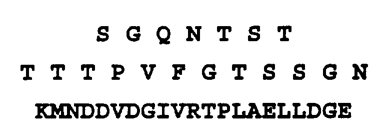

17. Recombinant or hybrid peptide containing a peptide sequence of one of the peptides according to any one of Claims 6 to 16 and a sequence of an immunogenic peptide capable of inducing in vivo the production of antibodies capable of neutralizing an HIV, in particular HIV-1 and HIV-2, infection.

18. Recombinant or hybrid peptide according to Claim 17, characterized in that the sequence of the immunogenic peptide capable of inducing in vivo antibodies capable of neutralizing an HIV infection is the sequence coding for the V₃ region of the HIV-1 gp 120.

19. Peptide according to any one of Claims 6 to 18, characterized in that it is itself grafted onto a carrier protein or polypeptide capable of enhancing the immunogenicity of the said peptide.

20. Purified M. pirum protein having a molecular weight of 130 kDa corresponding to the adhesin, this protein being recognized by antibodies formed against the peptide 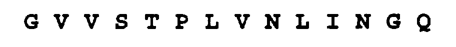 of Claim 10.

21. Purified M. pirum protein having a molecular weight of less than 65 kDa in particular of the order of 64 kDa, 45 or 42 kDa, which protein is recognized by antibodies formed against the peptide 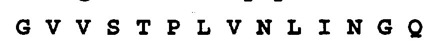 of Claim 10, the said protein originating from the M. pirum strain deposited on 3rd May 1990 with the CNCM under No.I-950 and 17th May 1990 at the NCIMB under No. 40.283.

22. Monoclonal or polyclonal antibody against one of the peptides or proteins according to any one of Claims 6 to 21.

23. Antibody according to Claim 22 against those peptides which contain the active site of binding of the M. genitalium adhesin, and which themselves inhibit the infection by HIV of T lymphocytes in culture.

24. Antibody according to Claim 22 against those peptides which contain the active site of binding of the M. pirum adhesin, and which themselves inhibit the infection by HIV of T lymphocytes in culture.

25. Use for the production of a medicinal product capable of inducing in vivo antibodies capable of inhibiting the infection of cells permissive to HIV, such as HIV-1 or HIV-2,
- either of a peptide consisting of a fragment of a structural protein of a mycoplasma and displaying a percentage of at least 60% isology or, depending on the case, of 70% homology with a peptide, protein or glycoprotein sequence originating from HIV,
- or of a peptide according to any one of Claims 14 to 17,
- or of a peptide according to either of Claims 18 and 19, in combination with a pharmaceutical vehicle.

26. Use according to Claim 25, characterized in that the peptide used originates from a mycoplasma adhesin.

27. Use according to either of Claims 25 and 26, characterized in that the peptide originates from an M. pneumoniae, M. genitalium or M. pirum adhesin.

28. Use according to either of Claims 25 and 26, characterized in that the peptide used is one of those defined in Claims 6 to 13 and in Claim 18.

29. Use of a protein according to Claim 20 or Claim 21 for the production of a medicinal product capable of inducing in vivo antibodies capable of inhibiting the infection of cells permissive to HIV, such as HIV-1 or HIV-2.

30. Immunogenic composition against HIV infection, containing one of the peptides according to any one of Claims 6 to 19 or a protein according to Claim 20 or Claim 21.

31. Immunogenic composition according to Claim 30, characterized in that it contains, in addition, a separate peptide vaccinating against HIV.

32. Composition according to Claim 31, characterized in that the separate peptide capable of inducing in vivo the production of antibodies capable of neutralizing an HIV infection is a peptide contained in the V₃ region of the HIV-1 gp 120.

33. Vaccine against an HIV infection containing, on the one hand at least one of the peptides according to Claims 6 to 20 or a protein according to Claim 20 or Claim 21, and on the other hand a peptide originating from at least HIV-1 or HIV-2.

34. Immunogenic composition capable of inducing in vivo the production of antibodies which are active against mycoplasmas, characterized in that it contains the protein or protein fragment according to Claim 20 or Claim 21.

35. Method for detecting antibodies against mycoplasma peptides in a biological sample taken from an asymptomatic person or from a patient, the said method being characterized by the bringing of this biological sample into contact with a peptide capable of inducing in vivo the production of antibodies capable of inhibiting or decreasing HIV infection in vitro, the said peptide displaying a percentage of at least 60% isology or, depending on the case, of 70% homology with a peptide, protein or glycoprotein sequence originating from HIV.

36. Method for detecting antibodies in a biological sample taken from a person liable to be infected with HIV, with mycoplasmas or with both at once, characterized by the bringing of this sample into contact with a peptide according to any one of Claims 6 to 19.

37. Method for detecting antibodies against mycoplasma peptides capable of inducing antibodies which inhibit or decrease an HIV infection in vitro or in vivo, in a biological sample taken from an asymptomatic person or from a patient, characterized by the bringing of this biological sample into contact with a protein or protein fragment according to Claim 20 or Claim 21.

38. Method for detecting antibodies specific for M. genitalium in a biological sample taken from an asymptomatic person or from a patient, characterized by the bringing of this biological sample into contact with a peptide containing the V₃ region of gp 120, especially the peptide whose amino acids bear the numbers 307 to 321 of HIV-1 or the peptide of Claim 10.

39. Method for the detection of antibodies specific for M. genitalium in a biological sample, characterized by the bringing of this sample into contact with a peptide according to any one of Claims 6 to 9, containing from 5 to 25 amino acids, and preferably from 8 to 15 amino acids, possessing one of the following amino acid sequences: 
G V V S T P L V N L I N G Q
G V V S T P L V N L I N
K M D G K L T G V V S T P L V N L I N G Q
G I V R T P L A E L L D G E
 or a sequence which is either longer or shorter but which comprises the site which confers on the corresponding peptide the capacity to induce in vivo the production of antibodies having the property of inhibiting an infection of T lymphocytes by HIV.

40. Method for preparing "hybrid molecules" or "hybrid peptides", characterized by:
- the transformation of an organism capable of expressing them with a recombinant nucleic acid consisting in part of a nucleotide sequence coding for at least one peptide belonging to HIV and in part of a sequence coding for at least one peptide according to one of Claims 6 to 19,
- the culturing of the organisms thus transformed, and
- the recovery of the said "hybrid molecules" or "hybrid peptides" from among the products expressed.

41. Composition of hybrid peptides, characterized in that it contains peptides belonging to HIV and, in part, a mycoplasma peptide according to one of Claims 6 to 19.

42. Composition according to Claim 41, characterized in that the hybrid peptides are linked to one another covalently.

43. Composition, characterized in that it consists of a mixture of an HIV peptide and a mycoplasma peptide, not linked to one another covalently, the mycoplasma peptides displaying a percentage of at least 60% isology or, depending on the case, of 70% homology with a peptide from a protein or glycoprotein sequence originating from HIV.

44. Composition, characterized in that it consists of a mixture of an HIV peptide and a protein according to Claim 20 or Claim 21.

45. Use of a composition according to one of Claims 41 to 44, for the production of a vaccine against an HIV infection.

46. Use of a composition comprising a peptide according to any one of Claims 6 to 19 or a sequence consisting of a fragment of a structural protein of a mycoplasma according to Claim 20 or 21, for the production of a composition capable of inducing an in vivo production of antibodies capable of inhibiting infection by an HIV, and an HIV peptide or polypeptide.
